# EUROPEAN PATENT APPLICATION

(11) **EP 4 834 946 A1**
(43) Date of publication of application: **04.11.2026**
(21) Application number: 24911615.3
(22) Date of filing: 30.12.2024
(51) Int. Cl.: C07K 16/46, C12N 15/85, C12N 5/10, A61K 39/395, A61P 35/00

(54) **ANTI-EGFR MONOCLONAL ANTIBODY, BISPECIFIC ANTIBODY THEREOF, AND PHARMACEUTICAL COMPOSITION AND USE THEREOF**

(30) Priority: 29.12.2023 CN 202311871929
(71) Applicant: Shanghai Allink Biotherapeutics Co., Ltd., Shanghai 201208 (CN)
(72) Inventor: LI, Li, Shanghai 201208 (CN); FENG, Hui, Shanghai 201208 (CN)
(74) Representative: Bandpay & Greuter
(86) International application number: PCT/CN2024/143726
(87) International publication number: WO 2025/140701

(57) **Abstract**

The present invention belongs to the field of biomedicine and relates to an anti-EGFR monoclonal antibody, a bispecific antibody thereof, and a pharmaceutical composition and use thereof. Specifically, the present invention relates to an anti-EGFR antibody or an antigen-binding fragment thereof. The present invention further relates to an anti-EGFR-anti-Met bispecific antibody. The anti-EGFR monoclonal antibody and the bispecific antibody of the present invention have good anti-tumor activity.

## Description

### TECHNICAL FIELD

This invention belongs to the field of biomedicine and relates to an anti-EGFR monoclonal antibody, bispecific antibody thereof, pharmaceutical composition, and use thereof. This invention further relates to an anti-EGFR-anti-Met bispecific antibody.

### BACKGROUND

EGFR (Epidermal Growth Factor Receptor) is the receptor for epidermal growth factor (EGF) and belongs to the ErbB receptor family. EGFR is a transmembrane glycoprotein with a molecular weight of 170 kDa. It is a receptor-type tyrosine kinase. Under the action of related ligands such as epidermal growth factor (EGF) and transforming growth factor-α (TGFα), EGFR is activated by converting from a monomer to a dimer, thereby further activating downstream signaling pathways, such as phosphorylation of kinases like Akt and ERK, and regulating cell proliferation (Jorissen RN, Walker F, Pouliot N, et al. Epidermal growth factor receptor: mechanisms of activation and signaling. Exp Cell Res 2003; 284:31-53.). Numerous studies have shown that EGFR is highly expressed or abnormally expressed in most tumors, such as glial cell carcinoma, renal cell carcinoma, lung cancer, prostate cancer, pancreatic cancer, and breast cancer (Modjtahedi H, and Dean C. The receptor for EGF and its ligands Expression, prognostic value and target for therapy in cancer. International Journal of Oncology 1994; (4): 277-96.). Abnormal EGFR function is associated with tumor cell proliferation, angiogenesis, tumor invasion, metastasis, and inhibition of apoptosis (Castillo L, Etienne Grimaldi MC, Fischel JL, et al. Pharmacological background of EGFR targeting. Ann Oncol 2004; 15: 1007-12.). Its abnormal function is mainly manifested in two aspects: firstly, excessive and abnormal expression in tumor tissues; and secondly, persistent activation of EGFR mutants in tumor cells (without ligand stimulation or the formation of a self-circulating stimulation pathway).

c-mesenchymal-epithelial transition factor (referred to as c-Met, cMet, or Met) is a member of the receptor tyrosine kinase family. The Met receptor binds to its ligand, hepatocyte growth factor (HGF), inducing Met dimerization and activation, which in turn activates downstream signaling pathways, such as phosphorylation of Akt and ERK kinases. Met plays a crucial role in embryonic development, organ growth, and wound healing, and is typically expressed only in stem cells and progenitor cells. In cancer, abnormal Met activation due to mutations promotes angiogenesis and cancer metastasis.

Lung cancer is the leading cause of cancer morbidity and mortality, with non-small cell lung cancer (NSCLC) accounting for 90% of lung cancer cases. EGFR mutation is a major driving factor in NSCLC, affecting over 40% of NSCLC patients in Asia. EGFR kinase inhibitors (EGFR-TKIs) are the primary treatment; however, a high rate of drug resistance and complex mechanisms exist, with 7-15% of patients developing Met amplification. Furthermore, Met exon 14 skipping mutations, Met fusions, Met amplification, and overexpression are also primary driving factors in NSCLC. Bispecific antibodies targeting both EGFR and Met can simultaneously inhibit both signaling pathways, offering promise for patients who are unresponsive or resistant to EGFR-TKIs.

Currently, several EGFR×Met bispecific antibodies have been publicly reported. US9328173B2 (Eli Lilly and Company), WO2018221969A1 (Chung Kun Tang Biopharmaceutical Co., Ltd.), and WO2022104236A2 (Ab theraputics) disclose the construction methods of "2+2" type EGFR×Met bispecific antibodies, where both the anti-EGFR and anti-Met ends are bivalent. Because EGFR antibodies have skin toxicity, "2+2" type EGFR×Met bispecific antibodies have potentially higher safety risks. Johnson & Johnson's Amivantamab (US2017275367A1) discloses the construction method of a "1+1" type EGFR×Met bispecific antibody, where both the anti-EGFR and anti-Met ends are monovalent. This antibody has been approved for marketing and has shown good safety.

Constructing "1+1" type EGFR×Met bispecific antibodies presents significant technical challenges, requiring solutions to the correct pairing of light and heavy chains and the heterodimerization of the heavy chain. As reported in US2017275367A1, Amivantamab employed Fab arm exchange technology; however, this method necessitates the separate preparation of anti-EGFR and anti-Met antibodies, followed by in vitro recombination to obtain the bispecific antibody, increasing production costs. Shared light chain technology is one method for constructing bispecific antibodies, as it eliminates the light-heavy chain mismatch problem, thus greatly facilitating bispecific antibody preparation.

There is still a need to develop new anti-EGFR antibodies and anti-EGFR-anti-Met bispecific antibodies.

### Summary

Through in-depth research and creative work, the inventors have obtained bispecific antibodies, particularly anti-EGFR-anti-Met bispecific antibodies. This invention utilizes common light chain technology to construct EGFR×Met bispecific antibodies, which can inhibit EGF and HGF-induced Akt and ERK phosphorylation; promote EGFR and Met endocytosis; and kill tumor cells through ADCC effects. The bispecific antibodies of this invention exhibit good antitumor activity. Therefore, the following invention is provided:
One aspect of the present invention relates to an anti-EGFR antibody or an antigen-binding fragment thereof, the anti-EGFR antibody comprising a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 25 or SEQ ID NO: 26, the amino acid sequence of HCDR2 is as shown by SEQ ID NO: 27, and the amino acid sequence of HCDR3 is as shown by SEQ ID NO: 37; and
the amino acid sequence of LCDR1 is as shown by SEQ ID NO: 38, the amino acid sequence of LCDR2 is as shown by SEQ ID NO: 39, and the amino acid sequence of LCDR3 is as shown by SEQ ID NO: 40, SEQ ID NO: 41 or SEQ ID NO: 76.

In some embodiments of the present invention, the anti-EGFR antibody or antigen-binding fragment thereof, wherein,
the amino acid sequence of HCDR3 is as shown by any one of SEQ ID NO: 28 to SEQ ID NO: 36.

In some embodiments of the present invention, the anti-EGFR antibody or antigen-binding fragment thereof, wherein,
the amino acid sequence of the heavy chain variable region of the anti-EGFR antibody is selected from any one of SEQ ID NO: 1 to SEQ ID NO: 10; and
the amino acid sequence of the light chain variable region of the anti-EGFR antibody is selected from SEQ ID NO: 42, SEQ ID NO: 43, SEQ ID NO: 44 and SEQ ID NO: 77.

In some embodiments of the present invention, the anti-EGFR antibody or antigen-binding fragment thereof, wherein,
the amino acid sequence of the heavy chain variable region is as shown by SEQ ID NO: 1, and the amino acid sequence of the light chain variable region is as shown by SEQ ID NO: 42;
the amino acid sequence of the heavy chain variable region is as shown by SEQ ID NO: 2, and the amino acid sequence of the light chain variable region is as shown by SEQ ID NO: 42;
the amino acid sequence of the heavy chain variable region is as shown by SEQ ID NO: 3, and the amino acid sequence of the light chain variable region is as shown by SEQ ID NO: 42;
the amino acid sequence of the heavy chain variable region is as shown by SEQ ID NO: 4, and the amino acid sequence of the light chain variable region is as shown by SEQ ID NO: 42;
the amino acid sequence of the heavy chain variable region is as shown by SEQ ID NO: 5, and the amino acid sequence of the light chain variable region is as shown by SEQ ID NO: 42;
the amino acid sequence of the heavy chain variable region is as shown by SEQ ID NO: 6, and the amino acid sequence of the light chain variable region is as shown by SEQ ID NO: 42;
the amino acid sequence of the heavy chain variable region is as shown by SEQ ID NO: 7, and the amino acid sequence of the light chain variable region is as shown by SEQ ID NO: 42;
the amino acid sequence of the heavy chain variable region is as shown by SEQ ID NO: 8, and the amino acid sequence of the light chain variable region is as shown by SEQ ID NO: 42;
the amino acid sequence of the heavy chain variable region is as shown by SEQ ID NO: 9, and the amino acid sequence of the light chain variable region is as shown by SEQ ID NO: 42;
the amino acid sequence of the heavy chain variable region is as shown by SEQ ID NO: 10, and the amino acid sequence of the light chain variable region is as shown by SEQ ID NO: 42;
the amino acid sequence of the heavy chain variable region is as shown by SEQ ID NO: 1, and the amino acid sequence of the light chain variable region is as shown by SEQ ID NO: 43;
the amino acid sequence of the heavy chain variable region is as shown by SEQ ID NO: 2, and the amino acid sequence of the light chain variable region is as shown by SEQ ID NO: 43;
the amino acid sequence of the heavy chain variable region is as shown by SEQ ID NO: 3, and the amino acid sequence of the light chain variable region is as shown by SEQ ID NO: 43;
the amino acid sequence of the heavy chain variable region is as shown by SEQ ID NO: 4, and the amino acid sequence of the light chain variable region is as shown by SEQ ID NO: 43;
the amino acid sequence of the heavy chain variable region is as shown by SEQ ID NO: 5, and the amino acid sequence of the light chain variable region is as shown by SEQ ID NO: 43;
the amino acid sequence of the heavy chain variable region is as shown by SEQ ID NO: 6, and the amino acid sequence of the light chain variable region is as shown by SEQ ID NO: 43;
the amino acid sequence of the heavy chain variable region is as shown by SEQ ID NO: 7, and the amino acid sequence of the light chain variable region is as shown by SEQ ID NO: 43;
the amino acid sequence of the heavy chain variable region is as shown by SEQ ID NO: 8, and the amino acid sequence of the light chain variable region is as shown by SEQ ID NO: 43;
the amino acid sequence of the heavy chain variable region is as shown by SEQ ID NO: 9, and the amino acid sequence of the light chain variable region is as shown by SEQ ID NO: 43;
the amino acid sequence of the heavy chain variable region is as shown by SEQ ID NO: 10, and the amino acid sequence of the light chain variable region is as shown by SEQ ID NO: 43;
the amino acid sequence of the heavy chain variable region is as shown by SEQ ID NO: 1, and the amino acid sequence of the light chain variable region is as shown by SEQ ID NO: 44;
the amino acid sequence of the heavy chain variable region is as shown by SEQ ID NO: 2, and the amino acid sequence of the light chain variable region is as shown by SEQ ID NO: 44;
the amino acid sequence of the heavy chain variable region is as shown by SEQ ID NO: 3, and the amino acid sequence of the light chain variable region is as shown by SEQ ID NO: 44;
the amino acid sequence of the heavy chain variable region is as shown by SEQ ID NO: 4, and the amino acid sequence of the light chain variable region is as shown by SEQ ID NO: 44;
the amino acid sequence of the heavy chain variable region is as shown by SEQ ID NO: 5, and the amino acid sequence of the light chain variable region is as shown by SEQ ID NO: 44;
the amino acid sequence of the heavy chain variable region is as shown by SEQ ID NO: 6, and the amino acid sequence of the light chain variable region is as shown by SEQ ID NO: 44;
the amino acid sequence of the heavy chain variable region is as shown by SEQ ID NO: 7, and the amino acid sequence of the light chain variable region is as shown by SEQ ID NO: 44;
the amino acid sequence of the heavy chain variable region is as shown by SEQ ID NO: 8, and the amino acid sequence of the light chain variable region is as shown by SEQ ID NO: 44;
the amino acid sequence of the heavy chain variable region is as shown by SEQ ID NO: 9, and the amino acid sequence of the light chain variable region is as shown by SEQ ID NO: 44;
the amino acid sequence of the heavy chain variable region is as shown by SEQ ID NO: 10, and the amino acid sequence of the light chain variable region is as shown by SEQ ID NO: 44;
   or
the amino acid sequence of the heavy chain variable region is as shown by SEQ ID NO: 1, 2, 3, 4, 5, 6, 7, 8, 9 or 10, and the amino acid sequence of the light chain variable region is as shown by SEQ ID NO: 77.

In some embodiments of the present invention, the anti-EGFR antibody or antigen-binding fragment thereof, wherein,
the anti-EGFR antibody comprises a non-CDR region, and the non-CDR region is derived from a species other than murine, e.g., derived from a human antibody.

In some embodiments of the present invention, the anti-EGFR antibody or antigen-binding fragment thereof, wherein the constant region of the anti-EGFR antibody is selected from the constant regions of human IgG1, IgG2, IgG3 or IgG4.

In some embodiments of the present invention, the anti-EGFR antibody or antigen-binding fragment thereof, wherein, the heavy chain constant region of the anti-EGFR antibody is Ig gamma-1 chain C region or Ig gamma-4 chain C region; the light chain constant region is Ig kappa chain C region or Ig lambda chain C region.

In some embodiments of the present invention, the anti-EGFR antibody or antigen-binding fragment thereof, wherein the heavy chain constant region sequence of the anti-EGFR antibody is selected from SEQ ID NO: 79, 80 and 81.

In some embodiments of the present invention, the heavy chain of the anti-EGFR antibody is selected from SEQ ID NO: 17, 18, 19, 58, 59, 60, 61, 62, 63, 64, 65, 66, 67, 68, 69, 70, 71, 72, 73, 74 and 75; and
the light chain of the anti-EGFR antibody is selected from SEQ ID NO: 11, SEQ ID NO: 12 and SEQ ID NO: 13.

In some embodiments of the present invention, the anti-EGFR antibody or antigen-binding fragment thereof, wherein:
the amino acid sequence of the heavy chain is as shown by SEQ ID NO: 17, and the amino acid sequence of the light chain is as shown by SEQ ID NO: 11;
the amino acid sequence of the heavy chain is as shown by SEQ ID NO: 17, and the amino acid sequence of the light chain is as shown by SEQ ID NO: 12;
the amino acid sequence of the heavy chain is as shown by SEQ ID NO: 17, and the amino acid sequence of the light chain is as shown by SEQ ID NO: 13;
the amino acid sequence of the heavy chain is as shown by SEQ ID NO: 18, and the amino acid sequence of the light chain is as shown by SEQ ID NO: 11;
the amino acid sequence of the heavy chain is as shown by SEQ ID NO: 18, and the amino acid sequence of the light chain is as shown by SEQ ID NO: 12;
the amino acid sequence of the heavy chain is as shown by SEQ ID NO: 18, and the amino acid sequence of the light chain is as shown by SEQ ID NO: 13;
the amino acid sequence of the heavy chain is as shown by SEQ ID NO: 19, and the amino acid sequence of the light chain is as shown by SEQ ID NO: 11;
the amino acid sequence of the heavy chain is as shown by SEQ ID NO: 19, and the amino acid sequence of the light chain is as shown by SEQ ID NO: 12;
the amino acid sequence of the heavy chain is as shown by SEQ ID NO: 19, and the amino acid sequence of the light chain is as shown by SEQ ID NO: 13;
   or
the amino acid sequence of the heavy chain is as shown by SEQ ID NO: 17, 18, 19, 58, 59, 60, 61, 62, 63, 64, 65, 66, 67, 68, 69, 70, 71, 72, 73, 74 or 75, and the amino acid sequence of the light chain is as shown by SEQ ID NO: 57.

In some embodiments of the present invention, the anti-EGFR antibody or antigen-binding fragment thereof, wherein the anti-EGFR antibody or antigen-binding fragment thereof is selected from Fab, Fab', F(ab')₂, Fd, Fv, dAb, complementarity-determining region fragment, single-chain antibody, humanized antibody or chimeric antibody.

In some embodiments of the present invention, the heavy chain constant region is Ig gamma-1 chain C region (e.g., NCBI ACCESSION: P01857) or Ig gamma-4 chain C region (e.g., NCBI ACCESSION: P01861.1); the light chain constant region is Ig kappa chain C region (e.g., NCBI ACCESSION: P01834).

Another aspect of the present invention relates to an isolated nucleic acid molecule encoding the anti-EGFR antibody or antigen-binding fragment thereof according to any embodiment of the present invention.

The present invention further relates to a recombinant vector comprising the isolated nucleic acid molecule of the present invention.

A further aspect of the present invention relates to a host cell comprising the isolated nucleic acid molecule of the present invention or the recombinant vector of the present invention.

A further aspect of the present invention relates to a pharmaceutical composition comprising an effective amount of the anti-EGFR antibody or antigen-binding fragment thereof according to any embodiment of the present invention, together with one or more pharmaceutically acceptable excipients.

In some embodiments of the present invention, the pharmaceutical composition further comprises an effective amount of an anti-Met antibody or its antigen-binding fragment;
preferably, the anti-Met antibody comprises a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
   and
the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

In some embodiments of the present invention, in the pharmaceutical composition,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, wherein the anti-Met antibody:
   the amino acid sequence of the heavy chain is as shown by SEQ ID NO: 16 or SEQ ID NO: 21, and the amino acid sequence of the light chain variable region is as shown by SEQ ID NO: 14.

Another aspect of the present invention relates to a combined medicament product comprising an individually packaged first medicament product and a second medicament product, wherein:
the first medicament product comprises an effective amount of any embodiment of the anti-EGFR antibodies or antigen-binding fragments thereof according to any embodiment of the present invention, and one or more pharmaceutically acceptable excipients;
the second medicament product comprises an effective amount of an anti-Met antibody or antigen-binding fragment thereof, and one or more pharmaceutically acceptable excipients;
optionally, the combined medicament product further comprises a product instruction.

In some embodiments of the present invention, the combined medicament product, wherein:
the anti-Met antibody comprises a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
   and
the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

In some embodiments of the present invention, the combined medicament product, wherein,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, wherein the anti-Met antibody:
   the amino acid sequence of the heavy chain is as shown by SEQ ID NO: 16 or SEQ ID NO: 21, and the amino acid sequence of the light chain variable region is as shown by SEQ ID NO: 14.

Another aspect of the present invention relates to a bispecific antibody, comprising:
a first protein functional region targeting EGFR, and a second protein functional region targeting a target different from EGFR (e.g., Met);
wherein, the first protein functional region comprises a heavy chain variable region of any anti-EGFR antibody or antigen-binding fragment thereof according to any embodiment of the present invention, or comprises a heavy chain variable region and a light chain variable region of any anti-EGFR antibody or antigen-binding fragment thereof according to any embodiment of the present invention.

In some embodiments of the present invention, the bispecific antibody is an anti-EGFR-anti-Met bispecific antibody, also known as an EGFR×Met bispecific antibody.

In some embodiments of the present invention, the bispecific antibody is a "1+1" type EGFR×Met bispecific antibody.

In some embodiments of the present invention, the bispecific antibody, wherein:
the second protein functional region comprises a heavy chain variable region of an anti-Met antibody or its antigen-binding fragment; or comprises a heavy chain variable region and a light chain variable region of an anti-Met antibody or its antigen-binding fragment;
wherein,
the anti-Met antibody comprises a heavy chain variable region and a light chain variable region, the heavy chain variable region comprising HCDR1 to HCDR3, and the light chain variable region comprising LCDR1 to LCDR3, wherein:
   the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
      and
   the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

In some embodiments of the present invention, the bispecific antibody, wherein,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, wherein the anti-Met antibody:
   the amino acid sequence of the heavy chain is as shown by SEQ ID NO: 16 or SEQ ID NO: 21, and the amino acid sequence of the light chain variable region is as shown by SEQ ID NO: 14.

In some embodiments of the present invention, the bispecific antibody, wherein,
the first protein functional region and the second protein functional region are independently a fusion protein of a single-chain antibody or a half-molecule monovalent antibody (IgG half-molecule, IgG-HM).

In some embodiments of the present invention, the bispecific antibody, wherein,
the first protein functional region is a half-molecule monovalent antibody, and
the second protein functional region is a half-molecule monovalent antibody.

In some embodiments of the present invention, the bispecific antibody, wherein,
the first protein functional region is a half-molecule monovalent antibody targeting EGFR, and
the second protein functional region is a half-molecule monovalent antibody targeting Met.

In some embodiments of the present invention, the bispecific antibody, wherein,
the heavy chain constant regions of the two half-molecule monovalent antibodies respectively comprise a first CH3 region and a second CH3 region, the sequences of the first CH3 region and the second CH3 region are different, and the heterodimeric interaction between the first CH3 region and the second CH3 region is stronger than the homodimeric interaction of the first CH3 region itself and the second CH3 region itself, respectively.

In some embodiments of the present invention, the bispecific antibody, wherein,
the heavy chain constant regions of the two half-molecule monovalent antibodies respectively comprise a first CH3 region and a second CH3 region, and the mutation at position 405 of the first CH3 region is Ala, Asp, Glu, His, Ile, Met, Asn, Gln, Thr, Val, Tyr, Leu, Lys, Ser, or Trp; and the mutation at position 409 of the second CH3 region is an amino acid Ala, Asp, Glu, Phe, Gly, His, Ile, Asn, Gln, Arg, Ser, Thr, Val, Trp, or Tyr, according to the EU Numbering System.

In some embodiments of the present invention, the bispecific antibody, wherein,
the heavy chain constant regions of the two half-molecule monovalent antibodies it comprises respectively comprise a first CH3 region and a second CH3 region, and position 405 of the first CH3 region is mutated to Leu; and position 409 of the second CH3 region is mutated to amino acid Arg, according to the EU Numbering System.

In some embodiments of the present invention, the bispecific antibody, wherein,
the heavy chain constant region of the half-molecule monovalent antibody is the heavy chain constant region of human IgG1.

In some embodiments of the present invention, the bispecific antibody, wherein,
the heavy chain constant region of the half-molecule monovalent antibody is the heavy chain constant region of human IgG1 and has a Knob mutation (e.g., S354C and T366W mutations); and,
the heavy chain constant region of the half-molecule monovalent antibody is the heavy chain constant region of human IgG1 and has a Hole mutation (e.g., Y349C, T366S, L368A, and Y407V mutations).

In this invention, the mutation locations of Knob and Hole are numbered according to the EU numbering system. In some embodiments of this invention, the Knob mutation refers to the S354C and T366W mutations. In some embodiments of this invention, the Hole mutation refers to the Y349C, T366S, L368A, and Y407V mutations.

In some embodiments of the present invention, the bispecific antibody, wherein the heavy chain constant region of the half-molecule monovalent antibody is the heavy chain constant region of human IgG1, and the 435th position of the heavy chain constant region of one half-molecule monovalent antibody is mutated to amino acid Arg(R), and the 436th position is mutated to amino acid Phe(F), according to the EU Numbering System.

In some embodiments of the present invention, the bispecific antibody is in IgG form, preferably in IgG1 form;
preferably, the light chains in the bispecific antibody have identical sequences;
preferably, the bispecific antibody has two light chains with identical sequences;
preferably, the bispecific antibody is composed of the following peptide chains:
   (1) a peptide chain selected from SEQ ID NO: 17 to SEQ ID NO: 19, and SEQ ID NO: 58 to SEQ ID NO: 75,
   (2) a peptide chain selected from SEQ ID NO: 16 and SEQ ID NO: 20, and
   (3) a peptide chain selected from SEQ ID NO: 11 to SEQ ID NO: 13, and SEQ ID NO: 57,
wherein, the peptide chain in (3) is two identical copies;
preferably, the peptide chains in (1) and (2), the peptide chains in (2) and (3), and the peptide chains in (1) and (3) are linked by one or more disulfide bonds (e.g., two or three disulfide bonds).

In some embodiments of the present invention, the bispecific antibody consists of the following peptide chains:
the peptide chain shown in SEQ ID NO: 17, SEQ ID NO: 16, and SEQ ID NO: 12, wherein the peptide chain shown in SEQ ID NO: 12 is two identical copies;
the peptide chain shown in SEQ ID NO: 17, SEQ ID NO: 16, and SEQ ID NO: 13, wherein the peptide chain shown in SEQ ID NO: 13 is two identical copies;
the peptide chain shown in SEQ ID NO: 18, SEQ ID NO: 20, and SEQ ID NO: 12, wherein the peptide chain shown in SEQ ID NO: 12 is two identical copies;
the peptide chain shown in SEQ ID NO: 18, SEQ ID NO: 20, and SEQ ID NO: 13, wherein the peptide chain shown in SEQ ID NO: 13 is two identical copies;
the peptide chain shown in SEQ ID NO: 19, SEQ ID NO: 20, and SEQ ID NO: 12, wherein the peptide chain shown in SEQ ID NO: 12 is two identical copies;
the peptide chains shown in SEQ ID NO: 19, SEQ ID NO: 20, and SEQ ID NO: 13, wherein the peptide chain shown in SEQ ID NO: 13 is two identical copies;
the peptide chain shown in any of SEQ ID NO: 17, 18, 19, 58, 59, 60, 61, 62, 63, 64, 65, 66, 67, 68, 69, 70, 71, 72, 73, 74, or 75, and the peptide chain shown in SEQ ID NO: 16 and SEQ ID NO: 57, wherein the peptide chain shown in SEQ ID NO: 57 is two identical copies;
   or
the peptide chain shown in any one of 17, 18, 19, 58, 59, 60, 61, 62, 63, 64, 65, 66, 67, 68, 69, 70, 71, 72, 73, 74, or 75, as well as the peptide chain shown in SEQ ID NO: 20 and the peptide chain shown in SEQ ID NO: 57, wherein the peptide chain shown in SEQ ID NO: 57 is two identical copies.

Another aspect of the invention relates to an isolated nucleic acid molecule that encodes the bispecific antibody according to any embodiment of the inventions.

Another aspect of the invention relates to a recombinant expression vector comprising the isolated nucleic acid molecules of the present invention.

Another aspect of the present invention relates to a recombinant host cell comprising the recombinant expression vector of the present invention, preferably, the recombinant host cell being a recombinant CHO-K1 cell.

Another aspect of the invention relates to a pharmaceutical composition comprising the bispecific antibody according to any embodiment of the invention, and one or more pharmaceutically acceptable excipients.

Another aspect of the present invention relates to the use of any anti-EGFR antibody or antigen-binding fragment thereof according to any embodiment of the present invention, or any bispecific antibody according to any embodiment of the present invention, in the preparation of a medicament for treating or preventing tumors;
preferably, the tumor is a tumor that highly expresses EGFR and/or Met;
preferably, the tumor is one or more selected from the following: glial cell carcinoma, kidney cancer, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, stomach cancer, brain cancer, thyroid cancer, and head and neck cancer;
preferably, the lung cancer is non-small cell lung cancer.

The anti-EGFR antibody or its antigen-binding fragment according to any embodiment of the present invention, or the bispecific antibody according to any embodiment of the present invention, is used for the treatment or prevention of tumors;
preferably, the tumor is a tumor that highly expresses EGFR and/or Met;
preferably, the tumor is one or more selected from glial cell carcinoma, renal cell carcinoma, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, gastric cancer, brain cancer, thyroid cancer, and head and neck cancer;
preferably, the lung cancer is non-small cell lung cancer.

Another aspect of the invention relates to a method for treating or preventing tumors, comprising the step of administering to a subject in need an effective amount of any embodiment of the anti-EGFR antibodies or antigen-binding fragments thereof, or any embodiment of the bispecific antibodies of the present invention;
preferably, the tumor is a tumor that highly expresses EGFR and/or Met;
preferably, the tumor is one or more selected from glial carcinoma, renal cell carcinoma, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, gastric cancer, brain cancer, thyroid cancer, and head and neck cancer;
preferably, the lung cancer is non-small cell lung cancer.

In some embodiments of the present invention, the method for treating or preventing tumors includes administration of the drug before or after surgery, and/or before or after radiotherapy.

In some embodiments of the present invention, the method for treating or preventing a tumor, wherein,
the single dose of the anti-EGFR antibody or antigen-binding fragment thereof or the bispecific antibody is 0.1-100 mg per kilogram of body weight, preferably 5-50 mg or 5-15 mg per kilogram of body weight;
preferably, administration is once every 3 days, every 4 days, every 5 days, every 6 days, every 10 days, every 1 week, every 2 weeks or every 3 weeks;
preferably, the mode of administration is intravenous infusion or intravenous injection.

### DESCRIPTION OF DRAWINGS

Figure 1: schematic diagram of the structure of bispecific antibody E2mut34-91A×M5-91A-FAE-LF.
Figure 2: schematic diagram of the structure of bispecific antibody E2mut34-69×M5-69-FAE-LF.
Figure 3: schematic diagram of the structure of bispecific antibody JNJ-372.
Figure 4: results of EGFR ELISA binding assay.
Figure 5: results of Met ELISA binding assay.
Figure 6: results of EGFR ELISA blocking assay.
Figure 7: results of Met ELISA blocking assay.
Figures 8A to 8C: antibodies E2-mut34-91A×M5-91A-FAE-LF, E2-mut34-69×M5-69-FAE-LF, and JNJ-372 all exhibit binding activity with recombinant human EGFR. Specifically: Figure 8A: Binding and dissociation curve of antibody E2-mut34-91A×M5-91A-FAE-LF with human EGFR; Figure 8B: Binding and dissociation curve of antibody E2-mut34-69×M5-69-FAE-LF with human EGFR; Figure 8C: Binding and dissociation curve of antibody JNJ-372 with human EGFR.
Figures 9A to 9C: antibodies E2-mut34-91A×M5-91A-FAE-LF, E2-mut34-69×M5-69-FAE-LF, and JNJ-372 all exhibit binding activity with recombinant human Met. Specifically: Figure 9A: Binding and dissociation curve of antibody E2-mut34-91A×M5-91A-FAE-LF with human Met; Figure 9B: Binding and dissociation curve of antibody E2-mut34-69×M5-69-FAE-LF with human Met; Figure 9C: Binding and dissociation curve of antibody JNJ-372 with human Met.
Figures 10A to 10G: cellular binding assays of the test substance.
Figures 11A to 11M: detection results of phosphorylation of AKT or ERK by the test substance.
Figure 12A: results of ADCC assay detected by reporter gene method.
Figure 12B: results of ADCC assay detected by reporter gene method.
Figure 12C: results of ADCC assay detected by LDH method.
Figure 12D: results of ADCC assay detected by reporter gene method.
Figure 12E: results of ADCC assay detected by LDH method.
Figure 13: inhibitory effect of the test substance on NCI-H1975 human lung cancer xenograft model in CB-17 SCID mice.
Figure 14: inhibitory effect of the test substance on NCI-H1975 human lung cancer subcutaneous tumor model.
Figure 15: inhibitory effect of the test substance on H1975 (L858R/T790M/C797S) human lung cancer subcutaneous tumor model.
Figure 16: inhibitory effect of the test substance on H292 human lung cancer subcutaneous tumor model.

### DETAILED DESCRIPTION

### Scientific and Technical Terms

In the present invention, unless otherwise stated, the scientific and technical terms used herein have the meanings commonly understood by those skilled in the art. Furthermore, the cell culture, molecular genetics, nucleic acid chemistry, and immunology laboratory procedures used herein are all standard procedures widely used in their respective fields. To better understand this invention, definitions and explanations of relevant terms are provided below.

As used herein, the term EC50 refers to the concentration for 50% of the maximal effect (concentration for 50% of maximal effect), which is the concentration at which the maximal effect is achieved.

As used herein, the term "antibody" refers to an immunoglobulin molecule typically composed of two pairs of polypeptide chains (each pair consisting of one "light" (L) chain and one "heavy" (H) chain). Antibody light chains can be classified as κ and λ light chains. Heavy chains can be classified as µ, δ, γ, α, or ε, and antibody isotypes are defined as IgM, IgD, IgG, IgA, and IgE, respectively. Within both light and heavy chains, variable and constant regions are linked by "J" regions of approximately 12 or more amino acids, and the heavy chain also comprises "D" regions of approximately 3 or more amino acids. Each heavy chain consists of a heavy chain variable region (VH) and a heavy chain constant region (CH). The heavy chain constant region consists of three domains (CH1, CH2, and CH3). Each light chain consists of a light chain variable region (VL) and a light chain constant region (CL). The light chain constant region consists of one domain, CL. The constant regions of antibodies mediate the binding of immunoglobulins to host tissues or factors, including various cells of the immune system (e.g., effector cells) and the first component (C1q) of the classical complement system. The VH and VL regions can be further subdivided into highly degenerated regions (called complementarity-determining regions (CDRs)) interspersed with more conserved regions called framework regions (FRs). Each VH and VL consists of three CDRs and four FRs arranged in the following order: FR1, CDR1, FR2, CDR2, FR3, CDR3, and FR4, from the amino terminus to the carboxyl terminus. The variable regions (VH and VL) of each heavy/light chain pair form the antibody binding sites. The allocation of amino acids to various regions or domains follows the guidelines of Bethesda M.d., Kabat Sequences of Proteins of Immunological Interest (National Institutes of Health, (1987 and 1991)), or Chothia & Lesk J. Mol. Biol. 1987;196:901-917; Chothia et al. Nature 1989;342:878-883, or the IMGT numbering system definition, see Ehrenmann F, Kaas Q, Lefranc M P. IMGT/3Dstructure-DB and IMGT/DomainGapAlign: a database and a tool for immunoglobulins or antibodies, T cell receptors, MHC, IgSF and MhcSF[J]. Nucleic acids research, 2009; 38(suppl_1): D301-D307.

The term "antibody" is not limited to any particular method of producing antibodies. For example, it includes recombinant antibodies, monoclonal antibodies, and polyclonal antibodies. Antibodies can be different types of antibodies, such as IgG (e.g., IgG1, IgG2, IgG3, or IgG4 subtypes), IgA1, IgA2, IgD, IgE, or IgM antibodies.

As used herein, the terms "monoclonal antibody" and "monoclonal antibody" refer to an antibody or a fragment of an antibody derived from a group of highly homologous antibody molecules-that is, a group of identical antibody molecules except for the possibility of spontaneous natural mutations. Monoclonal antibodies exhibit high specificity for a single epitope on an antigen. Polyclonal antibodies, as opposed to monoclonal antibodies, typically comprise at least two or more different antibodies that typically recognize different epitopes on an antigen. Monoclonal antibodies are usually obtained using the hybridoma technique first reported by Kohler et al. (Köhler G, Milstein C. Continuous cultures of fused cells secreting antibody of predefined specificity[J]. nature, 1975; 256(5517): 495), but can also be obtained using recombinant DNA techniques (see U.S. Patent 4,816,567).

As used herein, the term "humanized antibody" refers to an antibody or antibody fragment obtained by replacing all or part of the CDR region of a human immunoglobulin (receptor antibody) with the CDR region of a non-human antibody (donor antibody), wherein the donor antibody can be a non-human antibody (e.g., mouse, rat, or rabbit) with the expected specificity, affinity, or reactivity. Furthermore, some amino acid residues in the framework region (FR) of the receptor antibody may also be replaced by amino acid residues of the corresponding non-human antibody, or by amino acid residues of other antibodies, to further improve or optimize the antibody's performance. For more detailed information on humanized antibodies, see, for example, Jones et al., Nature 1986; 321:522 525; Reichmann et al., Nature, 1988; 332:323 329; Presta, Curr. Op. Struct. Biol. 1992; 2:593-596; and Clark, Immunol. Today 2000; 21: 397 402. In some cases, the antigen-binding fragment of an antibody is a diabodies, in which the VH and VL domains are expressed on a single polypeptide chain, but the linker is too short to allow pairing between the two domains on the same chain, thus forcing the domain to pair with the complementary domain of another chain and creating two antigen-binding sites (see, for example, Holliger P. et al., Proc. Natl. Acad. Sci. USA 1993; 90:6444 6448 and Poljak R. J. et al., Structure 1994; 2:1121 1123).

As used herein, the term "single-chain fragment variable (single chain fragment variable, ScFv)" refers to a molecule comprising an antibody heavy chain variable region (VH) and an antibody light chain variable region (VL) linked by a linker. The VL and VH domains pair to form a monovalent molecule by enabling them to produce linker pairs as a single polypeptide chain (see, e.g., Bird et al, Science 1988; 242:423 426 and Huston et al, Proc. Natl. Acad. Sci. USA 1988; 85:5879 5883). Such scFv molecules may have a general structure: NH2-VL-linker-VH-COOH or NH2-VH-linker-VL-COOH. Suitable prior art linkers consist of a repeating GGGGS amino acid sequence or a variant thereof. For example, a linker having the amino acid sequence (GGGGS)4 can be used, but variants thereof can also be used (Holliger et al, Proc. Natl. Acad. Sci. USA 1993; 90: 6444-6448). Other linkers that can be used in this invention are described by Alfthan et al, Protein Eng. 1995; 8:725-731, Choi et al, Eur. J. Immunol. 2001; 31: 94-106, Hu et al, Cancer Res. 1996; 56:3055-3061, Kipriyanov et al, J. Mol. Biol. 1999; 293:41-56 and Roovers et al, Cancer Immunology, Immunotherapy, 2001, 50(1): 51-59.

As used herein, the terms "separated" or "isolated" refer to substances obtained artificially from their natural state. If a substance or component is found in nature as a "separated" entity, it may be due to an alteration of its natural environment, the separation of the substance from its natural environment, or both. For example, a certain unseparated polynucleotide or polypeptide may naturally exist in the body of a living animal, and a high-purity identical polynucleotide or polypeptide separated from this natural state is called a separated one. The terms "separated" or "isolated" do not exclude the presence of artificial or synthetic substances, nor do they exclude the presence of other impurities that do not affect the substance's activity.

As used herein, the term "vector" refers to a nucleic acid delivery vehicle into which polynucleotides can be inserted. When a vector enables the expression of a protein encoded by the inserted polynucleotide, it is called an expression vector. Vectors can be introduced into host cells through transformation, transduction, or transfection, allowing the genetic material elements they carry to be expressed in the host cells. Vectors are well-known to those skilled in the art and include, but are not limited to: plasmids; phage particles; Cos plasmids; artificial chromosomes, such as yeast artificial chromosomes (YAC), bacterial artificial chromosomes (BAC), or P1-derived artificial chromosomes (PAC); bacteriophages such as λ phage or M13 phage; and animal viruses. Animal viruses that can be used as vectors include, but are not limited to, retroviruses (including lentiviruses), adenoviruses, adeno-associated viruses, herpesviruses (such as herpes simplex virus), poxviruses, baculoviruses, papillomaviruses, and papillomaviruses (such as SV40). A vector may comprise multiple elements controlling expression, including but not limited to, promoter sequences, transcription initiation sequences, enhancer sequences, selection elements, and reporter genes. Additionally, a vector may include a replication initiation site.

As used herein, the term "host cell" refers to a cell that can be used to introduce a vector, including but not limited to prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, GS cells, COS cells, NSO cells, HeLa cells, BHK cells, HEK 293 cells, or human cells.

As used herein, the term "specific binding" refers to a non-random binding reaction between two molecules, such as the reaction between an antibody and the antigen it targets. In some embodiments, an antibody that specifically binds to an antigen (or an antibody that is specific to an antigen) means that the antibody binds to the antigen with an affinity (KD) of less than about 10⁻⁵ M, for example less than about 10⁻⁶ M, 10⁻⁷ M, 10⁻⁸ M, 10⁻⁹ M, or 10⁻¹⁰ M or less.

As used herein, the term "KD" refers to the dissociation equilibrium constant of a specific antibody-antigen interaction, which describes the binding affinity between the antibody and the antigen. A smaller equilibrium dissociation constant indicates a tighter antibody-antigen binding and a higher affinity between the antibody and the antigen. Typically, antibodies bind antigens (e.g., EGFR proteins) with a dissociation equilibrium constant (KD) of less than about 10⁻⁵ M, such as less than about 10⁻⁶ M, 10⁻⁷ M, 10⁻⁸ M, 10⁻⁹ M, or 10⁻¹⁰ M or even smaller. KD can be determined using methods known to those skilled in the art, such as those using a Fortebio molecular interaction analyzer.

As used herein, the terms "monoclonal antibody" and "monoclonal antibody" have the same meaning and are used interchangeably; the terms "polyclonal antibody" and "polyclonal antibody" have the same meaning and are used interchangeably. Furthermore, in this invention, amino acids are generally represented by single-letter and three-letter abbreviations known in the art. For example, alanine can be represented by A or Ala.

As used herein, the term "pharmaceutically acceptable carrier and/or excipient" means a carrier and/or excipient that is pharmacologically and/or physiologically compatible with the subject and the active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjusters, surfactants, adjuvants, and ionic strength enhancers. For example, pH adjusters include, but are not limited to, phosphate buffers; surfactants include, but are not limited to, cationic, anionic, or nonionic surfactants, such as Tween-80; and ionic strength enhancers include, but are not limited to, sodium chloride.

As used herein, the term "effective amount" means an amount sufficient to achieve, or at least partially achieve, the desired effect. For example, an effective amount for preventing disease (e.g., cancer) means an amount sufficient to prevent, stop, or delay the onset of disease (e.g., cancer); an effective amount for treating disease means an amount sufficient to cure or at least partially stop the disease and its complications in a patient already suffering from the disease. Determining such an effective amount is entirely within the capabilities of those skilled in the art. For example, an effective amount for therapeutic purposes will depend on the severity of the disease to be treated, the overall state of the patient's own immune system, the patient's general characteristics such as age, weight, and sex, the manner of administration of the drug, and other concurrent treatments, etc.

As used herein, when referring to the amino acid sequence of an EGFR protein, it includes the full-length EGFR protein, a fragment comprising the EGFR ECD, and also a fusion protein of the full-length EGFR protein or a fusion protein of the EGFR ECD, such as a fragment fused to a fragment of the Fc protein (mFc or hFc) of mouse or human IgG. However, those skilled in the art will understand that mutations or variations (including, but not limited to, substitutions, deletions, and/or additions) can be naturally generated or artificially introduced into the amino acid sequence of an EGFR protein without affecting its biological function. Therefore, in this invention, the term "added protein" should include all such sequences, including their natural or artificial variants. Furthermore, when describing a sequence fragment of an EGFR protein, it also includes the corresponding sequence fragment from its natural or artificial variants.

As used herein, when referring to the amino acid sequence of a Met protein, it includes the full-length Met protein, a fragment comprising the Met ECD; and also includes fusion proteins of the full-length Met protein or fusion proteins of the Met ECD, such as fragments fused with a fragment of the Fc protein (mFc or hFc) of mouse or human IgG. However, those skilled in the art will understand that mutations or variations (including, but not limited to, substitutions, deletions, and/or additions) can be naturally generated or artificially introduced into the amino acid sequence of a Met protein without affecting its biological function. Therefore, in this invention, the term "added protein" should include all such sequences, including their natural or artificial variants. Furthermore, when describing a sequence fragment of a Met protein, it also includes the corresponding sequence fragment from its natural or artificial variants.

In the present invention, the term "ADCC" refers to antibody-dependent cell-mediated cytotoxicity. The Fab fragment of an antibody binds to antigenic epitopes on virus-infected cells or tumor cells, while its Fc fragment binds to Fc receptors (FcRs) on the surface of killer cells (NK cells, macrophages, etc.), mediating the direct killing of target cells by killer cells.

In the present invention, unless otherwise specified, the terms "first" (e.g., first protein functional region or first medicament product) and "second" (e.g., second protein functional region or second medicament product) are used for distinguishing reference or for clarity of expression, and do not have a typical sequential meaning.

In the present invention, the term "half-molecule monovalent antibody (IgG half molecule, IgG-HM)" refers to an antibody molecule composed of one heavy chain and one light chain of an IgG antibody (e.g., IgG1, IgG2, IgG3, or IgG4), which is a monovalent antibody (see, for example, Feng Yifan et al., Construction and activity analysis of HIV-1 specific monoclonal antibody 2G12 monovalent antibody, Chinese Journal of Virology, May 2015, Vol. 5, No. 3, pp. 171-175).

The following are some of the sequences involved in the present invention (the underlined parts are the CDRs of the Kabat numbering scheme):
(1) E2-M1-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSIYKDSGFDYWGQGT
LVTVSS (SEQ ID NO: 1)
(2) E2-M2-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSVYKDSGFDYWGQG
TLVTVSS (SEQ ID NO: 2)
(3) E2-M5-VH QVQLQESGPGLVKPSQTLSLTCTVSGGSISSGDYYWSWIRQPPGKGLEWIGYIYYSG 
STDYNPSLKSRVTMSVDTSKNQFSLKVNSVTAADTAVYYCARVSVYQDSGFDYWGQG
TLVTVSS (SEQ ID NO: 3)
(4) E2-M12-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSVYEDSRFDYWGQGT
LVTVSS (SEQ ID NO: 4)
(5) E2-M13-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSVYSDSMFDYWGQG
TLVTVSS (SEQ ID NO: 5)
(6) E2-M16-VH 
QVQLQESGPGLVKPSQTLSLTCTVSGGSISNGDYYWSWIRQPPGKGLEWIGYIYYSG
STDYNPSLKSRVTMSVDTSKNQFSLKVNSVTAADTAVYYCARVSLYKDSRFDYWGQGT
LVTVSS (SEQ ID NO: 6)
(7) E2-M17-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSLYKDSRFDYWGQGTL
VTVSS (SEQ ID NO: 7)
(8) E2-M25-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSVYKDSRFDYWGQGT
LVTVSS (SEQ ID NO: 8)
(9) E2-M34-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSIYEDSGFDYWGQGT
LVTVSS (SEQ ID NO: 9)
(10) E2-M38-VH 
QVQLQESGPGLVKPSQTLSLTCTVSGGSISSGDYYWSWIRQPPGKGLEWIGYIYYSG
STDYNPSLKSRVTMSVDTSKNQFSLKVNSVTAADTAVYYCARVSLYEDSGFDYWGQGT
LVTVSS (SEQ ID NO: 10)
(11) E2-LC

EIVMTQSPATLSLSPGERATLSCRASQSVSSWLAWYQQKPGQAPRLLIYGASNRAT

GIPARFSGSGSGTDFTLTISSLEPEDFAVYYCLQVGSTPLTFGGGTKAEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 11)

The sequences of three light chain CDR are as follows:
LCDR1: RASQSVSSWLA (SEQ ID NO: 38)
LCDR2: GASNRAT (SEQ ID NO: 39)
LCDR3: QVGSTPLT (SEQ ID NO: 40)

The light chain variable region (VL) sequence is as follows:

EIVMTQSPATLSLSPGERATLSCRASQSVSSWLAWYQQKPGQAPRLLIYGASNRAT
GIPARFSGSGSGTDFTLTISSLEPEDFAVYYCLQVGSTPLTFGGGTKAEIK (SEQ ID NO:
42)

(12) E2-10-LC-13-91A

EIVMTQSPATLSLSPGERATLSCRASQSVSSWLAWYQQKPGQAPRLLIYGASNRAT
GIPARFSGSGSGTDFTLTISSLEPEDFAVYYCLQAGSTPLTFGGGTKAEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 12)

The sequences of three light chain CDR are as follows:
LCDR1: RASQSVSSWLA (SEQ ID NO: 38)
LCDR2: GASNRAT (SEQ ID NO: 39)
LCDR3: LQAGSTPLT (SEQ ID NO: 41)

The light chain variable region (VL) sequence is as follows:

EIVMTQSPATLSLSPGERATLSCRASQSVSSWLAWYQQKPGQAPRLLIYGASNRAT
GIPARFSGSGSGTDFTLTISSLEPEDFAVYYCLQAGSTPLTFGGGTKAEIK (SEQ ID NO:
43)

(13) E2-10-LC-69

EIVMTQSPATLSLSPGERATLSCRASQSVSSWLAWYQQKPGQAPRLLIYGASNLAK
GIPARFSGSGSGTDFTLTISSLEPEDFAVYYCLQAGSTPLTFGGGTKAEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 13)

The sequences of three light chain CDRs are as follows:
LCDR1: RASQSVSSWLA (SEQ ID NO: 38)
LCDR2: GASNRAT (SEQ ID NO: 39)
LCDR3: LQAGSTPLT (SEQ ID NO: 41)

The light chain variable region (VL) sequence is as follows:

EIVMTQSPATLSLSPGERATLSCRASQSVSSWLAWYQQKPGQAPRLLIYGASNLAK
GIPΛRFSGSGSGTDFTLTISSLEPEDFΛVYYCLQΛGSTPLTFGGGTKΛEIK (SEQ ID NO:
44)

(14) JNJ-372-ML (the italicized portion represents the variable region of the light chain.)

DIQMTQSPSSVSASVGDRVTITCRASQGISNWLAWFQHKPGKAPKLLIYAASSLLSGVPSR
FSGSGSGTDFTLTISSLQPEDFATYYCQQANSFPITFGQGTRLEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC (SEQ ID NO: 14)

The sequences of three light chain CDRs are as follows:
RASQGISNWLA (SEQ ID NO: 51)
AASSLLS (SEQ ID NO: 52)
QQANSFPIT (SEQ ID NO: 53)

The light chain variable region (VL) sequence is as follows:

DIQMTQSPSSVSASVGDRVTITCRASQGISNWLAWFQHKPGKAPKLLIYAASSLLSG
VPSRFSGSGSGTDFTLTISSLQPEDFATYYCQQANSFPITFGQGTRLEIK (SEQ ID NO: 56)

(15) JNJ-372-EL (the italicized portion represents the variable region of the light chain.)

AIQLTQSPSSLSASVGDRVTITCRASQDISSALVWYQQKPGKAPKLLIYDASSLESGVPSRFS
GSESGTDFTLTISSLQPEDFATYYCQQFNSYPLTFGGGTKVEIKRTVAAPSVFIFPPSDEQLKS
GTASVVCLLNNFYPREAKVQWKVDNALQSGNSQESVTEQDSKDSTYSLSSTLTLSKADY
EKHKVYACEVTHQGLSSPVTKSFNRGEC (SEQ ID NO: 15)

(16) M5-HC-K409R (the italicized portion represents the variable region of the heavy chain.)

QLQLQESGPGLVKPSETLSLTCTVSGGSISSSVYYWSWIRQPPGKGLEWIGVIYPSGNTYYS
PSLKSRVTISVDTSKNQFSLKLSSVTAADTAVYYCARTIYDLFDIWGQGTMVTVSSASTKGPSVF
PLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKP
KDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVS
LTCLVKGFYPSDIAVEWESNGQPENNYKTTPPVLDSDGSFFLYSRLTVDKSRWQQGNVF
SCSVMHEALHNHYTQKSLSLSPGK (SEQ ID NO: 16)

The sequences of the three heavy chain CDRs are as follows:
HCDR1: SSVYYWS (SEQ ID NO: 45)
HCDR2: VIYPSGNTYYSPSLKS (SEQ ID NO: 46)
HCDR3: TIYDLFDI (SEQ ID NO: 47)

The VH sequence of the heavy chain variable region is as follows:

QLQLQESGPGLVKPSETLSLTCTVSGGSISSSVYYWSWIRQPPGKGLEWIGVIYPSGN
TYYSPSLKSRVTISVDTSKNQFSLKLSSVTAADTAVYYCARTIYDLFDIWGQGTMVTVSS
(SEQ ID NO: 54)

The heavy chain constant region sequence is as follows:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFFLYSRLTVD
KSRWQQGNVFSCSVMHEALHNHYTQKSLSLSPGK (SEQ ID NO: 78)

(17) E2mut34-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSIYEDSGFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 17)

The heavy chain constant region sequence is as follows:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
SREEMTKNQVSLTCLVKGFYPSDIAVEWESNGQPENNYKTTPPVLDSDGSFLLYSKLTV
DKSRWQQGNVFSCSVMHEALHNHYTQKSLSLSPGK (SEQ ID NO: 79)

(18) E2mut34-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSIYEDSGFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 18)

The constant region sequence is as follows:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVCTLPP
SREEMTKNQVSLSCAVKGFYPSDIAVEWESNGQPENNYKTTPPVLDSDGSFFLVSKLTV
DKSRWQQGNVFSCSVMHEALHNHYTQKSLSLSPGK (SEQ ID NO: 80)

(19) E2mut34-HC-holeRF (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSIYEDSGFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNRFTQKSLSLSPGK (SEQ ID NO: 19)

The constant region sequence is as follows:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELL

GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVCTLPP
SREEMTKNQVSLSCAVKGFYPSDIAVEWESNGQPENNYKTTPPVLDSDGSFFLVSKLTV
DKSRWQQGNVFSCSVMHEALHNRFTQKSLSLSPGK (SEQ ID NO: 81)

(20) M5-HC-knob (the italicized portion represents the variable region of the heavy chain.)

QLQLQESGPGLVKPSETLSLTCTVSGGSISSSVYYWSWIRQPPGKGLEWIGVIYPSGNTYYS
PSLKSRVTISVDTSKNQFSLKLSSVTAADTAVYYCARTIYDLFDIWGQGTMVTVSSASTKGPSVF
PLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPELLGGPSVFLFPPKP
KDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPCREEMTKNQVS
LWCLVKGFYPSDIAVEWESNGQPENNYKTTPPVLDSDGSFFLYSKLTVDKSRWQQGNV
FSCSVMHEALHNHYTQKSLSLSPGK (SEQ ID NO: 20)

The constant region sequence is as follows:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELL
GGPSVFLFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQVYTLPP
CREEMTKNQVSLWCLVKGFYPSDIAVEWESNGQPENNYKTTPPVLDSDGSFFLYSKLTV
DKSRWQQGNVFSCSVMHEALHNHYTQKSLSLSPGK (SEQ ID NO: 82)

(21) JNJ-372-MH (the italicized portion represents the variable region of the heavy chain.)

QVQLVQSGAEVKKPGASVKVSCETSGYTFTSYGISWVRQAPGHGLEWMGWISAYNGYTN
YAQKLQGRVTMTTDTSTSTAYMELRSLRSDDTAVYYCARDLRGTNYFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSRLTVDKSRWQ
QGNVFSCSVMHEALHNHYTQKSLSLSPGK (SEQ ID NO: 21)

The sequences of the three heavy chain CDRs are as follows:
SYGIS (SEQ ID NO: 48)
WISAYNGYTNYAQKLQG (SEQ ID NO: 49)
DLRGTNYFDY (SEQ ID NO: 50)

The variable region VH of the heavy chain is as follows:

QVQLVQSGAEVKKPGASVKVSCETSGYTFTSYGISWVRQAPGHGLEWMGWISAYN
GYTNYΛQKLQGRVTMTTDTSTSTΛYMELRSLRSDDTΛVYYCΛRDLRGTNYFDYWGQG
TLVTVSS (SEQ ID NO: 55)

(22) JNJ-372-EH (the italicized portion represents the variable region of the heavy chain.)

QVQLVESGGGVVQPGRSLRLSCAASGFTFSTYGMHWVRQAPGKGLEWVAVIWDDGSYK
YYGDSVKGRFTISRDNSKNTLYLQMNSLRAEDTAVYYCARDGITMVRGVMKDYFDYWGQGTL
VTVSSASTKGPSVFPLAPSSKSTSGGTAALGCLVKDYFPEPVTVSWNSGALTSGVHTFPAV
LQSSGLYSLSSVVTVPSSSLGTQTYICNVNHKPSNTKVDKRVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFLLYSKLTV
DKSRWQQGNVFSCSVMHEALHNHYTQKSLSLSPGK (SEQ ID NO: 22)

(23) EGFR-his-tag

MRPSGTAGAALLALLAALCPASRALEEKKVCQGTSNKLTQLGTFEDHFLSLQRMFN
NCEVVLGNLEITYVQRNYDLSFLKTIQEVAGYVLIALNTVERIPLENLQIIRGNMYYENSY
ALAVLSNYDANKTGLKELPMRNLQEILHGAVRFSNNPALCNVESIQWRDIVSSDFLSNM
SMDFQNHLGSCQKCDPSCPNGSCWGAGEENCQKLTKIICAQQCSGRCRGKSPSDCCHNQ
CAAGCTGPRESDCLVCRKFRDEATCKDTCPPLMLYNPTTYQMDVNPEGKYSFGATCVK
KCPRNYVVTDHGSCVRACGADSYEMEEDGVRKCKKCEGPCRKVCNGIGIGEFKDSLSIN
ATNIKHFKNCTSISGDLHILPVAFRGDSFTHTPPLDPQELDILKTVKEITGFLLIQAWPENR
TDLHAFENLEIIRGRTKQHGQFSLAVVSLNITSLGLRSLKEISDGDVIISGNKNLCYANTIN
WKKLFGTSGQKTKIISNRGENSCKATGQVCHALCSPEGCWGPEPRDCVSCRNVSRGREC
VDKCNLLEGEPREFVENSECIQCHPECLPQAMNITCTGRGPDNCIQCAHYIDGPHCVKTC
PAGVMGENNTLVWKYADAGHVCHLCHPNCTYGCTGPGLEGCPTNGPKIPSHHHHHH(S
EQ ID NO: 23)

(24) Met-his-tag

MKAPAVLAPGILVLLFTLVQRSNGECKEALAKSEMNVNMKYQLPNFTAETPIQNVI
LHEHHIFLGATNYIYVLNEEDLQKVAEYKTGPVLEHPDCFPCQDCSSKANLSGGVWKDN
INMALVVDTYYDDQLISCGSVNRGTCQRHVFPHNHTADIQSEVHCIFSPQIEEPSQCPDCV
VSALGAKVLSSVKDRFINFFVGNTINSSYFPDHPLHSISVRRLKETKDGFMFLTDQSYIDV

LPEFRDSYPIKYVHAFESNNFIYFLTVQRETLDAQTFHTRIIRFCSINSGLHSYMEMPLECIL
TEKRKKRSTKKEVFNILQAAYVSKPGAQLARQIGASLNDDILFGVFAQSKPDSAEPMDRS
AMCAFPIKYVNDFFNKIVNKNNVRCLQHFYGPNHEHCFNRTLLRNSSGCEARRDEYRTE
FTTALQRVDLFMGQFSEVLLTSISTFIKGDLTIANLGTSEGRFMQVVVSRSGPSTPHVNFL
LDSHPVSPEVIVEHTLNQNGYTLVITGKKITKIPLNGLGCRHFQSCSQCLSAPPFVQCGWC
HDKCVRSEECLSGTWTQQICLPAIYKVFPNSAPLEGGTRLTICGWDFGFRRNNKFDLKKT
RVLLGNESCTLTLSESTMNTLKCTVGPAMNKHFNMSIIISNGHGTTQYSTFSYVDPVITSI
SPKYGPMAGGTLLTLTGNYLNSGNSRHISIGGKTCTLKSVSNSILECYTPAQTISTEFAVK
LKIDLANRETSIFSYREDPIVYEIHPTKSFISGGSTITGVGKNLNSVSVPRMVINVHEAGRN
FTVACQHRSNSEIICCTTPSLQQLNLQLPLKTKAFFMLDGILSKYFDLIYVHNPVFKPFEKP
VMISMGNENVLEIKGNDIDPEAVKGEVLKVGNKSCENIHLHSEAVLCTVPNDLLKLNSE
LNIEWKQAISSTVLGKVIVQPDQNFTHHHHHH(SEQ ID NO: 24)

(25)E2-10-LC-13-91Q

EIVMTQSPATLSLSPGERATLSCRASQSVSSWLAWYQQKPGQAPRLLIYGASNRAT
GIPARFSGSGSGTDFTLTISSLEPEDFAVYYCLQQGSTPLTFGGGTKAEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC (SEQ ID NO: 57)

The sequences of three light chain CDRs are as follows:
LCDR1: RASQSVSSWLA (SEQ ID NO: 38)
LCDR2: GASNRAT (SEQ ID NO: 39)
LCDR3: LQQGSTPLT (SEQ ID NO: 76)

The light chain variable region (VL) sequence is as follows:

EIVMTQSPATLSLSPGERATLSCRASQSVSSWLAWYQQKPGQAPRLLIYGASNRAT
GIPARFSGSGSGTDFTLTISSLEPEDFAVYYCLQQGSTPLTFGGGTKAEIK (SEQ ID NO:
77)

(26)E2mut1-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSIYKDSGFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY

RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 58)

(27)E2mut2-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYKDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 59)

(28)E2mut5-HC-F405L (the italicized portion represents the variable region of the heavy chain)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYQDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 60)

(29)E2mut12-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYEDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ

QGNVFSCSVMHEALHNHYTQKSLSLSPGK (SEQ ID NO: 61)

(30)E2mut13-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYSDSMFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 62)

(31)E2mut16-HC-F405L (the italicized portion represents the variable region of the heavy chain.)

QVQLQESGPGLVKPSQTLSLTCTVSGGSISNGDYYWSWIRQPPGKGLEWIGYIYYSGSTDY
NPSLKSRVTMSVDTSKNQFSLKVNSVTAADTAVYYCARVSLYKDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 63)

(32)E2mut17-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSLYKDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 64)

(33)E2mut25-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYKDSRFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 65)

(34)E2mut38-HC-F405L (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSLYEDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFLLYSKLTVDKSRWQ
QGNVFSCSVMHEALHNHYTQKSLSLSPGK (SEQ ID NO: 66)

(35)E2mut1-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSIYKDSGFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 67)

(36)E2mut2-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYKDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 68)

(37)E2mut5-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYQDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 69)

(38)E2mut12-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYEDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 70)

(39)E2mut13-HC-hole (the italicized portion represents the variable region of the heavy chain)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYSDSMFDYWGQGTLVTVSSAST

KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 71)

(40)E2mut16-HC-hole (the italicized portion represents the variable region of the heavy chain.)

QVQLQESGPGLVKPSQTLSLTCTVSGGSISNGDYYWSWIRQPPGKGLEWIGYIYYSGSTDY
NPSLKSRVTMSVDTSKNQFSLKVNSVTAADTAVYYCARVSLYKDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 72)

(41)E2mut17-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSLYKDSRFDYWGQGTLVTVSSASTK
GPSVFPLAPSSKSTSGGTAALGCLVKDYFPEPVTVSWNSGALTSGVHTFPAVLQSSGLYS
LSSVVTVPSSSLGTQTYICNVNHKPSNTKVDKRVEPKSCDKTHTCPPCPAPELLGGPSVFL
FPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 73)

(42)E2mut25-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSVYKDSRFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF

LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 74)

(43)E2mut38-HC-hole (the italicized portion represents the variable region of the heavy chain.)

QVQLQESGPGLVKPSQTLSLTCTVSGGSISSGDYYWSWIRQPPGKGLEWIGYIYYSGSTDY
NPSLKSRVTMSVDTSKNQFSLKVNSVTAADTAVYYCARVSLYEDSGFDYWGQGTLVTVSSAST
KGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVF
LFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTVDKSRWQ
QGNVFSCSVMHEALHNHYTQKSLSLSPGK (SEQ ID NO: 75)

### Beneficial effects of the invention

The bispecific antibody of the present invention achieves one or more of the following technical effects (1)-(5):
(1) It has good affinity and/or specificity with the target EGFR and/or Met;
(2) It has high ADCC activity;
(3) It has high endocytosis rate;
(4) It has good antitumor activity;
(5) The production process is simple and the cost is low.

The embodiments of the present invention will be described in detail below with reference to examples. However, those skilled in the art will understand that the following examples are for illustrative purposes only and should not be considered as limiting the scope of the invention. Unless otherwise specified in the examples, conventional conditions or conditions recommended by the manufacturer are followed. Reagents or instruments whose manufacturers are not specified are all commercially available conventional products.

Preparation example 1: sequence design and preparation of anti-EGFR monoclonal antibody.

Ten anti-EGFR monoclonal antibodies were designed and screened, named E2-M1, E2-M2, E2-M5, E2-M12, E2-M13, E2-M16, E2-M17, E2-M25, E2-M34, and E2-M38, respectively. The amino acid sequences of the heavy chain variable region of these ten anti-EGFR monoclonal antibodies are shown in SEQ ID NO: 1-10, and the heavy chain constant region is selected from the amino acid sequences as shown in SEQ ID NO: 79, 80, and 81. The amino acid sequences of the light chain are shown in SEQ ID NO: 11. HCDR and LCDR were determined using the Kabat numbering system and are underlined, as shown in Table A below.

**Table A**

| Name of Antibody | HCDR1 | HCDR2 | HCDR3 |
|---|---|---|---|
| E2-M1 | SGDYYWS (SEQ ID NO: 25) | YIYYSGSTDYNPSLKS (SEQ ID NO: 27) | ARVSIYKDSGFDY (SEQ ID NO: 28) |
| E2-M2 | SGDYYWS | YIYYSGSTDYNPSLKS | ARVSVYKDSGFDY (SEQ ID NO: 29) |
| E2-M5 | SGDYYWS | YIYYSGSTDYNPSLKS | ARVSVYQDSGFDY (SEQ ID NO: 30) |
| E2-M12 | SGDYYWS | YIYYSGSTDYNPSLKS | ARVSVYEDSRFDY (SEQ ID NO: 31) |
| E2-M13 | SGDYYWS | YIYYSGSTDYNPSLKS | ARVSVYSDSMFDY (SEQ ID NO: 32) |
| E2-M16 | NGDYYWS (SEQ ID NO: 26) | YIYYSGSTDYNPSLKS | ARVSLYKDSRFDY (SEQ ID NO: 33) |
| E2-M17 | SGDYYWS | YIYYSGSTDYNPSLKS | ARVSLYKDSRFDY (SEQ ID NO: 33) |
| E2-M25 | SGDYYWS | YIYYSGSTDYNPSLKS | ARVSVYKDSRFDY (SEQ ID NO: 34) |
| E2-M34 | SGDYYWS | YIYYSGSTDYNPSLKS | ARVSIYEDSGFDY (SEQ ID NO: 35) |
| E2-M38 | SGDYYWS | YIYYSGSTDYNPSLKS | ARVSLYEDSGFDY (SEQ ID NO: 36) |

The structure of HCDR3 in the above antibody is shown in SEQ ID NO: 37 below: ARVSX1YX2DSX3FDY (SEQ ID NO: 37); wherein X1 is selected from amino acids I, V and L, X2 is selected from amino acids K, Q, E and S, and X3 is selected from amino acids G, R and M.

### Example 1: Octet Red 96e assay to determine the affinity of the E2 mutant for EGFR

The kinetic parameters of the binding of the 10 previously prepared anti-EGFR monoclonal antibodies to the antigen EGFR-his-tag were determined using the protein A capture method. Monoclonal antibodies at a concentration of 1 µg/ml and control antibody JNJ-372-E-LF (prepared in Preparation Example 2 below) were bound to a Protein A probe (Cat No: 18-5010; lot: 2001131). The antigen EGFR-his-tag was diluted 2-fold from 50 nM downwards in 1X Fortebio working solution (1X PBS + 0.05% Tween 20) to create four concentration gradients for binding with the antibody, and then dissociated in 1X Fortebio working solution.

The kinetic parameters of E2 mutant binding to EGFR-his-tag are shown in Table B.

**Table B**

| Name of Antibody | K_{D} (M) | ka (1/Ms) | kd (1/s) |
|---|---|---|---|
| JNJ-372-E-LF | 4.34E-09 | 2.47E+05 | 1.07E-03 |
| E2-M1 | 2.16E-09 | 4.11E+05 | 8.88E-04 |
| E2-M2 | 1.92E-09 | 4.19E+05 | 8.05E-04 |
| E2-M5 | 8.86E-09 | 6.25E+04 | 5.54E-04 |
| E2-M12 | 8.01E-09 | 4.75E+04 | 3.80E-04 |
| E2-M13 | 8.64E-09 | 5.24E+04 | 4.53E-04 |
| E2-M16 | 9.31E-09 | 4.88E+04 | 4.54E-04 |
| E2-M17 | 9.04E-09 | 5.02E+04 | 4.54E-04 |
| E2-M25 | 1.52E-09 | 2.79E+05 | 4.24E-04 |
| E2-M34 | 1.13E-09 | 4.06E+05 | 4.59E-04 |
| E2-M38 | 2.15E-09 | 4.04E+05 | 8.66E-04 |

The results showed that the affinity of all 10 E2 mutants reached the nM level. Among them, E2-M1, E2-M2, E2-M25, E2-M34, and E2-M38 had better affinity than the control antibody JNJ-372-E-LF, with E2-M34 having the highest affinity.

Preparation example 2: design and preparation of the first batch of EGFR×Met bispecific antibodies

### 1. Construction of bispecific antibody molecules

From the 10 high-affinity EGFR antibody heavy chain variable regions obtained in Preparation Example 1, E2mut34 was selected to construct the heavy chain of the EGFR-terminal parent antibody for the EGFR×Met bispecific antibody (see SEQ ID NO: 17-19). Alternatively, referring to the heavy chain of a published Met antibody (SEQ ID NO: 410 in CN105705519A), modifications were made to the constant region according to the needs of preparing the bispecific antibody, and this was used as the heavy chain of the Met-terminal parent antibody for the EGFR×Met bispecific antibody (see SEQ ID NO: 16 and 20). E2-10-LC-13-91A or E2-10-LC-69 was used as the common light chain.

The DNA sequences of four light chains E2-10-LC-13-91A, E2-10-LC-69, JNJ-372-ML, and JNJ-372-EL from Table 1 were synthesized by Tsingke Biotechnology. The DNA was digested with SapI (purchased from NEB, catalog number: R0569L) and ligated into the HXT2 vector (a self-modified vector of Junshi Biosciences, derived from pTT5) to obtain four expression vectors, named HXT2-E2-10-LC-13-91A, HXT2-E2-10-LC-69, HXT2-JNJ-372-ML, and HXT2-JNJ-372-EL, respectively.

HXT2-JNJ-372-ML is the Met-terminal antibody light chain of Amivantamab, and HXT2-JNJ-372-EL is the EGFR-terminal antibody light chain of Amivantamab (sequence source: IMGT website).

The DNA sequences of the seven heavy chains M5-HC-K409R, E2mut34-HC-F405L, E2mut34-HC-hole, E2mut34-HC-holeRF, M5-HC-knob, JNJ-372-MH, and JNJ-372-EH from Table 1 were synthesized by Tsingke Biotechnology. These sequences were then digested with SapI (purchased from NEB, catalog number: R0569L) and ligated into the HXT1S vector (a modified vector developed by Junshi Biosciences, derived from pTT5), resulting in seven expression vectors named HXT1S-E2mut34-HC-F405L, HXT1S-M5-HC-K409R, HXT1S-E2mut34-HC-hole, HXT1S-E2mut34-HC-holeRF, and HXT1S-M5-HC-knob, respectively. HXT1S-JNJ-372-MH and HXT1S-JNJ-372-EH.

HXT1S-JNJ-372-MH is the Met-terminal antibody heavy chain of Amivantamab, and HXT1S-JNJ-372-EH is the EGFR-terminal antibody heavy chain of Amivantamab (sequence source: IMGT website).

**Table 1: Name of heavy chain and light chain**

| Name of heavy chain | SEQ ID NO: | Name of light chain | SEQ ID NO: |
|---|---|---|---|
| M5-HC-K409R | 16 | E2-10-LC-13-91A | 12 |
| E2mut34-HC-F405L | 17 | E2-10-LC-69 | 13 |
| E2mut34-HC-hole | 18 | JNJ-372-ML | 14 |
| E2mut34-HC-holeRF | 19 | JNJ-372-EL | 15 |
| M5-HC-knob | 20 | / | / |
| JNJ-372-MH | 21 | / | / |
| JNJ-372-EH | 22 | / | / |

### 2. Expression and purification of transiently expressed protein

The required heavy chains, light chains and expression vectors are shown in Table 2. Specifically:
E2mut34-91A-F405L-LF and M5-91A-K409R-LF are used for the subsequent preparation of bispecific antibodies (in vitro recombination method);
E2mut34-69-F405L-LF and M5-69-K409R-LF are used for the subsequent preparation of bispecific antibodies (in vitro recombination method);
JNJ-372-M-LF and JNJ-372-E-LF are used for the subsequent preparation of bispecific antibodies (in vitro recombination method).

ALK101-2 and ALK101-4 are used to prepare bispecific antibodies by adopting a common light chain and the knob-into-hole (knob-into-hole) technique (with reference to WO1996027011A1).

**Table 2: Combinations of heavy chains and light chains and corresponding expression vectors**

| Name of Antibody | Heavy Chain of EGFR Antibody | Heavy Chain of Met Antibody | Light Chain of Antibody | Transfection Ratio |
|---|---|---|---|---|
| E2mut34-91A-F405L-LF | E2mut34-HC-F405L (SEQ ID NO: 17) | / | E2-10-LC-13-91A(SEQ ID NO: 12) | 1:1 |
| M5-91A-K409R-LF | / | M5-HC-K409R(SEQ ID NO: 16) | E2-10-LC-13-91A(SEQ ID NO: 12) | 1:1 |
| E2mut34-69-F405L-LF | E2mut34-HC-F405L (SEQ ID NO: 17) | / | E2-10-LC-69 (SEQ ID NO: 13) | 1:1 |
| M5-69-K409R-LF | / | M5-HC-K409R(SEQ ID NO: 16) | E2-10-LC-69 (SEQ ID NO: 13) | 1:1 |
| JNJ-372-M-LF | / | JNJ-372-MH (SEQ ID NO: 21) | JNJ-372-ML (SEQ ID NO: 14) | 1:1 |
| JNJ-372-E-LF | JNJ-372-EH (SEQ ID NO: 22) | / | JNJ-372-EL (SEQ ID NO: 15) | 1:1 |
| ALK101-2 | E2mut34-HC-hole (SEQ ID NO: 18) | M5-HC-knob (SEQ ID NO: 20) | E2-10-LC-13-91A(SEQ ID NO: 12) | 1:1:1 |
| ALK101-4 | E2mut34-HC-holeRF (SEQ ID NO: 19) | M5-HC-knob (SEQ ID NO: 20) | E2-10-LC-69(SEQ ID NO: 13) | 1:1:1 |

### 2.1 Expression of bispecific antibody molecules

CHO-K1 cells (owned by Suzhou Junmeng) were counted. When the cell density reached 2-6*10⁶/ml, they were passaged and expanded using CD CHO medium (purchased from Thermofisher, catalog number: 12490-001). The cell density was diluted to 1.8-2.5*10⁶/ml one day before transfection, and transfected the next day when the cell density reached approximately 3.5-5.0*10⁶/ml. One-tenth of the transfection volume of CD CHO medium was added, followed by 1-2 µg/ml of plasmid (prepared by the company) according to the combination shown in Table 2. Finally, 3-14 µg/ml of PEI (purchased from Polysciences, catalog number: 24765-1) was added, mixed well, and incubated at room temperature. The transfection mixture was then slowly added to the pre-treated cells, mixing constantly. The transfected mixture was placed in a shaker for culture. On the first day after transfection, 6% glycan modifier (purchased from OPM, Cat. No.: R170026) was added, together with 4% Cell Boost 7a (purchased from Hyclone, Cat. No.: SH31026.05) and 0.4% Cell Boost 7b (purchased from Hyclone, Cat. No.: SH31027.04CN). Feeding was then performed every two days, and samples were harvested 5-9 days after transfection.

### 3. In vitro recombination and purification

### 3.1 Affinity capture of bispecific antibody molecules

After culture, the cells were centrifuged at 1000g for 5 min using a floor centrifuge (ThermoFisher, R404A) to discard the precipitate, and then centrifuged at 8000g for 30 min to collect the cell supernatant. The supernatant was then aseptically filtered using a 0.22µm filter cup (JET, FPE-214-000). Purification was performed using a protein purification system (GE, AKTA Avant). The Mabselect Sure LX column (Cytiva, 17547403) was equilibrated with PBS equilibration buffer (Wuxi Aorui Dongyuan Biotechnology Co., Ltd., ZLI-9061). After sample loading, the sample was first rinsed with affinity chromatography elution buffer A (pH 5.5, 45 mM acetate-sodium acetate + 1 M sodium chloride system), then rinsed with elution buffer B (pH 5.5, 45 mM acetate-sodium acetate system), and finally eluted with affinity elution buffer (pH 3.6, 10 mM acetate-sodium acetate buffer). The sample was then neutralized with 1 M Tris buffer (purchased from Merck, catalog number: E300016981946) and the pH was adjusted to 5.5-6 for the next step of in vitro recombination.

### 3.2 In vitro recombinant of bispecific antibody molecules

Referring to the technical solution disclosed in WO2011131746, bispecific antibodies are prepared using Fab arm exchange technology, as follows:
After affinity, the two mother antibodies were concentrated and transferred to PBS equilibration buffer (purchased from Wuxi Aorui Dongyuan Biotechnology Co., Ltd., ZLI-9061) to set the protein concentration at 1±0.05mg/ml.

Preparation of 750 mM Cysteamine hydrochloride (purchased from VETEC, V900342-25G) stock Solution: 2.556 g of cysteamine hydrochloride is added to 14 mL of PBS, and the volume is adjusted to 30 mL with PBS. The mixture is then filtered through a 0.22 µm filter (purchased from Sartorius, Cat. No. 16541-K), and finally wrapped in aluminum foil and stored away from light.

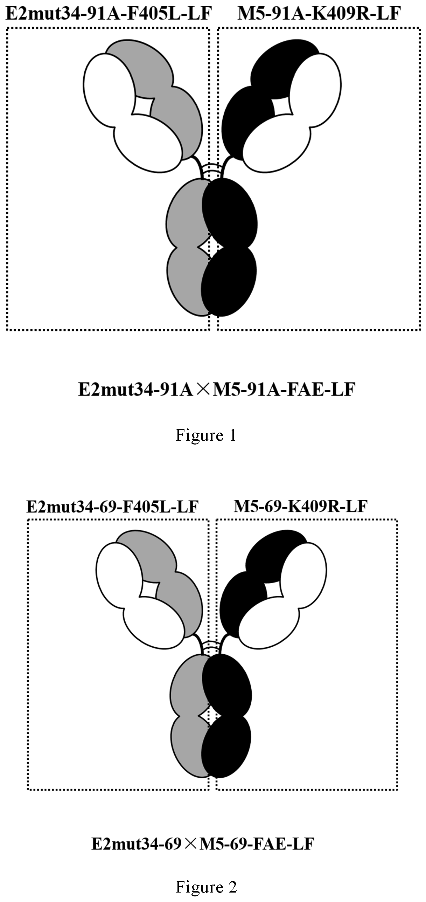

Construction of the incubation system: the maternal antibody processed as described above was taken and, as shown in Table 3, added to a 15ml centrifuge tube (Genemore, G3210015) at a molar ratio of M:E = 1:1.2. 2ml of M maternal antibody, 2.4ml of E maternal antibody, and 0.489ml of 750mM Cysteamine hydrochloride were added. The constructed incubation system was sealed with aluminum foil and incubated in a 31°C water bath (Shanghai Jing Hong Laboratory Instrument Co., Ltd., DK-S28) for 3 hours, protected from light. After incubation, the solution was concentrated and replaced with PBS equilibration buffer, and then incubated at room temperature in the dark for 16-24 hours. The final bispecific antibody molecular structures prepared by the in vitro recombinant method are shown in Figures 1 to 3.

**Table 3: In vitro recombinant combinations of bispecific antibodies**

| Name of the bispecific antibody | Parent antibody M (anti-Met antibody) | Parent antibody E (anti-EGFR antibody) | molar ratio M:E |
|---|---|---|---|
| E2mut34-91A×M5-91A-FAE-LF | M5-91A-K409R-LF | E2mut34-91A-F405L-LF | 1:1.2 |
| E2mut34-69×M5-69-FAE-LF | M5-69-K409R-LF | E2mut34-69-F405L-LF | 1:1.2 |
| JNJ-372(Amivantamab as a control) | JNJ-372-M-LF | JNJ-372-E-LF | 1:1.2 |

### 3.3 Purification of bispecific antibody molecules

The Capto^{™} MMC Impres (Cytiva, 17371602) packing material was selected for purification. Pre-equilibration was performed using washing buffer (pH 7.5, 20 mM Tris-HCl + 1 M NaCl buffer system), followed by equilibration using equilibration buffer (pH 7.5, 20 mM Tris-HCl system). After sample loading, equilibration was performed with 3-6 column volumes of equilibration buffer, and finally, linear elution was performed with elution buffer (pH 7.5, 20 mM Tris-HCl + 1 M NaCl buffer system), collecting the target protein. The three bispecific antibodies shown in Table 3 are thus obtained.

### Example 2: ELISA binding assay

### 4.1 EGFR ELISA binding assay

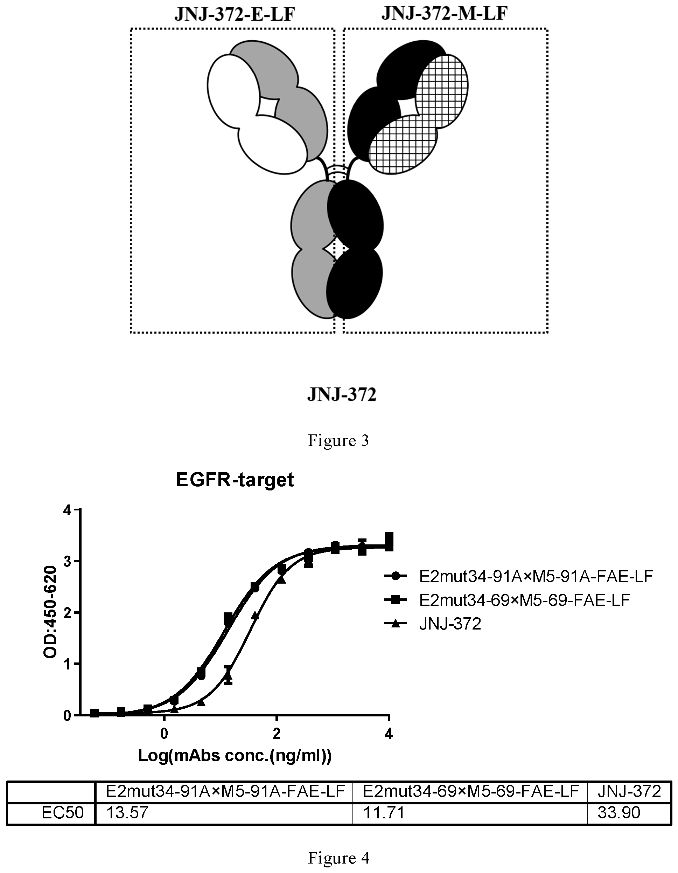

EGFR-his-tag (purchased from Suzhou Junmeng, Batch No.: 20210803; sequence source: UniProt website) was diluted to 5.0 µg/ml with PBS (purchased from Hyclone, Cat. No.: SH30256.01), added to an ELISA plate at 100 µl/well, and incubated for 60 minutes at 37°C in a constant temperature incubator for coating. After washing, 200 µl/well of 2% BSA (purchased from Sigma, Cat. No.: B2064) was added, followed by incubation at 37°C for 60 minutes and washing. Samples were diluted to 10 µg/ml with 2% BSA and serially diluted 3-fold to 0.056 ng/ml, then added to the plate at 100 µl/well and incubated at 37°C for 60 minutes, followed by washing. Horseradish peroxidase (HRP)-conjugated goat anti-human IgG (Fc-specific) antibody (purchased from Sigma, Cat. No.: A0170) was diluted 5000-fold with 2% BSA, added at 100 µl/well, incubated at 37°C for 60 minutes, and washed. Chromogenic substrate TMB (purchased from Sigma, Cat. No.: T2885) at 0.1 mg/ml was added at 100 µl/well avoiding bubbles, and developed at 37°C in the dark for 10 minutes. Finally, 2 M hydrochloric acid solution was added at 100 µl/well to stop the reaction, avoiding bubbles. The absorbance was read using a microplate reader within 10 minutes (detection wavelength: 450 nm; reference wavelength: 620 nm), and the EC50 values were fitted using a four-parameter logistic (4PL) regression model. As shown in Figure 4, the EC50 values of E2mut34-91A×M5-91A-FAE-LF, E2mut34-69×M5-69-FAE-LF, and JNJ-372 for binding to EGFR were 13.57 ng/ml, 11.71 ng/ml, and 33.90 ng/ml, respectively. The binding affinities of E2mut34-91A×M5-91A-FAE-LF and E2mut34-69×M5-69-FAE-LF to EGFR were superior to that of JNJ-372.

### 4.2 Met ELISA binding assay

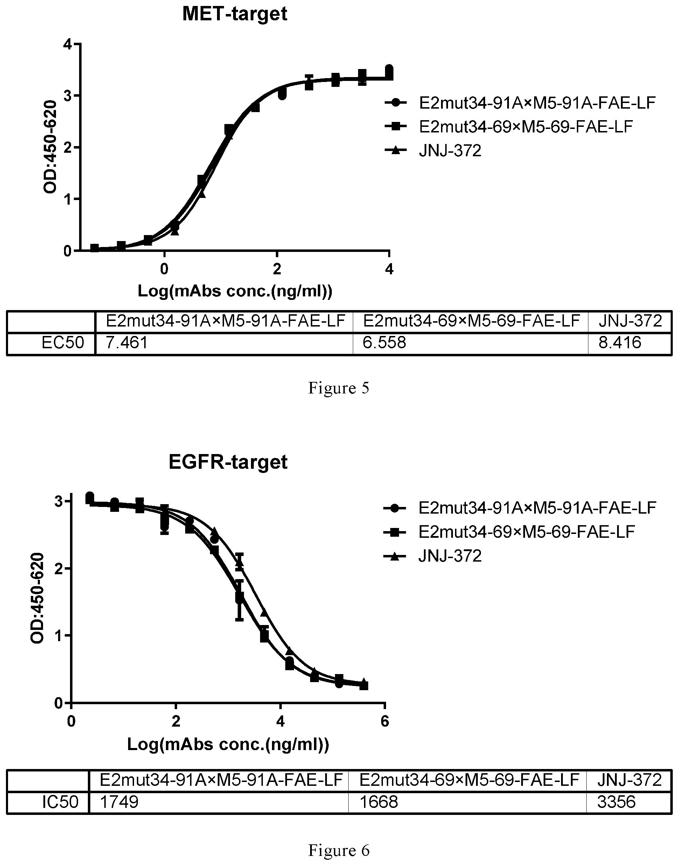

Met-his-tag (purchased from Suzhou Junmeng, Batch No.: 20220404; sequence source: UniProt website) was diluted to 2.0 µg/ml with PBS (purchased from Hyclone, Cat. No.: SH30256.01), added to an ELISA plate at 100 µl/well, and incubated for 60 minutes at 37°C in a constant temperature incubator for coating. After washing, 200 µl/well of 2% BSA (purchased from Sigma, Cat. No.: B2064) was added, followed by incubation at 37°C for 60 minutes and washing. Samples were diluted to 10 µg/ml with 2% BSA and serially diluted 3-fold to 0.056 ng/ml, then added to the plate at 100 µl/well and incubated at 37°C for 60 minutes. After washing, horseradish peroxidase (HRP)-conjugated goat anti-human IgG (Fc-specific) antibody (purchased from Sigma, Cat. No.: A0170) was diluted 5000-fold with 2% BSA, added at 100 µl/well, incubated at 37°C for 60 minutes, and washed. Chromogenic substrate TMB (purchased from Sigma, Cat. No.: T2885) at 0.1 mg/ml was added at 100 µl/well avoiding bubbles, and developed at 37°C in the dark for 10 minutes. Finally, 2 M hydrochloric acid solution was added at 100 µl/well to terminate the reaction, avoiding bubbles. The absorbance was read using a microplate reader within 10 minutes (detection wavelength: 450 nm; reference wavelength: 620 nm), and the EC50 values were fitted using a four-parameter logistic (4PL) regression model. As shown in Figure 5, the EC50 values of E2mut34-91A×M5-91A-FAE-LF, E2mut34-69×M5-69-FAE-LF, and JNJ-372 for binding to Met were 7.461 ng/ml, 6.558 ng/ml, and 8.416 ng/ml, respectively. The binding affinities of E2mut34-91A×M5-91A-FAE-LF and E2mut34-69×M5-69-FAE-LF to Met were comparable to that of JNJ-372.

### Example 3: ELISA blocking assay

### 5.1 EGFR ELISA blocking assay

EGFR-his-tag (purchased from Suzhou Junmeng, Batch No.: 20210803) was diluted to 2.0 µg/ml with PBS (purchased from Hyclone, Cat. No.: SH30256.01), added to an ELISA plate at 100 µl/well, and incubated for 60 minutes at 37°C in a constant temperature incubator for coating. After washing, 200 µl/well of 2% BSA (purchased from Sigma, Cat. No.: B2064) was added, followed by incubation at 37°C for 60 minutes and washing. EGF mFc (purchased from Acro, Cat. No.: EGF-H525b) was diluted to 3.0 µg/ml with 2% BSA. Samples were diluted to 400 µg/ml with the 3.0 µg/ml EGF mFc solution, then serially diluted 3-fold to 0.0023 µg/ml, added to the ELISA plate at 100 µl/well, and incubated at 37°C for 60 minutes. After washing, horseradish peroxidase (HRP)-conjugated anti-mouse Fc antibody (purchased from Sigma, Cat. No.: A2554) was diluted 5000-fold with 2% BSA, added at 100 µl/well, incubated at 37°C for 60 minutes, and washed. Chromogenic substrate TMB (purchased from Sigma, Cat. No.: T2885) at 0.1 mg/ml was added at 100 µl/well avoiding bubbles, and developed at 37°C in the dark for 10 minutes. Finally, 2 M hydrochloric acid solution was added at 100 µl/well to terminate the reaction, avoiding bubbles. The absorbance was read using a microplate reader within 10 minutes (detection wavelength: 450 nm; reference wavelength: 620 nm), and IC₅₀ values were fitted using a four-parameter logistic (4PL) regression model. As shown in Figure 6, the IC₅₀ values of E2mut34-91A×M5-91A-FAE-LF, E2mut34-69×M5-69-FAE-LF, and JNJ-372 for blocking EGFR were 1749 ng/ml, 1668 ng/ml, and 3356 ng/ml, respectively. The blocking abilities of E2mut34-91A×M5-91A-FAE-LF and E2mut34-69×M5-69-FAE-LF against EGFR were slightly superior to that of JNJ-372.

### 5.2 Met ELISA blocking assay

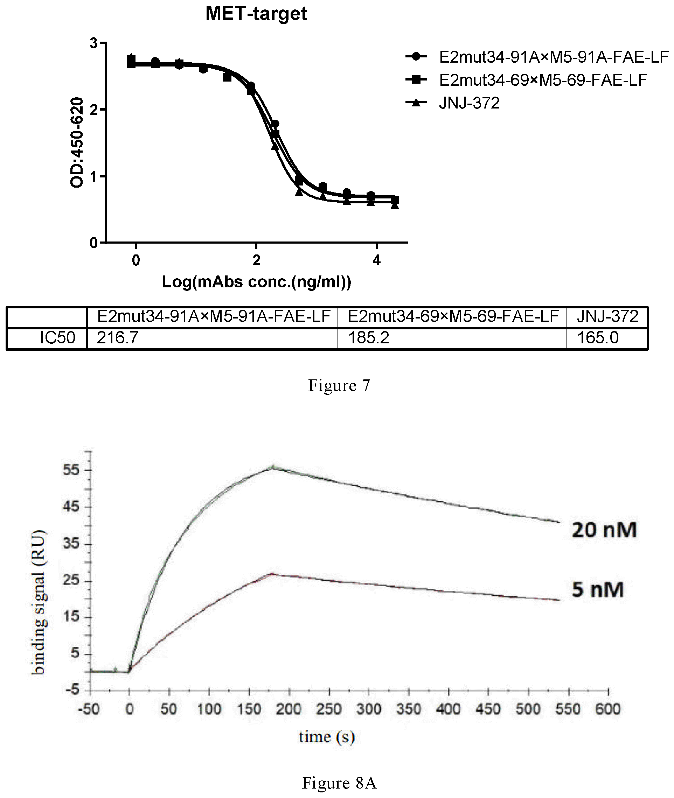

HGF-his-tag (purchased from Acro, Batch No.: HGF-H52H3) was diluted to 4.0 µg/ml with PBS (purchased from Hyclone, Cat. No.: SH30256.01), added to an ELISA plate at 100 µl/well, and coated overnight at 4°C. After washing, 200 µl/well of 2% BSA (purchased from Sigma, Cat. No.: B2064) was added, incubated at room temperature for 90 minutes, and washed. Met biotinylated (purchased from Suzhou Junmeng, Batch No.: 20220526) was diluted to 0.1 µg/ml with 2% BSA. Samples were diluted to 20 µg/ml with the 0.1 µg/ml Met biotinylated solution, then serially diluted 2.5-fold to 0.84 ng/ml, added to the ELISA plate at 100 µl/well, incubated at room temperature for 60 minutes, and washed. Horseradish peroxidase (HRP)-conjugated streptavidin (purchased from Jackson ImmunoResearch, Cat. No.: 016-030-084) was diluted 5000-fold with 2% BSA, added at 100 µl/well, incubated at room temperature for 60 minutes, and washed. Chromogenic substrate TMB (purchased from Sigma, Cat. No.: T2885) at 0.1 mg/ml was added at 100 µl/well avoiding bubbles, and developed at room temperature in the dark for 10 minutes. Finally, 2 M hydrochloric acid solution was added at 100 µl/well to terminate the reaction, avoiding bubbles. The absorbance was read using a microplate reader within 10 minutes (detection wavelength: 450 nm; reference wavelength: 620 nm), and IC₅₀ values were fitted using a four-parameter logistic (4PL) regression model. As shown in Figure 7, the IC₅₀ values of E2mut34-91A×M5-91A-FAE-LF, E2mut34-69×M5-69-FAE-LF, and JNJ-372 for blocking Met were 216.7 ng/ml, 185.2 ng/ml, and 165 ng/ml, respectively. The blocking abilities of E2mut34-91A×M5-91A-FAE-LF and E2mut34-69×M5-69-FAE-LF against Met were comparable to that of JNJ-372.

### Example 4: Affinity determination by Biacore

The binding affinity of antibodies E2-mut34-91A×M5-91A-FAE-LF, E2-mut34-69×M5-69-FAE-LF, and JNJ-372 to recombinant human EGFR and Met was determined using a Biacore T200 molecular interaction analyzer (GE Healthcare Life Sciences).

The method for determining the antibody-EGFR binding affinity is as follows: 40 µg/ml of goat anti-human IgG-Fc fragment antibody was conjugated to the surface of a CM5 chip (Cytiva, catalog number BR-1005-30) for antibody capture. 1 µg/ml of E2-mut34-91A×M5-91A-FAE-LF, E2-mut34-69×M5-69-FAE-LF, and JNJ-372 antibodies were captured on the CM5 chip surface. 20 nM and 5 nM of EGFR were injected and bound to E2-mut34-91A×M5-91A-FAE-LF and E2-mut34-69×M5-69-FAE-LF, respectively; 80 nM and 20 nM of EGFR were injected and bound to JNJ-372. Binding and dissociation kinetics were detected using a Biacore T200 system (GE Healthcare). The affinity KD value was calculated by fitting the dissociation curve using Biacore T200 Evaluation Software 3.0.

The method for determining the antibody-Met binding affinity is as follows: 40 µg/ml goat anti-human IgG-Fc fragment antibody (Jackson ImmunoResearch) was conjugated to the surface of a CM5 chip (Cytiva, catalog number BR-1005-30) for antibody capture. 1 µg/ml of E2-mut34-91A×M5-91A-FAE-LF, E2-mut34-69×M5-69-FAE-LF, and JNJ-372 antibodies were captured on the CM5 chip surface. 50 nM and 12.5 nM of Met were injected to bind to the three antibodies. Binding and dissociation kinetics were detected using a Biacore T200 system (GE Healthcare). The affinity KD value was calculated using binding and dissociation curves fitted with Biacore T200 Evaluation Software 3.0.

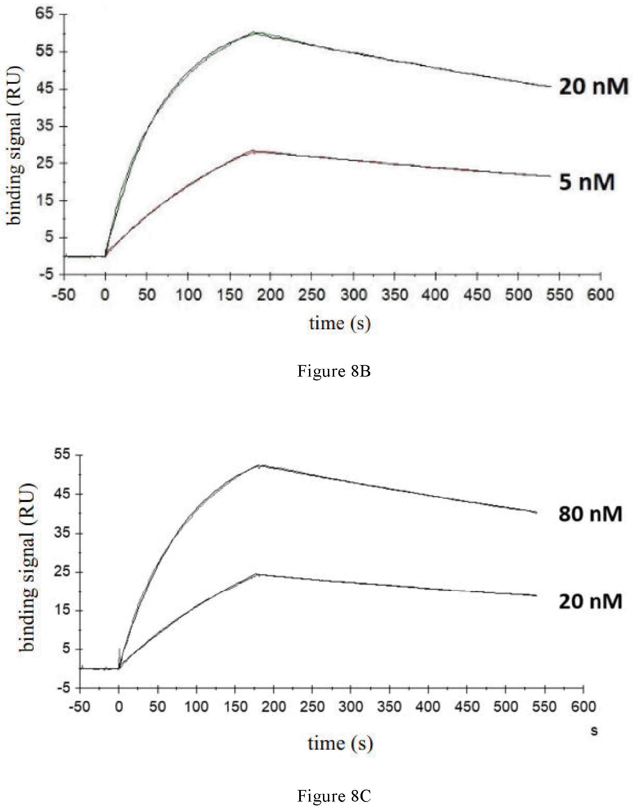

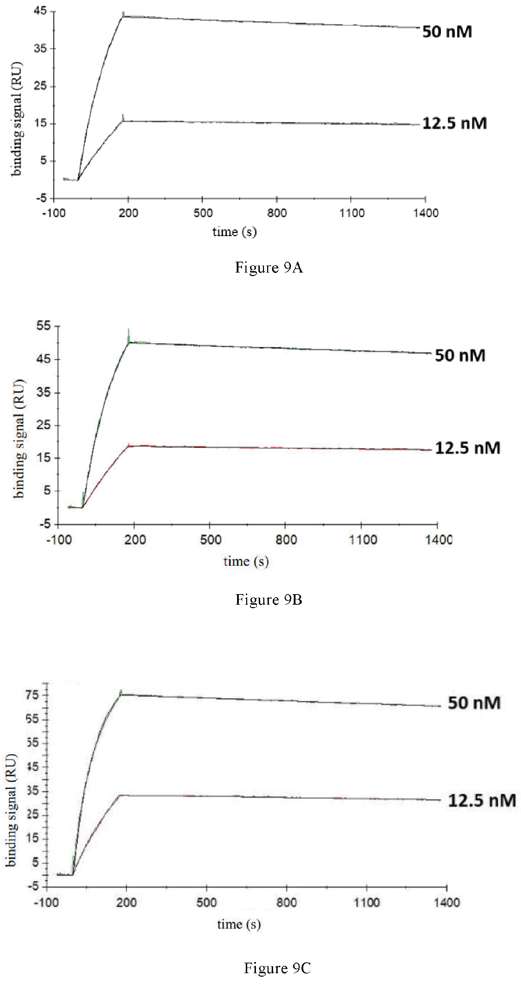

Biacore data are as shown in Table 4, Figures 8A to 8C, and Figures 9A to 9C.

**Table 4: Statistics of Antibody Affinity Assays by Biacore**

| Antigen name | Name of the bispecific antibody | kₐ (1/Ms) | k_{d} (1/s) | K_{D} (M) |
|---|---|---|---|---|
| Recombinant human EGFR | E2-mut34-91A×M5-91A-FAE-LF | 7.08E+05 | 8.49E-04 | 1.20E-09 |
| | E2-mut34-69×M5-69-FAE-LF | 6.98E+05 | 7.59E-04 | 1.09E-09 |
| | JNJ-372 | 1.41E+05 | 7.19E-04 | 5.10E-09 |
| Recombinant human Met | E2-mut34-91A×M5-91A-FAE-LF | 1.32E+05 | 5.66E-05 | 4.30E-10 |
| | E2-mut34-69×M5-69-FAE-LF | 1.52E+05 | 5.43E-05 | 3.57E-10 |
| | JNJ-372 | 2.46E+05 | 5.18E-05 | 2.10E-10 |

The results showed that antibodies E2-mut34-91A×M5-91A-FAE-LF, E2-mut34-69×M5-69-FAE-LF, and JNJ-372 all exhibited binding activity to human EGFR and Met. The EGFR affinity of E2-mut34-91A×M5-91A-FAE-LF and E2-mut34-69×M5-69-FAE-LF was similar, and approximately 5 times stronger than that of JNJ-372. The Met affinity of E2-mut34-91A×M5-91A-FAE-LF and E2-mut34-69×M5-69-FAE-LF was similar, and approximately 2 times weaker than that of JNJ-372.

### Example 5: Cell binding assay

293F-EGFR and 293-cMet cells, which overexpress human EGFR and human cMet, respectively (parental 293F cells were purchased from ATCC, Cat. No. CRL-1573; stable cell lines overexpressing human EGFR or human cMet were subsequently constructed in-house from these cells. These two cell lines were used to assess the binding affinities of the bispecific antibodies to the EGFR and cMet arms, respectively. The amino acid sequence of human EGFR was derived from NCBI Accession No. NP_005219.2, and the sequence of human cMet was derived from NCBI Accession No. NP_000236.2), were incubated with various concentrations of E2mut34-91A×M5-91A-FAE-LF, E2mut34-69×M5-69-FAE-LF, JNJ-372, and an anti-KLH IgG1 control antibody (starting concentration: 25 µg/mL; 4-fold serial dilutions were performed to yield 10 concentration points) at 4°C for 30 minutes. Unbound antibodies were removed by washing with staining buffer (PBS + 1% v/v FBS). The cells were then incubated with a fluorescent secondary antibody, goat anti-human IgG PE (SouthernBiotech, Cat#2040-09), diluted in staining buffer to a final concentration of 5% (v/v), for 30 minutes at 4°C in the dark. Finally, cells were collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and EC50 values were calculated using GraphPad software.

NCI-H1975 cells (purchased from Shanghai Nobiotech Corporation, Cat. No. C01-HE; these cells endogenously co-express human EGFR and human cMet and were used to assess the cooperative binding affinity of the bispecific antibodies) were incubated with various concentrations of E2mut34-91A×M5-91A-FAE-LF, E2mut34-69×M5-69-FAE-LF, JNJ-372, and an anti-KLH IgG1 control antibody (starting concentration: 100 µg/mL; serial dilutions were performed using a 4-fold dilution scheme for the first 7 concentration points and a 10-fold dilution scheme for the subsequent 5 concentration points) at 4°C for 30 minutes. Unbound antibodies were removed by washing with staining buffer (PBS + 1% v/v FBS). The cells were then incubated with a fluorescent secondary antibody, goat anti-human IgG PE (SouthernBiotech, Cat#2040-09), diluted in staining buffer to a final concentration of 5% (v/v), for 30 minutes at 4°C in the dark. Finally, cells were collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and EC50 values were calculated using GraphPad software.

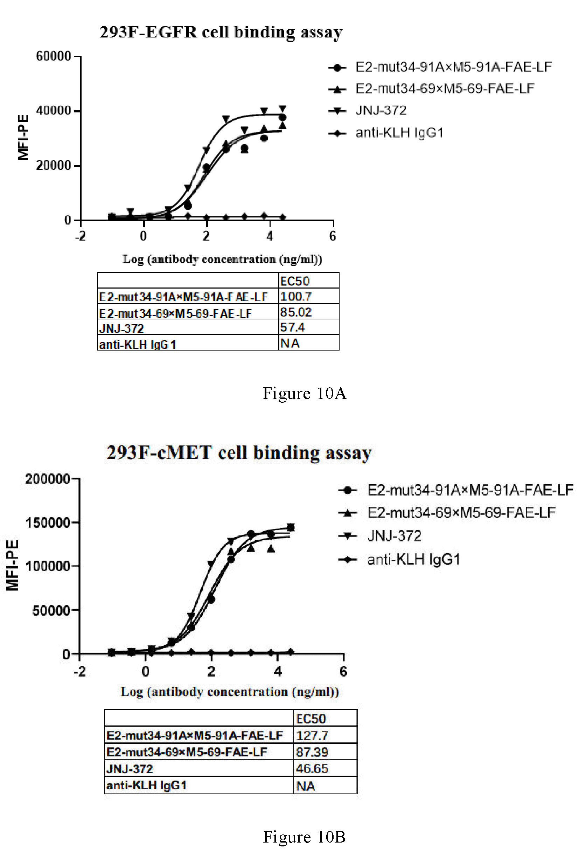

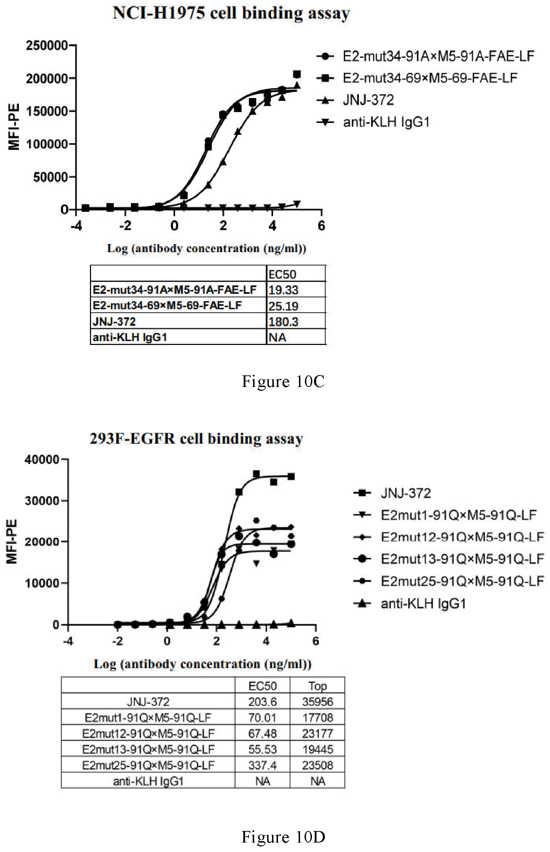

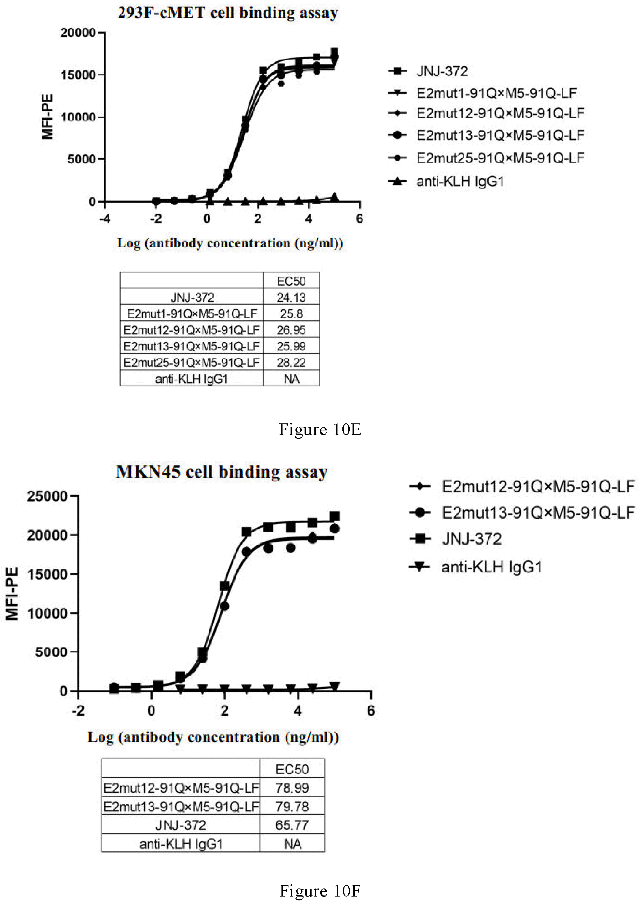

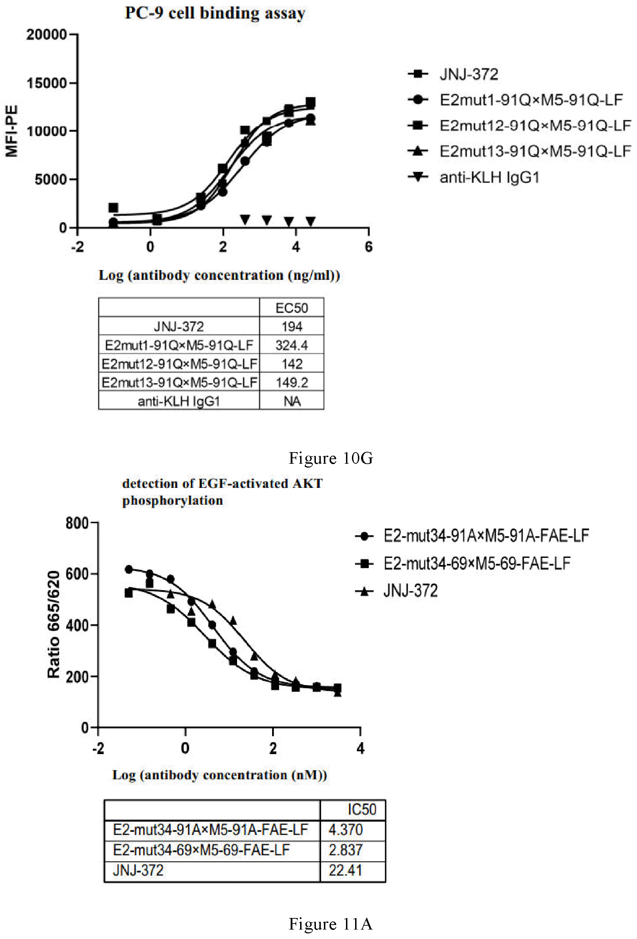

As shown in Figures 10A to 10C, the EC50 values for the binding of E2mut34-91A×M5-91A-FAE-LF, E2mut34-69×M5-69-FAE-LF, and JNJ-372 to 293F-EGFR cells were 100.7 ng/mL, 85.02 ng/mL, and 57.40 ng/mL, respectively; their EC50 values for binding to 293F-cMet cells were 127.7 ng/mL, 87.39 ng/mL, and 46.65 ng/mL, respectively; and their EC50 values for binding to H1975 cells were 19.33 ng/mL, 25.19 ng/mL, and 180.3 ng/mL, respectively. The results showed that the binding affinities of E2mut34-91A×M5-91A-FAE-LF and E2mut34-69×M5-69-FAE-LF to the single antigens EGFR or cMet were slightly weaker than those of the positive control, JNJ-372. However, their binding affinities to the dual antigens EGFR & cMet were significantly superior to that of the positive control, JNJ-372.

### Example 6: Phosphorylation Inhibition Assay

NCI-H1975 cells were digested, centrifuged, and resuspended in complete medium (RPMI-1640 medium + 10% v/v FBS). The cells were seeded into 96-well flat-bottom plates (Corning, Cat. No. 3599) at a density of 2 × 10⁴ cells per well and incubated overnight in a CO₂ incubator. On the second day, the medium in the 96-well plate was replaced with serum-free RPMI-1640 medium, and the cells were incubated overnight in the CO₂ incubator. On the third day, antibodies E2mut34-91A×M5-91A-FAE-LF, E2mut34-69×M5-69-FAE-LF, and JNJ-372 were prepared at 2× assay concentrations using serum-free RPMI-1640 medium (for EGF (R&D Systems, Cat. No. 236-EG-200) stimulated conditions: starting concentration 6 µM, 3-fold serial dilutions, 11 concentration points; for HGF (R&D Systems, Cat. No. 294-HG-100/CF) stimulated conditions: starting concentration 660 nM, 3-fold serial dilutions, 12 concentration points). The medium was removed from the 96-well plate, and 50 µL of the diluted antibodies prepared above was added to each well, followed by pre-incubation at 37°C for 30 minutes. Agonists, EGF (at a concentration of 8 ng/mL) or HGF (at a concentration of 140 ng/mL), were prepared at 2× assay concentrations using serum-free RPMI-1640 medium and kept in the incubator until use. After pre-incubation, the 96-well flat-bottom plate was removed from the incubator, and 50 µL per well of the diluted EGF or HGF agonist solution was added. Incubation was continued at 37°C for 30 minutes. Following incubation, the 96-well flat-bottom plate was removed, and the supernatant was discarded. Then, 50 µL of 1× lysis buffer was added according to the kit instructions (phospho-AKT (Ser473) kit (Cisbio, Cat. No. 64AKSPEH) & Advanced phospho-ERK1/2 (Thr202/Tyr204) (Cisbio, Cat. No. 64AERPEH)). The plate was shaken at room temperature at 350 rpm for 30 minutes. Subsequently, 16 µL of cell lysate and 4 µL of the antibody mix from the kits were added to HTRF 96-well low volume plates (Cisbio, Cat. No. 66PL96025). After gently mixing with a pipette, the plate was sealed with film and centrifuged at 1000 rpm for 30 seconds. Finally, the plate was incubated protected from light at room temperature (20°C-25°C). The absorbance was measured at wavelengths 655 nm and 620 nm within 4-24 hours using a microplate reader (BioTek, Synergy H1 model). The ratio (Ratio 665/620) was calculated using the formula: Ratio 665/620 = (A655 / A620) × 10000. Data analysis for the Ratio 665/620 was performed using GraphPad Prism software.

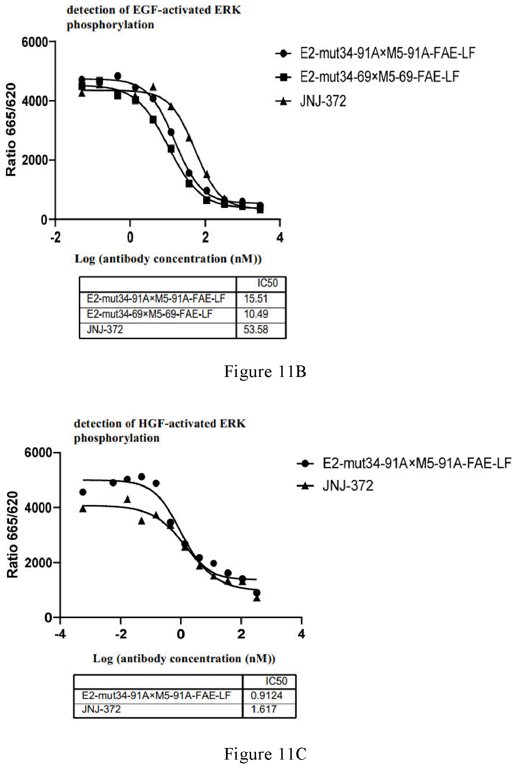

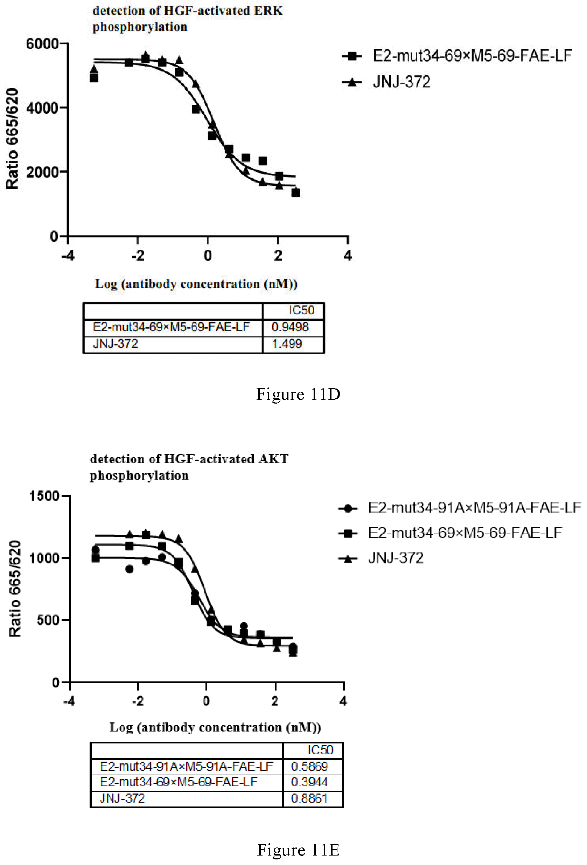

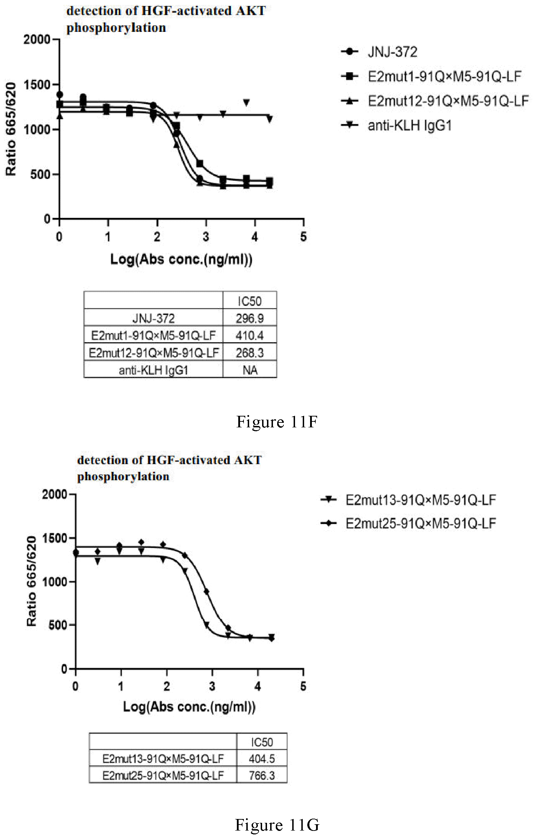

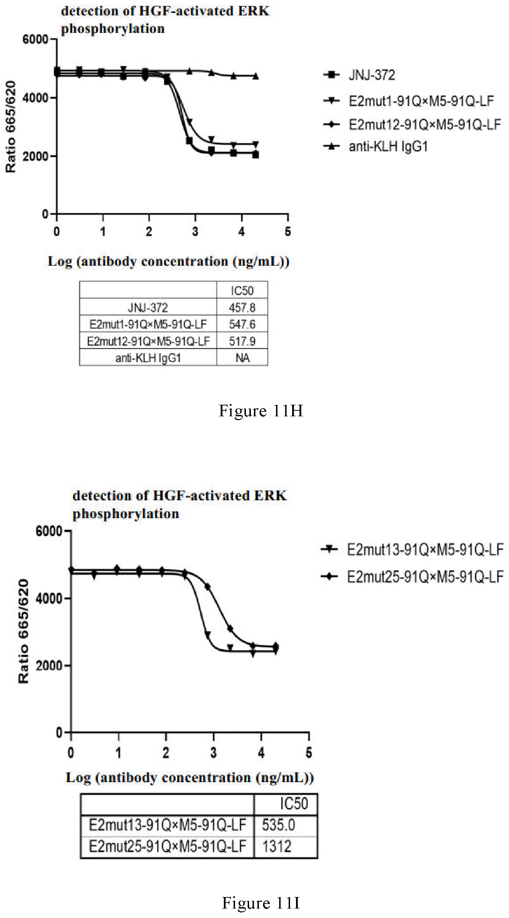

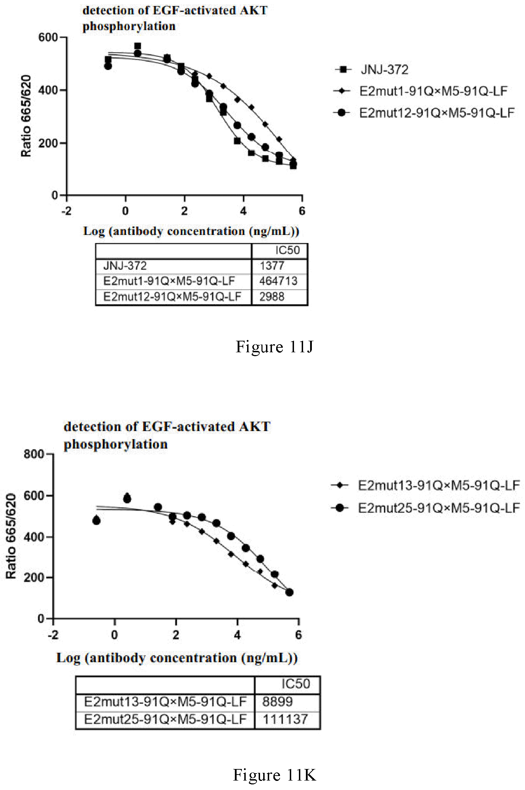

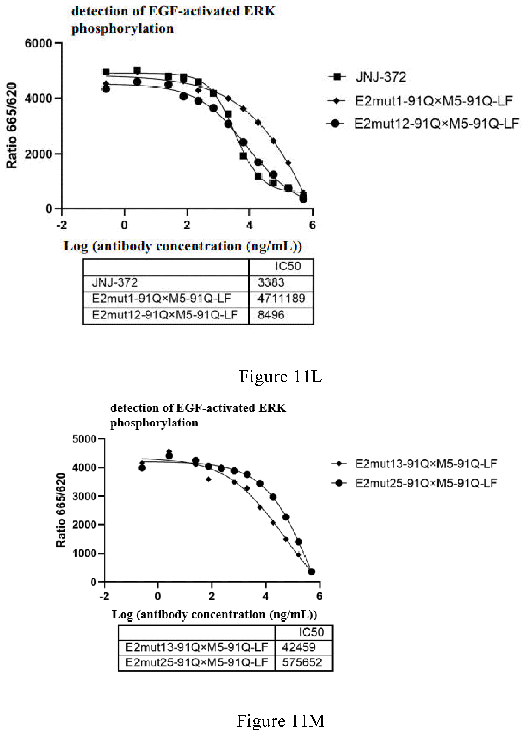

As shown in Figures 11A to 11E, under EGF-stimulated conditions, the IC₅₀ values for the inhibition of AKT phosphorylation by E2mut34-91A×M5-91A-FAE-LF, E2mut34-69×M5-69-FAE-LF, and JNJ-372 were 4.370 nM, 2.837 nM, and 22.41 nM, respectively; the IC₅₀ values for the inhibition of ERK phosphorylation were 15.51 nM, 10.49 nM, and 53.58 nM, respectively. These results indicated that, under EGF-stimulated conditions, the activities of E2mut34-91A×M5-91A-FAE-LF and E2mut34-69×M5-69-FAE-LF in inhibiting AKT and ERK phosphorylation were superior to those of the positive control, JNJ-372. Under HGF-stimulated conditions, the IC₅₀ values for the inhibition of AKT phosphorylation by E2mut34-91A×M5-91A-FAE-LF, E2mut34-69×M5-69-FAE-LF, and JNJ-372 were 0.5869 nM, 0.3944 nM, and 0.8861 nM, respectively; the IC₅₀ values for the inhibition of ERK phosphorylation were 0.9124 nM, 0.9498 nM, and 1.617 nM, respectively. These results indicated that, under HGF-stimulated conditions, the activities of E2mut34-91A×M5-91A-FAE-LF and E2mut34-69×M5-69-FAE-LF in inhibiting AKT and ERK phosphorylation were comparable to those of the positive control, JNJ-372.

### Example 7: ADCC Activity Assay

Reporter gene method: NCI-H1975 cells were seeded into a 96-well white plate (Costar, Cat. No. 3917) at a density of 5 × 10⁴ cells per well and incubated overnight in a CO₂ incubator. On the following day, the 96-well white plate was removed from the CO₂ incubator, the culture supernatant was discarded, and 40 µL per well of antibodies E2mut34-91A×M5-91A-FAE-LF, E2mut34-69×M5-69-FAE-LF, and JNJ-372 diluted in assay buffer (RPMI-1640 medium + 2% v/v FBS) was added (starting concentration: 50 µg/mL; 3-fold serial dilutions for the first 3 concentration points; 5-fold serial dilutions for the subsequent 7 concentration points). The antibodies were pre-incubated with the target cells for 30 minutes. After pre-incubation, 40 µL per well of effector Jurkat ADCC cells (expressing NFAT-Luc and FcγRIIIa) resuspended in assay buffer (RPMI-1640 medium + 2% v/v FBS) was added at a density of 1 × 10⁵ cells per well. Incubation was continued for 6 hours at 37°C. Finally, One-Lite substrate (Vazyme, Cat. No. DD1203-03) was added, and the luciferase signal was detected using a microplate reader (TECAN, M1000 pro model). A higher fluorescence reading indicated a stronger ADCC effect. Antibody dose-dependent ADCC effect curves were fitted using GraphPad software.

LDH method: PBMC effector cells were first revived and allowed to recover overnight in a CO₂ incubator. Target NCI-H1975 cells were digested and centrifuged; the supernatant was discarded, and the cell density was adjusted to 4 × 10⁵ cells/mL using phenol red-free RPMI Medium 1640 supplemented with 1% v/v FBS. Then, 50 µL of target cells was added to each well of a 96-well low-attachment plate (Costar, Cat. No. 7007). Subsequently, antibodies E2mut34-91A×M5-91A-FAE-LF, E2mut34-69×M5-69-FAE-LF, JNJ-372, and anti-KLH IgG1 were diluted to 4× assay concentrations using phenol red-free RPMI Medium 1640 + 1% FBS (antibody starting concentration: 66.67 µg/mL; 3-fold serial dilutions for the first 2 concentration points; 5-fold serial dilutions for the subsequent 7 concentration points). Then, 50 µL per well of the diluted antibody solution was added, gently mixed by pipetting several times, and incubated in the incubator for 30 minutes. The revived and overnight-recovered PBMCs were taken, and their density was adjusted to 5 × 10⁶ cells/mL (effector-to-target ratio: 25:1). Next, 100 µL of PBMCs was added to each well of the 96-well low-attachment plate and co-cultured in a CO₂ incubator for 4 hours. Thirty minutes before the end of the co-culture period, 20 µL per well of the cell lysis buffer from the LDH-cytotoxicity kit (BioVision, Cat. No. K311-400) was added to the Tmax group wells, and the 96-well low-attachment plate was returned to the incubator for continued incubation. The 96-well low-attachment plate was then removed and centrifuged (1500 rpm × 5 minutes). Subsequently, 50 µL of culture supernatant from each well was transferred to a new 96-well flat-bottom plate (Corning, Cat. No. 3599). Then, 50 µL of the prepared Dye Solution from the LDH-cytotoxicity kit was added to each well, and the plate was incubated on a shaker protected from light for 10-30 minutes. Finally, the plate was read using a multimode microplate reader (TECAN, M1000 pro model), and data were analyzed using GraphPad Prism software. The cytotoxicity percentage was calculated using the formula: ADCC % = [(Sample - Buffer) / (Tmax - Ts)] × 100.

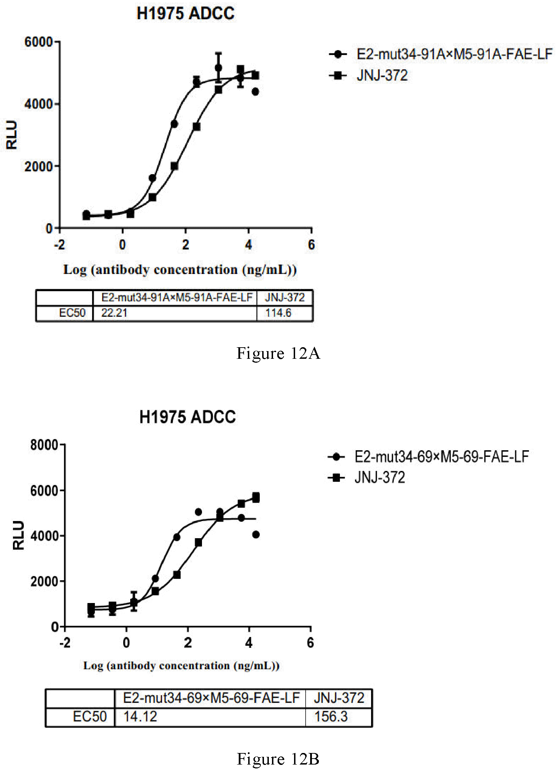

As shown in Figures 12A to 12B, in the reporter gene assay, both E2mut34-91A×M5-91A-FAE-LF and E2mut34-69×M5-69-FAE-LF exhibited ADCC activity, with EC50 values of 22.21 ng/mL and 14.12 ng/mL, respectively. Furthermore, the ADCC activity of E2mut34-91A×M5-91A-FAE-LF and E2mut34-69×M5-69-FAE-LF was stronger than that of the positive control, JNJ-372.

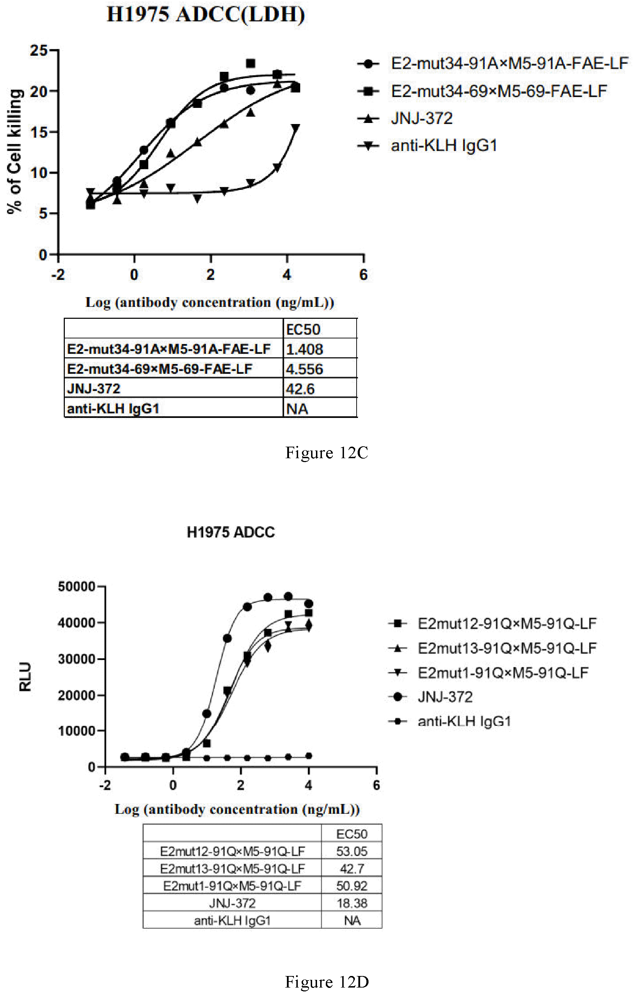

As shown in Figure 12C, when PBMCs were used as effector cells and NCI-H1975 cells were used as target cells, E2mut34-91A×M5-91A-FAE-LF, E2mut34-69×M5-69-FAE-LF, and JNJ-372 induced a significant ADCC effect, with EC50 values of 1.408 ng/mL, 4.556 ng/mL, and 42.60 ng/mL, respectively. In contrast, the negative control anti-KLH IgG1 did not induce an ADCC effect. The ADCC activity of E2mut34-91A×M5-91A-FAE-LF and E2mut34-69×M5-69-FAE-LF was stronger than that of the positive control, JNJ-372.

### Example 8: Internalization Assay

MKN45 cells (purchased from Shanghai Nobiotech Corporation, Cat. No. C01-1C; endogenously expressing human EGFR and human cMet) were digested, centrifuged, and resuspended in staining buffer (PBS + 1% v/v FBS). The cells were seeded into a 96-well round-bottom plate (Corning, Cat. No. 3799) at a density of 2 × 10⁵ cells per well. Antibodies E2mut34-91A×M5-91A-FAE-LF, E2mut34-69×M5-69-FAE-LF, JNJ-372, and anti-KLH IgG1 (starting concentration: 100 µg/mL; 4-fold serial dilutions, 11 concentration points) diluted in staining buffer (PBS + 1% v/v FBS) were added and incubated at 4°C for 30 minutes. Unbound antibodies were then removed by washing with staining buffer (PBS + 1% v/v FBS). The cells were resuspended in 200 µL of complete medium (RPMI-1640 medium + 10% v/v FBS) and split into two equal portions: one portion was incubated at 4°C for 24 hours, and the other portion was incubated at 37°C for 24 hours. After the incubation period, the plates were centrifuged, the supernatant was discarded, and a fluorescent secondary antibody, goat anti-human IgG PE (SouthernBiotech, Cat#2040-09), diluted in staining buffer to a final concentration of 5% (v/v), was added. The cells were incubated at 4°C for 30 minutes in the dark. Subsequently, unbound antibodies were removed by washing with staining buffer (PBS + 1% v/v FBS). The cells were fixed with PFA (Absin, Cat. No. abs9179) and then collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and Top values were calculated using GraphPad software. The internalization rate was calculated using the formula: Internalization Rate (%) = (1 - Top_37°C / Top_4°C) × 100.

The results are shown in Table 5.

**Table 5: Assay Results of Internalization Activity in MKN45 Cells**

| Group | Top_4°C | Top_37°C | Internalization Rate, % |
|---|---|---|---|
| JNJ-372 | 26097 | 13295 | 49 |
| E2mut34-91A×M5-91A-FAE-LF | 33839 | 9374 | 72 |
| E2mut34-69A×M5-91A-FAE-LF | 32544 | 8943 | 72 |
| Anti-KLH hIgG1 | 1572 | 1613 | NA |

The results showed that E2mut34-91A×M5-91A-FAE-LF, E2mut34-69×M5-69-FAE-LF, and JNJ-372 were all internalized by MKN45 cells, whereas the negative control anti-KLH IgG1 exhibited almost no internalization. The results indicated that E2mut34-91A×M5-91A-FAE-LF and E2mut34-69×M5-69-FAE-LF showed comparable internalization activity, with an internalization rate of 72% at 24 hours, which was superior to that of JNJ-372 (49% internalization rate at 24 hours).

### Example 9: H1975 Xenograft Model

In this example, the inhibitory effect of E2mut34-91A×M5-91A-FAE-LF of the invention on a CB-17 SCID mouse model transplanted with human lung adenocarcinoma NCI-H1975 cells was evaluated.

### 1. Testing procedure

Female CB-17 SCID mice, aged 6-8 weeks (purchased from Shanghai Jihui Laboratory Animal Breeding Co., Ltd., Animal Certificate No. 20170012022482), were inoculated subcutaneously in the right flank with 5 × 10⁶ NCI-H1975 cells. When the mean tumor volume reached approximately 164 mm³, suitable animals were selected and randomly divided into 5 groups (n = 6 per group) based on tumor volume. The groups were as follows:
G1: Normal saline control group (vehicle control group);
G2: JNJ-372 (5 mg/kg) group (positive control group);
G3: E2mut34-91A×M5-91A-FAE-LF (5 mg/kg) group (treatment group).

Administration was performed via intraperitoneal injection twice weekly for 3 consecutive weeks. The experiment was terminated 3 days after the last dose. Tumor volume and body weight were measured twice weekly, and the data were recorded. At the end of the experiment, the mice were euthanized. The relative tumor proliferation rate was calculated using the formula: TGI (%) = [1 - (Ti - T0) / (Vi - V0)] × 100% (Ti: the mean tumor volume of the treatment group on day i of dosing, T0: the mean tumor volume of the treatment group on day 0 of dosing, Vi: the mean tumor volume of the vehicle control group on day i of dosing, V0: the mean tumor volume of the vehicle control group on day 0 of dosing).

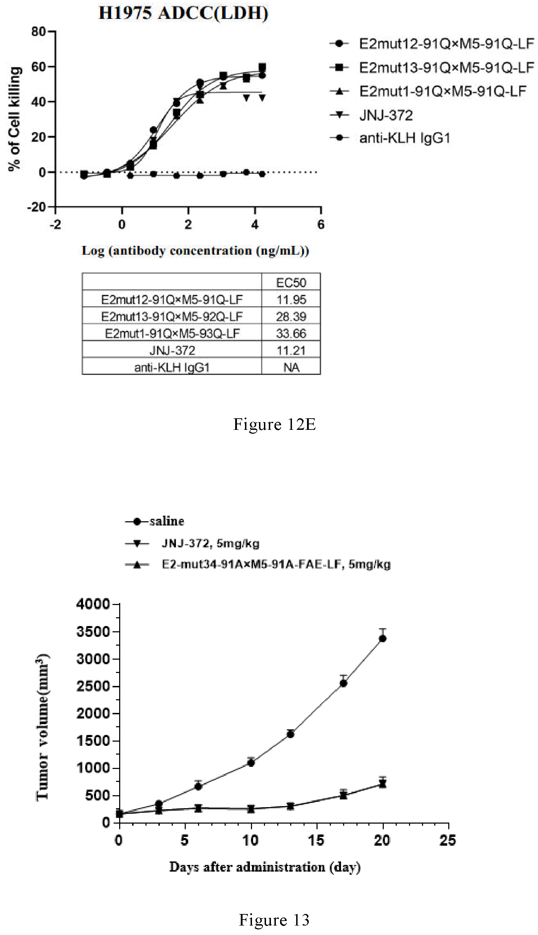

The results are shown in Table 6 and Figure 13.

**Table 6: Efficacy Analysis of Each Group in the NCI-H1975 Human Lung Cancer Subcutaneous Tumor Model**

| Experimental group | Day 20 after the initiation of administration | | |
|---|---|---|---|
| | Tumor volume( *x̅* ±S) | TGI (%) | P Value (compared to the control group) |
| G1, normal saline | 3374±178 | - | - |
| G2, JNJ-372, 5mg/kg | 708±135 | 83.0 | <0.001 |
| G3, E2mut34-91A×M5-91A-FAE-LF, 5mg/kg | 711±73 | 83.0 | <0.001 |

| | | | |
|---|---|---|---|
| Note: 1. Data are presented as "Mean ± SEM"; 2. TGI% = [1-(Ti-T0)/(Vi-V0)]×100%; 3. P values were obtained by T-test comparing the tumor volumes of the groups. | | | |

The results showed that on day 20 after administration, the mean tumor volume in the normal saline control group was 3374 mm³. In the JNJ-372 (5 mg/kg) group, the mean tumor volume was 708 mm³, corresponding to a tumor growth inhibition (TGI) rate of 83.0% compared to the normal saline control group, indicating significant inhibition of tumor growth. In the E2mut34-91A×M5-91A-FAE-LF (5 mg/kg) group, the mean tumor volume was 711 mm³, also achieving a TGI rate of 83.0% compared to the normal saline control group, demonstrating significant tumor growth inhibition. These results indicate that in the CB-17 SCID mouse model transplanted with NCI-H1975 cells, E2mut34-91A×M5-91A-FAE-LF exhibited significant tumor inhibitory effects at a dose level of 5 mg/kg.

Preparation Example 3: Design and preparation of the second batch of EGFR×Met bispecific antibody mutants

### 1. Construction of bispecific antibody molecules

The heavy chains of the 10 high-affinity EGFR antibodies obtained from the previous Preparation Example 1 were used as the EGFR-arm parental antibody heavy chains for the EGFR×Met bispecific antibodies. Additionally, the heavy chain of a previously disclosed Met antibody (SEQ ID NO: 410 in CN105705519A) was referenced and modified accordingly in the constant region as required for bispecific antibody preparation. This modified sequence was used as the Met-arm parental antibody heavy chain for the EGFR×Met bispecific antibody (see SEQ ID NO: 20). E2-10-LC-13-91Q was used as the common light chain.

The heavy chains and light chains listed in Table 7 were synthesized by Tsingke Biotechnology, following the method described in Preparation Example 2.

**Table 7: Names and Combinations of Mutant Heavy and Light Chains**

| Antibody name | Heavy chain of EGFR antibody | Heavy chain of Met antibody | Light chain of antibody | Transfection ratio |
|---|---|---|---|---|
| E2mut1-91Q-F405L-LF | E2mut1-HC-F405L (SEQ ID NO: 58) | / | E2-10-LC-13-91Q (SEQ ID NO: 57) | 1:1 |
| E2mut2-91Q-F405L-LF | E2mut2-HC-F405L (SEQ ID NO: 59) | / | E2-10-LC-13-91Q (SEQ ID NO: 57) | 1:1 |
| E2mut5-91Q-F405L-LF | E2mut5-HC-F405L (SEQ ID NO: 60) | / | E2-10-LC-13-91Q (SEQ ID NO: 57) | 1:1 |
| E2mut12-91Q-F405L-LF | E2mut12-HC-F405L (SEQ ID NO: 61) | / | E2-10-LC-13-91Q (SEQ ID NO: 57) | 1:1 |
| E2mut13-91Q-F405L-LF | E2mut13-HC-F405L (SEQ ID NO: 62) | / | E2-10-LC-13-91Q (SEQ ID NO: 57) | 1:1 |
| E2mut16-91Q-F405L-LF | E2mut16-HC-F405L (SEQ ID NO: 63) | / | E2-10-LC-13-91Q (SEQ ID NO: 57) | |
| E2mut17-91Q-F405L-LF | E2mut-HC-F405L (SEQ ID NO: 64) | / | E2-10-LC-13-91Q(S EQ ID NO: 57) | |
| E2mut25-91Q-F405L-LF | E2mut25-HC-F405L (SEQ ID NO: 65) | / | E2-10-LC-13-91Q (SEQ ID NO: 57) | |
| E2mut34-91Q-F405L-LF | E2mut34-HC-F405L (SEQ ID NO: 17) | / | E2-10-LC-13-91Q (SEQ ID NO: 57) | |
| E2mut38-91Q-F405L-LF | E2mut38-HC-F405L (SEQ ID NO: 66) | / | E2-10-LC-13-91Q (SEQ ID NO: 57) | |
| M5-91Q-K409R-LF | / | M5-HC-K409R (SEQ ID NO: 16) | E2-10-LC-13-91Q (SEQ ID NO: 57) | 1:1 |
| E2mut1-91Q×M5-91Q-KIH-LF | E2mut1-HC-hole (SEQ ID NO: 67) | M5-HC-knob (SEQ ID NO: 20) | E2-10-LC-13-91Q (SEQ ID NO: 57) | 1:1:1 |
| E2mut2-91Q×M5-91Q-KIH-LF | E2mut2-HC-hole (SEQ ID NO: 68) | M5-HC-knob (SEQ ID NO: 20) | E2-10-LC-13-91Q (SEQ ID NO: 57) | 1:1:1 |
| E2mut5-91Q×M5-91Q-KIH-LF | E2mut5-HC-hole (SEQ ID NO: 69) | M5-HC-knob (SEQ ID NO: 20) | E2-10-LC-13-91Q (SEQ ID NO: 57) | 1:1:1 |
| E2mut12-91Q×M5-91Q-KIH-LF | E2mut12-HC-hole (SEQ ID NO: 70) | M5-HC-knob (SEQ ID NO: 20) | E2-10-LC-13-91Q (SEQ ID NO: 57) | 1:1:1 |
| E2mut13-91Q×M5-91Q-KIH-LF | E2mut13-HC-hole (SEQ ID NO: 71) | M5-HC-knob (SEQ ID NO: 20) | E2-10-LC-13-91Q (SEQ ID NO: 57) | |
| E2mut16-91Q×M5-91Q-KIH-LF | E2mut16-HC-hole (SEQ ID NO: 72) | M5-HC-knob (SEQ ID NO: 20) | E2-10-LC-13-91Q (SEQ ID NO: 57) | |
| E2mut17-91Q×M5-91Q-KIH-LF | E2mut17-HC-hole (SEQ ID NO: 73) | M5-HC-knob (SEQ ID NO: 20) | E2-10-LC-13-91Q (SEQ ID NO: 57) | |
| E2mut25-91Q×M5-91Q-KIH-LF | E2mut25-HC-hole (SEQ ID NO: 74) | M5-HC-knob (SEQ ID NO: 20) | E2-10-LC-13-91Q (SEQ ID NO: 57) | |
| E2mut34-91Q×M5-91Q-KIH-LF | E2mut34-HC-hole (SEQ ID NO: 18) | M5-HC-knob (SEQ ID NO: 20) | E2-10-LC-13-91Q (SEQ ID NO: 57) | |
| E2mut38-91Q×M5-91Q-KIH-LF | E2mut38-HC-hole (SEQ ID NO: 75) | M5-HC-knob (SEQ ID NO: 20) | E2-10-LC-13-91Q (SEQ ID NO: 57) | |

### 2. Transient protein expression and purification

The required heavy and light chains are shown in Table 7. Among them:
E2mut1-91Q-F405L-LF, E2mut2-91Q-F405L-LF, E2mut5-91Q-F405L-LF, E2mut12-91Q-F405L-LF, E2mut13-91Q-F405L-LF, E2mut16-91Q-F405L-LF, E2mut17-91Q-F405L-LF, E2mut25-91Q-F405L-LF, E2mut34-91Q-F405L-LF, E2mut38-91Q-F405L-LF, and M5-91Q-K409R-LF were used for the subsequent preparation of bispecific antibodies (in vitro recombinant method);
For E2mut1-91Q×M5-91Q-KIH-LF, E2mut2-91Q×M5-91Q-KIH-LF, E2mut5-91Q×M5-91Q-KIH-LF, E2mut12-91Q×M5-91Q-KIH-LF, E2mut13-91Q×M5-91Q-KIH-LF, E2mut16-91Q×M5-91Q-KIH-LF, E2mut17-91Q×M5-91Q-KIH-LF, E2mut25-91Q×M5-91Q-KIH-LF, E2mut34-91Q×M5-91Q-KIH-LF, and E2mut38-91Q×M5-91Q-KIH-LF, bispecific antibodies were prepared using a common light chain and a "knob-into-hole" technique (refer to WO1996027011A1).

### 2.1 Expression of bispecific antibody molecules

CHO-K1 cells (owned by Suzhou Junmeng) in culture were counted. When the cell density reached 2-6 × 10⁶ cells/mL, they were passaged and expanded using CD CHO medium (purchased from Thermo Fisher, Cat. No. 12490-001). One day before transfection, the cell density was diluted to 1.8-2.5 × 10⁶ cells/mL. On the following day, when the cell density reached approximately 3.5-5.0 × 10⁶ cells/mL, transfection was performed. First, one-tenth of the transfection volume of CD CHO medium was added. Then, 1-2 µg/mL of plasmid (prepared in-house) was added according to the combinations shown in Table 2. Finally, 3-14 µg/mL of PEI (purchased from Polysciences, Cat. No. 24765-1) was added. The mixture was mixed well and incubated at room temperature. Subsequently, the transfection mixture was slowly added to the pre-prepared cells, mixing continuously while adding. The transfected mixture was placed in a shaker for culture. On the first day after transfection, 6% glycan regulator (purchased from OPM, Cat. No. R170026) was added, and the culture was fed with 4% Cell Boost 7a (purchased from Hyclone, Cat. No. SH31026.05) and 0.4% Cell Boost 7b (purchased from Hyclone, Cat. No. SH31027.04CN). Subsequently, feeding was performed every two days. Samples were collected 5-9 days after transfection.

### 3. In vitro recombination and purification

### 3.1 Affinity capture of bispecific antibody molecules

After culture, the cells were centrifuged at 1000g for 5 min using a floor centrifuge (ThermoFisher, R404A) to discard the precipitate, and then centrifuged at 8000g for 30 min to collect the cell supernatant. The supernatant was then aseptically filtered using a 0.22µm filter cup (JET, FPE-214-000). Purification was performed using a protein purification system (GE, AKTA Avant). The Mabselect Sure LX column (Cytiva, 17547403) was equilibrated with PBS equilibration buffer (Wuxi Aorui Dongyuan Biotechnology Co., Ltd., ZLI-9061). After sample loading, the sample was first rinsed with affinity chromatography elution buffer A (pH 5.5, 45 mM acetate-sodium acetate + 1 M sodium chloride system), then rinsed with elution buffer B (pH 5.5, 45 mM acetate-sodium acetate system), and finally eluted with affinity elution buffer (pH 3.6, 10 mM acetate-sodium acetate buffer). The sample was then neutralized with 1 M Tris buffer (purchased from Merck, catalog number: E300016981946) and the pH was adjusted to 5.5-6 for the next step of in vitro recombination.

### 3.2 In vitro recombination of bispecific antibody molecules

Referring to the technical solution disclosed in WO2011131746, the bispecific antibodies were prepared using Fab arm exchange technology. The method was as follows:
The two affinity-purified parental antibodies were concentrated and buffer-exchanged into PBS equilibration buffer (purchased from Wuxi Original East Ocean Biotechnology Co., Ltd., ZLI-9061). The protein concentration was adjusted to 1 ± 0.05 mg/mL.

Preparation of 750 mM Cysteamine Hydrochloride (purchased from VETEC, V900342-25G) Stock Solution: 2.556 g of Cysteamine hydrochloride was added to 14 mL of PBS. The volume was brought to 30 mL with additional PBS. The solution was then filtered using a 0.22 µm filter (purchased from Sartorius, 16541-K). Finally, the container was wrapped in aluminum foil and stored protected from light.

Construction of the incubation system: the parental antibodies processed as described above were taken. As shown in Table 3, according to a molar ratio of M:E = 1:1.2, 2 mL of the M parental antibody, 2.4 mL of the E parental antibody, and 0.489 mL of 750 mM Cysteamine hydrochloride were added to a 15 mL centrifuge tube (purchased from Genemore, G3210015). The prepared incubation system was sealed with aluminum foil and incubated protected from light in a 31°C water bath (Shanghai Jinghong Experimental Equipment Co., Ltd., DK-S28) for 3 hours. After incubation, the mixture was concentrated and buffer-exchanged with PBS equilibration buffer and then placed at room temperature protected from light for 16-24 hours.

**Table 8: In Vitro Recombination Combination Table of Bispecific Antibody Mutants**

| Name of bispecific antibody | Parent antibody M (anti-Met antibody) | Parent antibody E (anti-EGFR antibody) | Molar ratio M:E |
|---|---|---|---|
| E2mut1-91Q×M5-91Q-FAE-LF | M5-91Q-K409R-LF | E2mut1-91Q-F405L-LF | 1:1.2 |
| E2mut2-91Q×M5-91Q-FAE-LF | M5-91Q-K409R-LF | E2mut2-91Q-F405L-LF | 1:1.2 |
| E2mut5-91Q×M5-91Q-FAE-LF | M5-91Q-K409R-LF | E2mut5-91Q-F405L-LF | 1:1.2 |
| E2mut12-91Q×M5-91Q-FAE-LF | M5-91Q-K409R-LF | E2mut12-91Q-F405L-LF | 1:1.2 |
| E2mut13-91Q×M5-91Q-FAE-LF | M5-91Q-K409R-LF | E2mut13-91Q-F405L-LF | 1:1.2 |
| E2mut16-91Q×M5-91Q-FAE-LF | M5-91Q-K409R-LF | E2mut16-91Q-F405L-LF | 1:1.2 |
| E2mut17-91Q×M5-91Q-FAE-LF | M5-91Q-K409R-LF | E2mut17-91Q-F405L-LF | 1:1.2 |
| E2mut25-91Q×M5-91Q-FAE-LF | M5-91Q-K409R-LF | E2mut25-91Q-F405L-LF | 1:1.2 |
| E2mut34-91Q×M5-91Q-FAE-LF | M5-91Q-K409R-LF | E2mut34-91Q-F405L-LF | 1:1.2 |
| E2mut38-91Q×M5-91Q-FAE-LF | M5-91Q-K409R-LF | E2mut38-91Q-F405L-LF | 1:1.2 |

### 3.3 Purification of bispecific antibody molecules

Capto^{™} MMC Impres (Cytiva, 17371602) packing material was used for purification. The system was pre-equilibrated using washing buffer (pH 7.5, 20 mM Tris-HCl + 1 M NaCl buffer) and then equilibrated using equilibration buffer (pH 7.5, 20 mM Tris-HCl). After loading the sample, equilibrate with 3-6 column volumes of equilibration buffer, and finally perform linear elution with elution buffer (pH 7.5, 20 mM Tris-HCl + 1 M NaCl buffer) to collect the target protein.

### Example 10: Biacore detection of binding and dissociation kinetics of bispecific antibody mutants

The binding affinity of the antibodies to recombinant human EGFR and human c-Met was determined using a Biacore T200 molecular interaction analyzer (GE Healthcare Life Sciences).

The binding affinity of the antibody to human EGFR was determined as follows: 40 µg/mL of goat anti-human IgG-Fc fragment antibody (Jackson ImmunoResearch) was immobilized on the surface of a CM5 chip (Cytiva, Cat. No. BR-1005-30) for antibody capture. Subsequently, 1 µg/mL of the ALK101 antibody was captured on the CM5 chip surface, and 40 nM and 10 nM concentrations of human EGFR were injected to allow binding to the antibody. Binding and dissociation kinetics were detected using a Biacore T200 system (GE Healthcare). The binding and dissociation curves were fitted using Biacore T200 Evaluation Software 3.0 to calculate the affinity KD value.

The binding affinity of the antibody to human c-Met was determined as follows: 40 µg/mL of goat anti-human IgG-Fc fragment antibody (Jackson ImmunoResearch) was immobilized on the surface of a CM5 chip (Cytiva, Cat. No. BR-1005-30) for antibody capture. Subsequently, 1 µg/mL of the ALK101 antibody was captured on the CM5 chip surface, and 40 nM and 10 nM concentrations of human c-Met were injected to allow binding to the antibody. Binding and dissociation kinetics were detected using a Biacore T200 system (GE Healthcare). The binding and dissociation curves were fitted using Biacore T200 Evaluation Software 3.0 to calculate the affinity KD value.

The results are shown in Table 9. E2mut1-91Q-F405L-LF, E2mut2-91Q-F405L-LF, E2mut5-91Q-F405L-LF, E2mut12-91Q-F405L-LF, E2mut13-91Q-F405L-LF, E2mut16-91Q-F405L-LF, E2mut17-91Q-F405L-LF, E2mut25-91Q-F405L-LF, E2mut34-91Q-F405L-LF, and E2mut38-91Q-F405L-LF all exhibited binding activity to human EGFR, with affinities comparable to or slightly weaker than that of JNJ-372. M5-91Q-K409R-LF showed binding activity to human c-Met, with an affinity comparable to that of JNJ-372. Furthermore, the affinities of four bispecific antibody molecules, E2mut1-91Q×M5-91Q-FAE-LF, E2mut12-91Q×M5-91Q-FAE-LF, E2mut13-91Q×M5-91Q-FAE-LF, and E2mut25-91Q×M5-91Q-FAE-LF, were tested. The results showed that their monovalent affinities were consistent with those of the bivalent monoclonal antibodies.

**Table 9: Determination of Affinity of Antibody Mutants by Biacore**

| Antigen name | Antibody name | kₐ (1/Ms) | k_{d} (1/s) | K_{D} (M) |
|---|---|---|---|---|
| EGFR | JNJ-372 | 1.72E+05 | 8.36E-04 | 4.84E-09 |
| | E2mut1-91Q×M5-91Q-FAE-LF | 2.91E+05 | 5.51E-03 | 1.89E-08 |
| | E2mut12-91Q×M5-91Q-FAE-LF | 2.97E+05 | 1.68E-03 | 5.67E-09 |
| | E2mut13-91Q×M5-91Q-FAE-LF | 3.16E+05 | 2.86E-03 | 9.05E-09 |
| | E2mut25-91Q×M5-91Q-FAE-LF | 1.42E+04 | 1.30E-03 | 9.18E-08 |
| | E2mut1-91Q-F405L-LF | 3.39E+05 | 5.22E-03 | 1.54E-08 |
| | E2mut2-91Q-F405L-LF | 3.11E+05 | 6.05E-03 | 1.95E-08 |
| | E2mut5-91Q-F405L-LF | 4.70E+05 | 9.46E-03 | 2.01E-08 |
| | E2mut12-91Q-F405L-LF | 3.27E+05 | 1.64E-03 | 5.03E-09 |
| | E2mut13-91Q-F405L-LF | 3.50E+05 | 2.78E-03 | 7.94E-09 |
| | E2mut16-91Q-F405L-LF | 7.22E+04 | 2.78E-03 | 3.85E-08 |
| | E2mut17-91Q-F405L-LF | 8.76E+04 | 2.28E-03 | 2.60E-08 |
| | E2mut25-91Q-F405L-LF | 5.99E+04 | 1.19E-03 | 1.99E-08 |
| | E2mut34-91Q-F405L-LF | 5.18E+05 | 3.62E-03 | 6.99E-09 |
| | E2mut38-91Q-F405L-LF | 5.92E+05 | 1.47E-02 | 2.48E-08 |

| Antigen name | Antibody name | kₐ (1/Ms) | k_{d} (1/s) | K_{D} (M) |
|---|---|---|---|---|
| c-Met | JNJ-372 | 2.20E+05 | 9.15E-05 | 4.17E-10 |
| | E2mut1-91Q×M5-91Q-FAE-LF | 1.96E+05 | 1.28E-04 | 6.51E-10 |
| | E2mut12-91Q×M5-91Q-FAE-LF | 1.89E+05 | 1.34E-04 | 7.06E-10 |
| | E2mut13-91Q×M5-91Q-FAE-LF | 1.95E+05 | 1.29E-04 | 6.62E-10 |
| | E2mut25-91Q×M5-91Q-FAE-LF | 1.88E+05 | 1.28E-04 | 6.84E-10 |
| | M5-91Q-K409R-LF | 2.60E+05 | 1.09E-04 | 4.18E-10 |

### Example 11: Cell Binding Assay of Bispecific Antibody Mutants

293F-EGFR and 293-cMet cells were incubated with various concentrations of JNJ372, E2mut1-91Q×M5-91Q-LF, E2mut12-91Q×M5-91Q-LF, E2mut13-91Q×M5-91Q-LF, E2mut25-91Q×M5-91Q-LF, and an anti-KLH IgG1 control antibody (starting concentration: 100 µg/mL; 5-fold serial dilutions were performed to yield 12 concentration points) at 4°C for 30 minutes. Unbound antibodies were removed by washing with staining buffer (PBS + 1% v/v FBS). The cells were then incubated with a fluorescent secondary antibody, goat anti-human IgG PE (SouthernBiotech, Cat#2040-09), diluted in staining buffer to a final concentration of 5% (v/v), for 30 minutes at 4°C in the dark. Finally, cells were collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and EC50 values were calculated using GraphPad software.

MKN45 cells (which endogenously co-express human EGFR and human cMet and were used to assess the cooperative binding affinity of the bispecific antibodies) were incubated with various concentrations of JNJ372, E2mut12-91Q×M5-91Q-LF, E2mut13-91Q×M5-91Q-LF, and an anti-KLH IgG1 control antibody (starting concentration: 100 µg/mL; 4-fold serial dilutions were performed to yield 11 concentration points) at 4°C for 30 minutes. Unbound antibodies were removed by washing with staining buffer (PBS + 1% v/v FBS). The cells were then incubated with a fluorescent secondary antibody, goat anti-human IgG PE (SouthernBiotech, Cat#2040-09), diluted in staining buffer to a final concentration of 5% (v/v), for 30 minutes at 4°C in the dark. Finally, cells were collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and EC50 values were calculated using GraphPad software.

PC-9 cells (which endogenously co-express human EGFR and human cMet and were used to assess the cooperative binding affinity of the bispecific antibodies) were incubated with various concentrations of JNJ372, E2mut1-91Q×M5-91Q-LF, E2mut12-91Q×M5-91Q-LF, E2mut13-91Q×M5-91Q-LF, and an anti-KLH IgG1 control antibody (starting concentration: 25 µg/mL; 4-fold serial dilutions were performed to yield 8 concentration points) at 4°C for 30 minutes. Unbound antibodies were removed by washing with staining buffer (PBS + 1% v/v FBS). The cells were then incubated with a fluorescent secondary antibody, goat anti-human IgG PE (SouthernBiotech, Cat#2040-09), diluted in staining buffer to a final concentration of 5% (v/v), for 30 minutes at 4°C in the dark. Finally, cells were collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and EC50 values were calculated using GraphPad software.

As shown in Figures 10D to 10G, the EC50 values for the binding of JNJ372, E2mut1-91Q×M5-91Q-LF, E2mut12-91Q×M5-91Q-LF, E2mut13-91Q×M5-91Q-LF, and E2mut25-91Q×M5-91Q-LF to 293F-EGFR cells were 203.6 ng/mL, 70.01 ng/mL, 67.48 ng/mL, 55.53 ng/mL, and 337.4 ng/mL, respectively; their TOP values were 35956, 17708, 23177, 19445, and 23508, respectively. The EC50 values for the binding of JNJ372, E2mut1-91Q×M5-91Q-LF, E2mut12-91Q×M5-91Q-LF, E2mut13-91Q×M5-91Q-LF, and E2mut25-91Q×M5-91Q-LF to 293F-cMet cells were 24.13 ng/mL, 25.80 ng/mL, 26.95 ng/mL, 25.99 ng/mL, and 28.22 ng/mL, respectively. The EC50 values for the binding of JNJ372, E2mut12-91Q×M5-91Q-LF, and E2mut13-91Q×M5-91Q-LF to MKN45 cells were 65.77 ng/mL, 78.99 ng/mL, and 79.78 ng/mL, respectively. The EC50 values for the binding of E2mut1-91Q×M5-91Q-LF, E2mut12-91Q×M5-91Q-LF, E2mut13-91Q×M5-91Q-LF, and JNJ372 to PC-9 cells were 324.4 ng/mL, 142.0 ng/mL, 149.2 ng/mL, and 194.0 ng/mL, respectively. These results showed that the binding affinities of E2mut1-91Q×M5-91Q-LF, E2mut12-91Q×M5-91Q-LF, E2mut13-91Q×M5-91Q-LF, and E2mut25-91Q×M5-91Q-LF to the single antigen EGFR were weaker than that of the positive control, JNJ-372; their binding affinities to the single antigen cMet were comparable to that of the positive control, JNJ-372; and their binding affinities to the dual antigens EGFR & cMet were comparable to that of the positive control, JNJ-372.

### Example 12: Phosphorylation inhibition assay of bispecific antibody mutants

NCI-H1975 cells were digested, centrifuged, and resuspended in complete medium (RPMI-1640 medium + 10% v/v FBS). The cells were seeded into a 96-well flat-bottom plate at a density of 2 × 10⁴ cells per well and incubated overnight in a CO₂ incubator. On the second day, the medium in the 96-well plate was replaced with serum-free RPMI-1640 medium, and the cells were incubated overnight in the CO₂ incubator. On the third day, antibodies JNJ372, E2mut1-91Q×M5-91Q-LF, E2mut12-91Q×M5-91Q-LF, E2mut13-91Q×M5-91Q-LF, and E2mut25-91Q×M5-91Q-LF were prepared at 2× assay concentrations using serum-free RPMI-1640 medium (starting concentration: 500 µg/mL; 3-fold serial dilutions, 12 concentration points). The medium was removed from the 96-well plate, and 50 µL of the diluted antibodies prepared above was added to each well, followed by pre-incubation at 37°C for 30 minutes. The agonist, EGF (at a concentration of 8 ng/mL), was prepared at 2× assay concentration using serum-free RPMI-1640 medium and kept in the incubator until use. After pre-incubation, the 96-well flat-bottom plate was removed from the incubator, and 50 µL per well of the diluted EGF agonist solution was added. Incubation was continued at 37°C for 30 minutes. Following incubation, the 96-well flat-bottom plate was removed, and the supernatant was discarded. Then, 50 µL of 1× lysis buffer was added according to the kit instructions (phospho-AKT (Ser473) kit (Cisbio, Cat. No. 64AKSPEH) & Advanced phospho-ERK1/2 (Thr202/Tyr204) (Cisbio, Cat. No. 64AERPEH)). The plate was shaken at room temperature at 350 rpm for 30 minutes. Subsequently, 16 µL of cell lysate and 4 µL of the antibody mix from the kits were added to HTRF 96-well low volume plates (Cisbio, Cat. No. 66PL96025). After gently mixing with a pipette, the plate was sealed with film and centrifuged at 1000 rpm for 30 seconds. Finally, the plate was incubated protected from light at room temperature (20°C-25°C). The absorbance was measured at wavelengths 655 nm and 620 nm within 4-24 hours using a microplate reader (BioTek, Synergy H1 model). The ratio (Ratio 665/620) was calculated using the formula: Ratio 665/620 = (A655 / A620) × 10000. Data analysis for the Ratio 665/620 was performed using GraphPad Prism software.

BxPC-3 cells were digested, centrifuged, and resuspended in complete medium (RPMI-1640 medium + 10% v/v FBS). The cells were seeded into a 96-well flat-bottom plate at a density of 2 × 10⁴ cells per well and incubated overnight in a CO₂ incubator. On the second day, the medium in the 96-well plate was replaced with serum-free RPMI-1640 medium, and the cells were incubated overnight in the CO₂ incubator. On the third day, antibodies JNJ372, E2mut1-91Q×M5-91Q-LF, E2mut12-91Q×M5-91Q-LF, E2mut13-91Q×M5-91Q-LF, E2mut25-91Q×M5-91Q-LF, and anti-KLH IgG1 were prepared at 2× assay concentrations using serum-free RPMI-1640 medium (starting concentration: 20 µg/mL; 3-fold serial dilutions, 10 concentration points). The medium was removed from the 96-well plate, and 50 µL of the diluted antibodies prepared above was added to each well, followed by pre-incubation at 37°C for 30 minutes. The agonist, HGF (at a concentration of 300 ng/mL), was prepared at 2× assay concentration using serum-free RPMI-1640 medium and kept in the incubator until use. After pre-incubation, the 96-well flat-bottom plate was removed from the incubator, and 50 µL per well of the diluted HGF agonist solution was added. Incubation was continued at 37°C for 30 minutes. Following incubation, the 96-well flat-bottom plate was removed, and the supernatant was discarded. Then, 50 µL of 1× lysis buffer was added according to the kit instructions (phospho-AKT (Ser473) kit (Cisbio, Cat. No. 64AKSPEH) & Advanced phospho-ERK1/2 (Thr202/Tyr204) (Cisbio, Cat. No. 64AERPEH)). The plate was shaken at room temperature at 350 rpm for 30 minutes. Subsequently, 16 µL of cell lysate and 4 µL of the antibody mix from the kits were added to HTRF 96-well low volume plates (Cisbio, Cat. No. 66PL96025). After gently mixing with a pipette, the plate was sealed with film and centrifuged at 1000 rpm for 30 seconds. Finally, the plate was incubated protected from light at room temperature (20°C-25°C). The absorbance was measured at wavelengths 655 nm and 620 nm within 4-24 hours using a microplate reader (BioTek, Synergy H1 model). The ratio (Ratio 665/620) was calculated using the formula: Ratio 665/620 = (A655 / A620) × 10000. Data analysis for the Ratio 665/620 was performed using GraphPad Prism software.

As shown in Figures 11F to 11M, under EGF-stimulated conditions, the IC₅₀ values for the inhibition of AKT phosphorylation by JNJ372, E2mut1-91Q×M5-91Q-LF, E2mut12-91Q×M5-91Q-LF, E2mut13-91Q×M5-91Q-LF, and E2mut25-91Q×M5-91Q-LF were 1377 ng/mL, 464713 ng/mL, 2988 ng/mL, 8899 ng/mL, and 111137 ng/mL, respectively; the IC₅₀ values for the inhibition of ERK phosphorylation were 3383 ng/mL, 4711189 ng/mL, 8496 ng/mL, 42459 ng/mL, and 575652 ng/mL, respectively. These results indicated that, under EGF-stimulated conditions, the activity of E2mut12-91Q×M5-91Q-LF in inhibiting AKT and ERK phosphorylation was slightly weaker than that of the positive control, JNJ-372, while the activities of E2mut1-91Q×M5-91Q-LF, E2mut13-91Q×M5-91Q-LF, and E2mut25-91Q×M5-91Q-LF in inhibiting AKT and ERK phosphorylation were significantly weaker than that of the positive control, JNJ-372. Under HGF-stimulated conditions, the IC₅₀ values for the inhibition of AKT phosphorylation by JNJ372, E2mut1-91Q×M5-91Q-LF, E2mut12-91Q×M5-91Q-LF, E2mut13-91Q×M5-91Q-LF, and E2mut25-91Q×M5-91Q-LF were 296.9 ng/mL, 410.4 ng/mL, 268.3 ng/mL, 404.5 ng/mL, and 766.3 ng/mL, respectively; the IC₅₀ values for the inhibition of ERK phosphorylation were 457.8 ng/mL, 547.6 ng/mL, 517.9 ng/mL, 535.0 ng/mL, and 1312 ng/mL, respectively. These results indicated that, under HGF-stimulated conditions, the activities of E2mut1-91Q×M5-91Q-LF, E2mut12-91Q×M5-91Q-LF, and E2mut13-91Q×M5-91Q in inhibiting AKT and ERK phosphorylation were comparable to those of the positive control, JNJ-372, while the activity of E2mut25-91Q×M5-91Q-LF was slightly weaker than that of the positive control, JNJ-372.

### Example 13: ADCC activity assay of bispecific antibody mutants

Reporter Gene method: NCI-H1975 cells were seeded into a 96-well white plate (Costar, Cat. No. 3917) at a density of 5 × 10⁴ cells per well and incubated overnight in a CO₂ incubator. On the following day, the 96-well white plate was removed from the CO₂ incubator, the culture supernatant was discarded, and 40 µL per well of antibodies diluted in assay buffer (RPMI-1640 medium + 2% v/v FBS) was added. The antibodies tested were E2mut1-91Q×M5-91Q-LF, E2mut12-91Q×M5-91Q-LF, E2mut13-91Q×M5-91Q-LF, JNJ-372, and anti-KLH IgG1 (starting concentration: 10 µg/mL; 4-fold serial dilutions to yield 10 concentration points). The antibodies were pre-incubated with the target cells for 30 minutes. After pre-incubation, 40 µL per well of effector Jurkat ADCC cells (expressing NFAT-Luc and FcγRIIIa) resuspended in assay buffer (RPMI-1640 medium + 2% v/v FBS) was added at a density of 1 × 10⁵ cells per well. Incubation was continued for 6 hours at 37°C. Finally, One-Lite substrate (Vazyme, Cat. No. DD1203-03) was added, and the luciferase signal was detected using a microplate reader (TECAN, M1000 pro model). A higher fluorescence reading indicated a stronger ADCC effect. Antibody dose-dependent ADCC effect curves were fitted using GraphPad software.

LDH method: PBMC effector cells were first revived and allowed to recover overnight in a CO₂ incubator. Target NCI-H1975 cells were digested and centrifuged; the supernatant was discarded, and the cell density was adjusted to 4 × 10⁵ cells/mL using phenol red-free RPMI Medium 1640 supplemented with 1% v/v FBS. Then, 50 µL of target cells was added to each well of a 96-well low-attachment plate (Costar, Cat. No. 7007). Subsequently, antibodies E2mut1-91Q×M5-91Q-LF, E2mut12-91Q×M5-91Q-LF, E2mut13-91Q×M5-91Q-LF, JNJ-372, and anti-KLH IgG1 were diluted to 4× assay concentrations using phenol red-free RPMI Medium 1640 + 1% FBS (antibody starting concentration: 66.67 µg/mL; 3-fold serial dilutions for the first 2 concentration points; 5-fold serial dilutions for the subsequent 7 concentration points). Then, 50 µL per well of the diluted antibody solution was added, gently mixed by pipetting several times, and incubated in the incubator for 30 minutes. The revived and overnight-recovered PBMCs were taken, and their density was adjusted to 5 × 10⁶ cells/mL (effector-to-target ratio: 25:1). Next, 100 µL of PBMCs was added to each well of the 96-well low-attachment plate and co-cultured in a CO₂ incubator for 4 hours. Thirty minutes before the end of the co-culture period, 20 µL per well of the cell lysis buffer from the LDH-cytotoxicity kit (BioVision, Cat. No. K311-400) was added to the Tmax group wells, and the 96-well low-attachment plate was returned to the incubator for continued incubation. The 96-well low-attachment plate was then removed and centrifuged (1500 rpm × 5 minutes). Subsequently, 50 µL of culture supernatant from each well was transferred to a new 96-well flat-bottom plate (Corning, Cat. No. 3599). Then, 50 µL of the prepared Dye Solution from the LDH-cytotoxicity kit was added to each well, and the plate was incubated on a shaker protected from light for 10-30 minutes. Finally, the plate was read using a multimode microplate reader (TECAN, M1000 pro model), and data were analyzed using GraphPad Prism software. The cytotoxicity percentage was calculated using the formula: ADCC % = [(Sample - Buffer) / (Tmax - Ts)] × 100.

As shown in Figure 12D, in the reporter gene assay, E2mut1-91Q×M5-91Q-LF, E2mut12-91Q×M5-91Q-LF, and E2mut13-91Q×M5-91Q-LF all exhibited ADCC activity, with EC50 values of 50.92 ng/mL, 53.05 ng/mL, and 42.70 ng/mL, respectively. Their ADCC activity was slightly weaker than that of the positive control, JNJ-372.

As shown in Figure 12E, when PBMC cells were used as effector cells and NCI-H1975 cells were used as target cells, E2mut1-91Q×M5-91Q-LF, E2mut12-91Q×M5-91Q-LF, and E2mut13-91Q×M5-91Q-LF all exhibited ADCC activity, with EC50 values of 33.66 ng/ml, 11.95 ng/ml, and 28.39 ng/ml, respectively. Their ADCC activity was comparable to that of the positive control JNJ-372. However, the negative control anti-KLH IgG1 did not induce an ADCC effect.

### Example 14: Internalization assay of bispecific antibody mutants

MKN45 cells were digested, centrifuged, and resuspended in staining buffer (PBS + 1% v/v FBS). The cells were seeded into a 96-well round-bottom plate (Corning, Cat. No. 3799) at a density of 2 × 10⁵ cells per well. Antibodies E2mut12-91Q×M5-91Q-LF, E2mut13-91Q×M5-91Q-LF, and JNJ-372 (25 µg/mL) diluted in staining buffer (PBS + 1% v/v FBS) were added and incubated at 4°C for 30 minutes. Unbound antibodies were then removed by washing with staining buffer (PBS + 1% v/v FBS). The cells were resuspended in 200 µL of complete medium (RPMI-1640 medium + 10% v/v FBS) and split into two equal portions: one portion was incubated at 4°C for 24 hours, and the other portion was incubated at 37°C for 24 hours. After the incubation period, the plates were centrifuged, the supernatant was discarded, and a fluorescent secondary antibody, goat anti-human IgG PE (SouthernBiotech, Cat#2040-09), diluted in staining buffer to a final concentration of 5% (v/v), was added. The cells were incubated at 4°C for 30 minutes in the dark. Subsequently, unbound antibodies were removed by washing with staining buffer (PBS + 1% v/v FBS). The cells were fixed with PFA (Absin, Cat. No. abs9179) and then collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and Top values were calculated using GraphPad software. The internalization rate was calculated using the formula: Internalization Rate (%) = (1 - Top_37°C / Top_4°C) × 100.

The results are shown in Table 10.

**Table 10: Results of endocytic activity assay in MKN45 cells 2**

| Group | Top_4°C | Top_37°C | Internalization Rate, % |
|---|---|---|---|
| JNJ-372 | 1273 | 324 | 75 |
| E2mut12-91Q×M5-91Q-FAE-LF | 1228 | 200 | 84 |
| E2mut13-91Q×M5-91Q-FAE-LF | 1171 | 235 | 80 |

The results showed that E2mut12-91Q×M5-91Q-FAE-LF, E2mut13-91Q×M5-91Q-FAE-LF, and JNJ-372 could all be internalized by MKN45 cells, and their endocytic activities were comparable, with endocytosis rates of 75%, 84%, and 80% at 24 h, respectively.

PC-9 cells were digested, centrifuged, and resuspended in staining buffer (PBS + 1% v/v FBS). The cells were seeded into a 96-well round-bottom plate (Corning, Cat. No. 3799) at a density of 2 × 10⁵ cells per well. Antibodies E2mut1-91Q×M5-91Q-LF, E2mut12-91Q×M5-91Q-LF, E2mut13-91Q×M5-91Q-LF, and JNJ-372 (25 µg/mL) diluted in staining buffer (PBS + 1% v/v FBS) were added and incubated at 4°C for 30 minutes. Unbound antibodies were then removed by washing with staining buffer (PBS + 1% v/v FBS). The cells were resuspended in 200 µL of complete medium (RPMI-1640 medium + 10% v/v FBS) and split into two equal portions: one portion was incubated at 4°C for 24 hours, and the other portion was incubated at 37°C for 24 hours. After incubation, the plates were centrifuged, the supernatant was discarded, and a fluorescent secondary antibody, goat anti-human IgG PE (SouthernBiotech, Cat#2040-09), diluted in staining buffer to a final concentration of 5% (v/v), was added. The cells were incubated at 4°C for 30 minutes in the dark. Subsequently, unbound antibodies were removed by washing with staining buffer (PBS + 1% v/v FBS). The cells were fixed with PFA (Absin, Cat. No. abs9179) and then collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and Top values were calculated using GraphPad software. The internalization rate was calculated using the formula: Internalization Rate (%) = (1 - Top_37°C / Top_4°C) × 100.

The results are shown in Table 11.

**Table 11: Results of endocytosis activity assay in PC-9 cells**

| Group | Top_4°C | Top_37°C | Internalization rate, % |
|---|---|---|---|
| JNJ-372 | 2284 | 425 | 81 |
| E2mut1-91Q×M5-91Q-FAE-LF | 2749 | 335 | 88 |
| E2mut12-91Q×M5-91Q-FAE-LF | 2992 | 403 | 87 |
| E2mut13-91Q×M5-91Q-FAE-LF | 3159 | 459 | 85 |

The results showed that E2mut1-91Q×M5-91Q-FAE-LF, E2mut12-91Q×M5-91Q-FAE-LF, E2mut13-91Q×M5-91Q-FAE-LF, and JNJ-372 were all internalized by MKN45 cells, and their internalization activities were comparable, with internalization rates at 24 hours of 88%, 87%, 85%, and 81%, respectively.

NUGC4 cells (endogenously expressing human EGFR and human cMet) and BaF3-EGFR-cMet cells (overexpressing human EGFR and human cMet) were digested, centrifuged, and resuspended in staining buffer (PBS + 1% v/v FBS). The cells were seeded into a 96-well round-bottom plate (Corning, Cat. No. 3799) at a density of 2 × 10⁵ cells per well. Antibodies E2mut1-91Q×M5-91Q-LF, E2mut12-91Q×M5-91Q-LF, E2mut13-91Q×M5-91Q-LF, E2mut25-91Q×M5-91Q-LF, and JNJ-372 (100 µg/mL) diluted in staining buffer (PBS + 1% v/v FBS) were added and incubated at 4°C for 30 minutes. Unbound antibodies were then removed by washing with staining buffer (PBS + 1% v/v FBS). The cells were resuspended in 200 µL of complete medium (RPMI-1640 medium + 10% v/v FBS) and split into two equal portions: one portion was incubated at 4°C for 24 hours, and the other portion was incubated at 37°C for 24 hours. After the incubation period, the plates were centrifuged, the supernatant was discarded, and a fluorescent secondary antibody, goat anti-human IgG PE (SouthernBiotech, Cat#2040-09), diluted in staining buffer to a final concentration of 5% (v/v), was added. The cells were incubated at 4°C for 30 minutes in the dark. Subsequently, unbound antibodies were removed by washing with staining buffer (PBS + 1% v/v FBS). The cells were fixed with PFA (Absin, Cat. No. abs9179) and then collected using a flow cytometer (BD, C6 PLUS model) to detect fluorescent antibodies bound to the cell surface. Raw data were analyzed using FlowJo software to obtain mean fluorescence intensity (MFI) values. Antibody dose-dependent binding curves were fitted, and Top values were calculated using GraphPad software. The internalization rate was calculated using the formula: Internalization Rate (%) = (1 - Top_37°C / Top_4°C) × 100.

The results are shown in Tables 12 and 13.

**Table 12: Results of endocytic activity assay in NUGC4 cells**

| Group | Top_4°C | Top_37°C | Internalization rate, % |
|---|---|---|---|
| JNJ-372 | 7348 | 2949 | 60 |
| E2mut1-91Q×M5-91Q-FAE-LF | 5059 | 2356 | 53 |
| E2mut12-91Q×M5-91Q-FAE-LF | 5940 | 1958 | 67 |
| E2mut13-91Q×M5-91Q-FAE-LF | 5485 | 2377 | 57 |
| E2mut25-91Q×M5-91Q-FAE-LF | 6440 | 2050 | 68 |

**Table 13: Results of endocytic activity assay in BaF3-EGFR-cMet cells**

| Group | Top_4°C | Top_37°C | Internalization rate, % |
|---|---|---|---|
| JNJ-372 | 13553 | 2608 | 81 |
| E2mut1-91Q×M5-91Q-FAE-LF | 24752 | 3907 | 84 |
| E2mut12-91Q×M5-91Q-FAE-LF | 18826 | 4428 | 76 |
| E2mut13-91Q×M5-91Q-FAE-LF | 18101 | 4804 | 73 |
| E2mut25-91Q×M5-91Q-FAE-LF | 14777 | 3578 | 76 |

The results showed that E2mut1-91Q×M5-91Q-FAE-LF, E2mut12-91Q×M5-91Q-FAE-LF, E2mut13-91Q×M5-91Q-FAE-LF, E2mut25-91Q×M5-91Q-FAE-LF, and JNJ-372 could all be internalized by NUGC4 cells and BaF3-EGFR-cMet cells.

### Example 15: H1975 xenograft model with bispecific antibody mutant

In this example, the inhibitory effects of E2mut34-91A×M5-91A-FAE-LF of the invention, as well as ALK101-15Q-LF, ALK101-17Q-LF, and ALK101-31Q-LF, on an NDG mouse model transplanted with human lung adenocarcinoma NCI-H1975 cells were evaluated. ALK101-15Q-LF, ALK101-17Q-LF, and ALK101-31Q-LF were selected from the bispecific antibodies listed in Table 8.

### 1. Testing Procedure

Female NDG mice, aged 6-8 weeks (purchased from Shanghai Jihui Laboratory Animal Breeding Co., Ltd., Animal Certificate No. 20170012022482), were inoculated subcutaneously in the right flank with 5 × 10⁶ NCI-H1975 cells. When the mean tumor volume reached approximately 125 mm³, suitable animals were selected and randomly divided into 6 groups (n = 6 per group) based on tumor volume. The groups were as follows:
G1: Normal saline control group (vehicle control group);
G2: JNJ-372 (3 mg/kg) group (positive control group);
G3: E2mut34-91A×M5-91A-FAE-LF (3 mg/kg) group (treatment group);
G4: ALK101-15Q-LF (3 mg/kg) group (treatment group);
G5: ALK101-31Q-LF (3 mg/kg) group (treatment group);
G6: ALK101-17Q-LF (3 mg/kg) group (treatment group).

Administration was performed via intraperitoneal injection twice weekly for 2 consecutive weeks. The experiment was terminated 28 days after the initial dose. Tumor volume and body weight were measured twice weekly, and the data were recorded. At the end of the experiment, the mice were euthanized. The relative tumor proliferation rate was calculated using the formula: TGI (%) = [1 - (Ti - T0) / (Vi - V0)] × 100% (Ti: the mean tumor volume of the treatment group on day i of dosing, T0: the mean tumor volume of the treatment group on day 0 of dosing, Vi: the mean tumor volume of the vehicle control group on day i of dosing, V0: the mean tumor volume of the vehicle control group on day 0 of dosing).

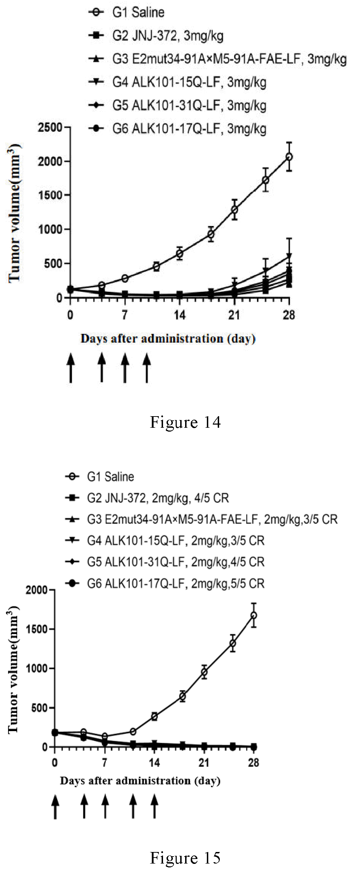

The results are shown in Table 14 and Figure 14.

**Table 14: Efficacy Analysis of Each Group in the NCI-H1975 Human Lung Cancer Subcutaneous Tumor Model**

| Group | Day 28 after administration | | |
|---|---|---|---|
| | Tumor volume(X̅±S) | TGI (%) | P Value (compared to the control group) |
| G1, normal saline | 2070±504 | - | - |
| G2, JNJ-372, 3mg/kg | 272±275 | 92.5 | <0.001 |
| G3, E2mut34-91A×M5-91A-FAE-LF, 3mg/kg | 223±158 | 95 | <0.001 |
| G4, ALK101-15Q-LF, 3mg/kg | 600±652 | 75.6 | 0.001 |
| G5, ALK101-31Q-LF, 3mg/kg | 348±243 | 88.5 | <0.001 |
| G6, ALK101-17Q-LF, 3mg/kg | 394±268 | 86.1 | <0.001 |

| | | | |
|---|---|---|---|
| Note: 1. Data are presented as "Mean ± SEM"; 2. TGI% = [1-(Ti-T0)/(Vi-V0)]×100%; 3. The p-value was obtained by comparing the tumor volume of each group using the t-test method. | | | |

The results showed that on day 28 after administration, the mean tumor volume in the saline control group was 2070 mm³. The mean tumor volume in the JNJ-372 (3 mg/kg) group was 272 mm3, with a tumor inhibition rate of 92.5% compared to the saline control group, significantly inhibiting tumor growth. The mean tumor volume in the E2mut34-91A×M5-91A-FAE-LF (3 mg/kg) group was 223 mm³, with a tumor inhibition rate of 95.0% compared to the saline control group, significantly inhibiting tumor growth. The mean tumor volume in the ALK101-15Q-LF (3 mg/kg) group was 600 mm³, with a tumor inhibition rate of 75.6% compared to the saline control group, significantly inhibiting tumor growth. The mean tumor volume in the ALK101-31Q-LF (3 mg/kg) group was 348 mm³, with a tumor inhibition rate of 88.5% compared to the saline control group, significantly inhibiting tumor growth. The mean tumor volume in the ALK101-17Q-LF (3 mg/kg) group was 394 mm³, and the tumor inhibition rate was 86.1% compared with the saline control group, significantly inhibiting tumor growth. These results indicate that in the NDG mouse NCI-H1975 transplantation model, at a dose level of 3 mg/kg, E2mut34-91A×M5-91A-FAE-LF, ALK101-15Q-LF, ALK101-17Q-L, and ALK101-31Q-LF exhibited significant tumor-inhibiting effects.

### Example 16: Xenograft model of bispecific antibody variant H1975 (L858R/T790M/C797S)

In this example, the inhibitory effects of E2mut34-91A×M5-91A-FAE-LF of the invention, as well as ALK101-15Q-LF, ALK101-17Q-LF, and ALK101-31Q-LF, on an NDG mouse model transplanted with human lung adenocarcinoma NCI-H1975 (L858R/T790M/C797S) cells were evaluated.

### 1. Testing Procedure

Female NDG mice, aged 6-8 weeks (purchased from Shanghai Jihui Laboratory Animal Breeding Co., Ltd., Animal Certificate No. 20170012022482), were inoculated subcutaneously in the right flank with 5 × 10⁶ NCI-H1975 cells. When the mean tumor volume reached approximately 186 mm³, suitable animals were selected and randomly divided into 6 groups (n = 5 per group) based on tumor volume. The groups were as follows:
G1: Normal saline control group (vehicle control group);
G2: JNJ-372 (2 mg/kg) group (positive control group);
G3: E2mut34-91A×M5-91A-FAE-LF (2 mg/kg) group (treatment group);
G4: ALK101-15Q-LF (2 mg/kg) group (treatment group);
G5: ALK101-31Q-LF (2 mg/kg) group (treatment group);
G6: ALK101-17Q-LF (2 mg/kg) group (treatment group).

Administration was performed via intraperitoneal injection twice weekly for a total of 5 doses. The experiment was terminated 28 days after the initial dose. Tumor volume and body weight were measured twice weekly, and the data were recorded. At the end of the experiment, the mice were euthanized. The relative tumor proliferation rate was calculated using the formula: TGI (%) = [1 - (Ti - T0) / (Vi - V0)] × 100% (Ti: the mean tumor volume of the treatment group on day i of dosing, T0: the mean tumor volume of the treatment group on day 0 of dosing, Vi: the mean tumor volume of the vehicle control group on day i of dosing, V0:the mean tumor volume of the vehicle control group on day 0 of dosing).

The results are shown in Table 15 and Figure 15.

**Table 15: Efficacy Analysis of Each Group in the H1975 (L858R/T790M/C797S) Human Lung Cancer Subcutaneous Tumor Model**

| Group | Day 28 after administration | | |
|---|---|---|---|
| | Tumor volume( *x̅* ±S) | TGI (%) | P Value (compared to the control group) |
| G1, Normal saline | 1678±342 | - | - |
| G2, JNJ-372, 2mg/kg | 4±8 | 112.2 | <0.001 |
| G3, E2mut34-91A×M5-91A-FAE-LF, 2mg/kg | 7±10 | 112 | <0.001 |
| G4, ALK101-15Q-LF, 2mg/kg | 9±14 | 111.9 | <0.001 |
| G5, ALK101-31Q-LF, 2mg/kg | 6±13 | 112.1 | <0.001 |
| G6, ALK101-17Q-LF, 2mg/kg | 0±0 | 112.5 | <0.001 |

| | | | |
|---|---|---|---|
| Note: 1. Data are presented as "Mean ± SEM"; 2. TGI% = [1-(Ti-T0)/(Vi-V0)]×100%; 3. P-value was obtained by comparing the tumor volume of each group using the t-test method. | | | |

The results showed that on day 28 after administration, the mean tumor volume in the normal saline control group was 1678 mm³. In the JNJ-372 (2 mg/kg) group, the mean tumor volume was 4 mm³, corresponding to a tumor growth inhibition (TGI) rate of 112.2% compared to the normal saline control group, indicating significant inhibition of tumor growth. In the E2mut34-91A×M5-91A-FAE-LF (2 mg/kg) group, the mean tumor volume was 7 mm³, achieving a TGI rate of 112.0% compared to the normal saline control group, demonstrating significant tumor growth inhibition. In the ALK101-15Q-LF (2 mg/kg) group, the mean tumor volume was 9 mm³, with a TGI rate of 111.9% compared to the normal saline control group, indicating significant tumor growth inhibition. In the ALK101-31Q-LF (2 mg/kg) group, the mean tumor volume was 6 mm³, achieving a TGI rate of 112.1% compared to the normal saline control group, demonstrating significant tumor growth inhibition. In the ALK101-17Q-LF (2 mg/kg) group, the mean tumor volume was 0 mm³, with a TGI rate of 112.5% compared to the normal saline control group, indicating significant tumor growth inhibition. These results demonstrate that in the NDG mouse model transplanted with NCI-H1975 (L858R/T790M/C797S) cells, E2mut34-91A×M5-91A-FAE-LF, ALK101-15Q-LF, ALK101-17Q-LF, and ALK101-31Q-LF exhibited significant tumor inhibitory effects at a dose level of 2 mg/kg.

### Example 17: H292 Xenograft Model of Bispecific Antibody Mutants

In this example, the inhibitory effects of E2mut34-91A×M5-91A-FAE-LF of the invention, as well as ALK101-15Q-LF, ALK101-17Q-LF, and ALK101-31Q-LF, on an NDG mouse model transplanted with human lung adenocarcinoma H292 cells were evaluated.

### 1. Testing Procedure

Female NDG mice, aged 6-8 weeks (purchased from Shanghai Jihui Laboratory Animal Breeding Co., Ltd., Animal Certificate No. 20170012022482), were inoculated subcutaneously in the right flank with 5 × 10⁶ H292 cells. When the mean tumor volume reached approximately 223 mm³, suitable animals were selected and randomly divided into 6 groups (n = 5 per group) based on tumor volume. The groups were as follows:
G1: Normal saline control group (vehicle control group);
G2: JNJ-372 (1 mg/kg) group (positive control group);
G3: E2mut34-91A×M5-91A-FAE-LF (1 mg/kg) group (treatment group);
G4: ALK101-15Q-LF (1 mg/kg) group (treatment group);
G5: ALK101-31Q-LF (1 mg/kg) group (treatment group);
G6: ALK101-17Q-LF (1 mg/kg) group (treatment group).

Administration was performed via intraperitoneal injection once in the first week, and twice weekly from the second week onwards, for a total of 6 doses. The experiment was terminated 28 days after the initial dose. Tumor volume and body weight were measured twice weekly, and the data were recorded. At the end of the experiment, the mice were euthanized. The relative tumor proliferation rate was calculated using the formula: TGI (%) = [1 - (Ti - T0) / (Vi - V0)] × 100% (where Ti is the mean tumor volume of the treatment group on day i of dosing, T0 is the mean tumor volume of the treatment group on day 0 of dosing; Vi is the mean tumor volume of the vehicle control group on day i of dosing, and V0 is the mean tumor volume of the vehicle control group on day 0 of dosing).

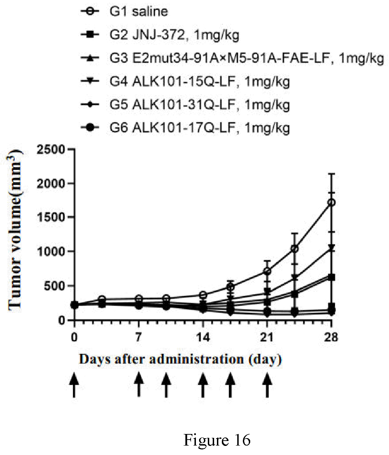

The results are shown in Table 16 and Figure 16.

**Table 16: Efficacy Analysis of Each Group in the H292 Human Lung Cancer Subcutaneous Tumor Model**

| Group | Day 28 after administration | | |
|---|---|---|---|
| | Tumor volume( *x̅* ±S) | TGI (%) | P Value (compared to the control group) |
| G1, Normal saline | 1713±960 | - | - |
| G2, JNJ-372, 1mg/kg | 625±854 | 73 | 0.095 |
| G3, E2mut34-91A×M5-91A-FAE-LF, 1mg/kg | 659±974 | 70.8 | 0.123 |
| G4, ALK101-15Q-LF, 1mg/kg | 1049±1832 | 44.6 | 0.493 |
| G5, ALK101-31Q-LF, 1mg/kg | 106±83 | 107.9 | 0.006 |
| G6, ALK101-17Q-LF, 1mg/kg | 152±119 | 104.7 | 0.007 |

| | | | |
|---|---|---|---|
| Note: 1. Data are presented as "Mean ± SEM"; 2. TGI% = [1-(Ti-T0)/(Vi-V0)] × 100%; 3. P-value was obtained by comparing the tumor volume of each group using the t-test method. | | | |

The results showed that on day after administration, the mean tumor volume in the normal saline control group was 1713 mm³. In the JNJ-372 (1 mg/kg) group, the mean tumor volume was 625 mm³, corresponding to a tumor growth inhibition (TGI) rate of 73% compared to the normal saline control group, indicating significant inhibition of tumor growth. In the E2mut34-91A×M5-91A-FAE-LF (1 mg/kg) group, the mean tumor volume was 659 mm³, achieving a TGI rate of 70.8% compared to the normal saline control group, demonstrating significant tumor growth inhibition. In the ALK101-15Q-LF (1 mg/kg) group, the mean tumor volume was 1049 mm³, with a TGI rate of 44.6% compared to the normal saline control group. In the ALK101-31Q-LF (1 mg/kg) group, the mean tumor volume was 106 mm³, achieving a TGI rate of 107.9% compared to the normal saline control group, indicating significant tumor growth inhibition. In the ALK101-17Q-LF (1 mg/kg) group, the mean tumor volume was 152 mm³, with a TGI rate of 104.7% compared to the normal saline control group, demonstrating significant tumor growth inhibition. These results indicate that in the NDG mouse model transplanted with H292 cells, E2mut34-91A×M5-91A-FAE-LF, ALK101-17Q-LF, and ALK101-31Q-LF exhibited significant tumor inhibitory effects at a dose level of 1 mg/kg.

### Example 18: Thermal Stability of Bispecific Antibodies

DSF (Differential Scanning Fluorimetry) technology is a method for detecting protein denaturation based on changes in intrinsic protein fluorescence. When a protein is in its folded state, the emission of its internal hydrophobic amino acids, such as tryptophan, is greater at 330 nm than at 350 nm. When the protein is subjected to heating or treatment with chemical denaturants, it unfolds, and tryptophan becomes exposed to the aqueous environment. At this point, the maximum emission wavelength shifts, and ultimately, the emission at 350 nm becomes greater than that at 330 nm. Therefore, the change in fluorescence value can be used to monitor the protein unfolding process induced by temperature or chemical denaturants, thereby allowing the calculation of the half-transition temperature, i.e., the Tm value. The stability of the bispecific antibody in a buffer system (20 mM histidine-histidine hydrochloride buffer, 150 mM arginine hydrochloride, pH 6.0) was investigated by setting 20°C as the starting temperature and 95°C as the final temperature, with a heating rate of 1°C/min.

The bispecific antibody samples were buffer-exchanged into the buffer system described above, and the sample concentration was adjusted to approximately 130 mg/mL. Detection was performed using nanoDSF. The results are shown in Table 17. The thermal transition temperatures (Tm) of the bispecific antibodies E2mut34-91A×M5-91A-FAE-LF, ALK101-15Q, ALK101-17Q, and ALK101-31Q provided in this application were all above 62°C, demonstrating good thermal stability.

**Table 17: Thermal Stability of Bispecific Antibodies**

| Sample | Tm1(°C) | Tm2(°C) | Tm3(°C) |
|---|---|---|---|
| E2mut34-91A×M5-91A-FAE-LF | 67.2 | 78.6 | / |
| ALK101-15Q | 62.5 | 70.6 | 76.7 |
| ALK101-17Q | 62.4 | 67.8 | 75.6 |
| ALK101-31Q | 65.8 | 74.3 | / |

| | | | |
|---|---|---|---|
| Note: "/" indicates does not exist. | | | |

Although the specific embodiments of the present invention have been described in detail, those skilled in the art will understand that various modifications and substitutions can be made to those details based on all the teachings that have been disclosed. Such changes fall within the protection scope of the present invention. The full scope of the invention is given by the appended claims and any equivalents thereof.

## Claims

1. An anti-EGFR antibody or an antigen-binding fragment thereof, comprising a heavy chain variable region and a light chain variable region, wherein the heavy chain variable region comprises HCDR1 to HCDR3, and the light chain variable region comprises LCDR1 to LCDR3, wherein:
the amino acid sequence of HCDR1 is as shown by SEQ ID NO: 25 or SEQ ID NO: 26, the amino acid sequence of HCDR2 is as shown by SEQ ID NO: 27, and the amino acid sequence of HCDR3 is as shown by SEQ ID NO: 37; and
the amino acid sequence of LCDR1 is as shown by SEQ ID NO: 38, the amino acid sequence of LCDR2 is as shown by SEQ ID NO: 39, and the amino acid sequence of LCDR3 is as shown by SEQ ID NO: 40, SEQ ID NO: 41, or SEQ ID NO: 76.

2. The anti-EGFR antibody or antigen-binding fragment thereof according to claim 1, wherein,
the amino acid sequence of HCDR3 is as shown by any one of SEQ ID NO: 28 to SEQ ID NO: 36.

3. The anti-EGFR antibody or antigen-binding fragment thereof according to claim 1, wherein,
the amino acid sequence of the heavy chain variable region of the anti-EGFR antibody is selected from any one of SEQ ID NO: 1 to SEQ ID NO: 10; and
the amino acid sequence of the light chain variable region of the anti-EGFR antibody is selected from SEQ ID NO: 42, SEQ ID NO: 43, SEQ ID NO: 44, and SEQ ID NO: 77.

4. The anti-EGFR or antigen-binding fragment thereof according to any one of claims 1 to 3, wherein,
the amino acid sequence of the heavy chain variable region is as shown by SEQ ID NO: 1, and the amino acid sequence of the light chain variable region is as shown by SEQ ID NO: 42;
the amino acid sequence of the heavy chain variable region is as shown by SEQ ID NO: 2, and the amino acid sequence of the light chain variable region is as shown by SEQ ID NO: 42;
the amino acid sequence of the heavy chain variable region is as shown by SEQ ID NO: 3, and the amino acid sequence of the light chain variable region is as shown by SEQ ID NO: 42;
the amino acid sequence of the heavy chain variable region is as shown by SEQ ID NO: 4, and the amino acid sequence of the light chain variable region is as shown by SEQ ID NO: 42;
the amino acid sequence of the heavy chain variable region is as shown by SEQ ID NO: 5, and the amino acid sequence of the light chain variable region is as shown by SEQ ID NO: 42;
the amino acid sequence of the heavy chain variable region is as shown by SEQ ID NO: 6, and the amino acid sequence of the light chain variable region is as shown by SEQ ID NO: 42;
the amino acid sequence of the heavy chain variable region is as shown by SEQ ID NO: 7, and the amino acid sequence of the light chain variable region is as shown by SEQ ID NO: 42;
the amino acid sequence of the heavy chain variable region is as shown by SEQ ID NO: 8, and the amino acid sequence of the light chain variable region is as shown by SEQ ID NO: 42;
the amino acid sequence of the heavy chain variable region is as shown by SEQ ID NO: 9, and the amino acid sequence of the light chain variable region is as shown by SEQ ID NO: 42;
the amino acid sequence of the heavy chain variable region is as shown by SEQ ID NO: 10, and the amino acid sequence of the light chain variable region is as shown by SEQ ID NO: 42;
the amino acid sequence of the heavy chain variable region is as shown by SEQ ID NO: 1, and the amino acid sequence of the light chain variable region is as shown by SEQ ID NO: 43;
the amino acid sequence of the heavy chain variable region is as shown by SEQ ID NO: 2, and the amino acid sequence of the light chain variable region is as shown by SEQ ID NO: 43;
the amino acid sequence of the heavy chain variable region is as shown by SEQ ID NO: 3, and the amino acid sequence of the light chain variable region is as shown by SEQ ID NO: 43;
the amino acid sequence of the heavy chain variable region is as shown by SEQ ID NO: 4, and the amino acid sequence of the light chain variable region is as shown by SEQ ID NO: 43;
the amino acid sequence of the heavy chain variable region is as shown by SEQ ID NO: 5, and the amino acid sequence of the light chain variable region is as shown by SEQ ID NO: 43;
the amino acid sequence of the heavy chain variable region is as shown by SEQ ID NO: 6, and the amino acid sequence of the light chain variable region is as shown by SEQ ID NO: 43;
the amino acid sequence of the heavy chain variable region is as shown by SEQ ID NO: 7, and the amino acid sequence of the light chain variable region is as shown by SEQ ID NO: 43;
the amino acid sequence of the heavy chain variable region is as shown by SEQ ID NO: 8, and the amino acid sequence of the light chain variable region is as shown by SEQ ID NO: 43;
the amino acid sequence of the heavy chain variable region is as shown by SEQ ID NO: 9, and the amino acid sequence of the light chain variable region is as shown by SEQ ID NO: 43;
the amino acid sequence of the heavy chain variable region is as shown by SEQ ID NO: 10, and the amino acid sequence of the light chain variable region is as shown by SEQ ID NO: 43;
the amino acid sequence of the heavy chain variable region is as shown by SEQ ID NO: 1, and the amino acid sequence of the light chain variable region is as shown by SEQ ID NO: 44;
the amino acid sequence of the heavy chain variable region is as shown by SEQ ID NO: 2, and the amino acid sequence of the light chain variable region is as shown by SEQ ID NO: 44;
the amino acid sequence of the heavy chain variable region is as shown by SEQ ID NO: 3, and the amino acid sequence of the light chain variable region is as shown by SEQ ID NO: 44;
the amino acid sequence of the heavy chain variable region is as shown by SEQ ID NO: 4, and the amino acid sequence of the light chain variable region is as shown by SEQ ID NO: 44;
the amino acid sequence of the heavy chain variable region is as shown by SEQ ID NO: 5, and the amino acid sequence of the light chain variable region is as shown by SEQ ID NO: 44;
the amino acid sequence of the heavy chain variable region is as shown by SEQ ID NO: 6, and the amino acid sequence of the light chain variable region is as shown by SEQ ID NO: 44;
the amino acid sequence of the heavy chain variable region is as shown by SEQ ID NO: 7, and the amino acid sequence of the light chain variable region is as shown by SEQ ID NO: 44;
the amino acid sequence of the heavy chain variable region is as shown by SEQ ID NO: 8, and the amino acid sequence of the light chain variable region is as shown by SEQ ID NO: 44;
the amino acid sequence of the heavy chain variable region is as shown by SEQ ID NO: 9, and the amino acid sequence of the light chain variable region is as shown by SEQ ID NO: 44;
the amino acid sequence of the heavy chain variable region is as shown by SEQ ID NO: 10, and the amino acid sequence of the light chain variable region is as shown by SEQ ID NO: 44;
or
the amino acid sequence of the heavy chain variable region is as shown by SEQ ID NO: 1, 2, 3, 4, 5, 6, 7, 8, 9 or 10, and the amino acid sequence of the light chain variable region is as shown by SEQ ID NO: 77.

5. The anti-EGFR antibody or antigen-binding fragment thereof according to any one of claims 1 to 4, wherein,
the anti-EGFR antibody comprises a non-CDR region, and the non-CDR region is derived from a species other than murine, for example, from a human antibody.

6. The anti-EGFR antibody or antigen-binding fragment thereof according to any one of claims 1 to 5, wherein the constant region of the anti-EGFR antibody is selected from the constant region of human IgG1, IgG2, IgG3 or IgG4.

7. The anti-EGFR antibody or antigen-binding fragment thereof according to any one of claims 1 to 6, wherein the heavy chain constant region of the anti-EGFR antibody is an Ig gamma-1 chain C region or an Ig gamma-4 chain C region; and the light chain constant region is an Ig kappa chain C region or an Ig lambda chain C region.

8. The anti-EGFR antibody or antigen-binding fragment thereof according to any one of claims 1 to 6, wherein the sequence of the heavy chain constant region of the anti-EGFR antibody is selected from SEQ ID NO: 79, 80 and 81.

9. The anti-EGFR antibody or antigen-binding fragment thereof according to any one of claims 1 to 8, wherein,
the heavy chain of the anti-EGFR antibody is selected from SEQ ID NO: 17, 18, 19, 58, 59, 60, 61, 62, 63, 64, 65, 66, 67, 68, 69, 70, 71, 72, 73, 74 and 75; and
the light chain of the anti-EGFR antibody is selected from SEQ ID NO: 11, 12, 13 and 57.

10. The anti-EGFR antibody or antigen-binding fragment thereof according to any one of claims 1 to 9, wherein:
the amino acid sequence of the heavy chain is as shown by SEQ ID NO: 17, and the amino acid sequence of the light chain is as shown by SEQ ID NO: 11;
the amino acid sequence of the heavy chain is as shown by SEQ ID NO: 17, and the amino acid sequence of the light chain is as shown by SEQ ID NO: 12;
the amino acid sequence of the heavy chain is as shown by SEQ ID NO: 17, and the amino acid sequence of the light chain is as shown by SEQ ID NO: 13;
the amino acid sequence of the heavy chain is as shown by SEQ ID NO: 18, and the amino acid sequence of the light chain is as shown by SEQ ID NO: 11;
the amino acid sequence of the heavy chain is as shown by SEQ ID NO: 18, and the amino acid sequence of the light chain is as shown by SEQ ID NO: 12;
the amino acid sequence of the heavy chain is as shown by SEQ ID NO: 18, and the amino acid sequence of the light chain is as shown by SEQ ID NO: 13;
the amino acid sequence of the heavy chain is as shown by SEQ ID NO: 19, and the amino acid sequence of the light chain is as shown by SEQ ID NO: 11;
the amino acid sequence of the heavy chain is as shown by SEQ ID NO: 19, and the amino acid sequence of the light chain is as shown by SEQ ID NO: 12;
the amino acid sequence of the heavy chain is as shown by SEQ ID NO: 19, and the amino acid sequence of the light chain is as shown by SEQ ID NO: 13;
or
the amino acid sequence of the heavy chain is as shown by SEQ ID NO: 17, 18, 19, 58, 59, 60, 61, 62, 63, 64, 65, 66, 67, 68, 69, 70, 71, 72, 73, 74 or 75, and the amino acid sequence of the light chain is as shown by SEQ ID NO: 57.

11. The anti-EGFR antibody or antigen-binding fragment thereof according to any one of claims 1 to 10, wherein the anti-EGFR antibody or antigen-binding fragment is selected from the group consisting of Fab, Fab', F(ab')₂, Fd, Fv, dAb, complementarity-determining region fragment, single-chain antibody, humanized antibody, and chimeric antibody.

12. An isolated nucleic acid molecule encoding the anti-EGFR antibody or antigen-binding fragment thereof according to any one of claims 1 to 11.

13. A recombinant vector comprising the isolated nucleic acid molecule of claim 12.

14. A host cell comprising the isolated nucleic acid molecule of claim 12 or the recombinant vector of claim 13.

15. A pharmaceutical composition comprising an effective amount of the anti-EGFR antibody or antigen-binding fragment thereof according to any one of claims 1 to 11, and one or more pharmaceutically acceptable excipients.

16. The pharmaceutical composition of claim 15, further comprising an effective amount of an anti-Met antibody or an antigen-binding fragment thereof;
preferably, the anti-Met antibody comprises a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
and
the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

17. The pharmaceutical composition according to any one of claims 15 to 16, wherein,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, for the anti-Met antibody:
the amino acid sequence of the heavy chain is as shown by SEQ ID NO: 16 or SEQ ID NO: 21, and the amino acid sequence of the light chain variable region is as shown by SEQ ID NO: 14.

18. A combined medicament product comprising an individually packaged first medicament product and a second medicament product, wherein:
the first medicament product comprises an effective amount of the anti-EGFR antibody or antigen-binding fragment thereof according to any one of claims 1 to 11, and one or more pharmaceutically acceptable excipients;
the second medicament product comprises an effective amount of an anti-Met antibody or its antigen-binding fragment, and one or more pharmaceutically acceptable excipients;
optionally, the combined medicament product further comprises a product instruction.

19. The combined medicament product according to claim 18, wherein,
the anti-Met antibody comprises a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
and
the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

20. The combined medicament product according to any one of claims 18 to 19, wherein,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, wherein the anti-Met antibody:
the amino acid sequence of the heavy chain is as shown by SEQ ID NO: 16 or SEQ ID NO: 21, and the amino acid sequence of the light chain variable region is as shown by SEQ ID NO: 14.

21. A bispecific antibody comprising:
a first protein functional region targeting EGFR, and
a second protein functional region targeting a target different from EGFR;
wherein, the first protein functional region comprises a heavy chain variable region of the anti-EGFR antibody or its antigen-binding fragment according to any one of claims 1 to 11; preferably, the first protein functional region comprises a heavy chain variable region and a light chain variable region of the anti-EGFR antibody or its antigen-binding fragment according to any one of claims 1 to 11.

22. The bispecific antibody according to claim 21, wherein,
the second protein functional region comprises a heavy chain variable region of the anti-Met antibody or antigen-binding fragment thereof; preferably comprising a heavy chain variable region and a light chain variable region of the anti-Met antibody or its antigen-binding fragment;
wherein, the anti-Met antibody comprises a heavy chain variable region and a light chain variable region, the heavy chain variable region comprising HCDR1 to HCDR3, and the light chain variable region comprising LCDR1 to LCDR3, wherein:
the amino acid sequence of HCDR1 is as shown by SEQ ID NO: 45, the amino acid sequence of HCDR2 is as shown by SEQ ID NO: 46, and the amino acid sequence of HCDR3 is as shown by SEQ ID NO: 47; or the amino acid sequence of HCDR1 is as shown by SEQ ID NO: 48, the amino acid sequence of HCDR2 is as shown by SEQ ID NO: 49, and the amino acid sequence of HCDR3 is as shown by SEQ ID NO: 50;
and
the amino acid sequence of LCDR1 is as shown by SEQ ID NO: 51, the amino acid sequence of LCDR2 is as shown by SEQ ID NO: 52, and the amino acid sequence of LCDR3 is as shown by SEQ ID NO: 53.

23. The bispecific antibody according to any one of claims 21 to 22, wherein,
the amino acid sequence of the heavy chain variable region of the anti-Met antibody is selected from SEQ ID NO: 54 and SEQ ID NO: 55; and
the amino acid sequence of the light chain variable region of the anti-Met antibody is as shown by SEQ ID NO: 56;
preferably, wherein the anti-Met antibody:
the amino acid sequence of the heavy chain is as shown by SEQ ID NO: 16 or SEQ ID NO: 21, and the amino acid sequence of the light chain variable region is as shown by SEQ ID NO: 14.

24. The bispecific antibody according to any one of claims 21 to 23, wherein,
the first and second protein functional regions are independently a fusion protein of a single-chain antibody or a half-molecule monovalent antibody (IgG half-molecule, IgG-HM).

25. The bispecific antibody according to any one of claims 21 to 24, wherein,
the first protein functional region is a half-molecule monovalent antibody, and
the second protein functional region is a half-molecule monovalent antibody.

26. The bispecific antibody according to any one of claims 21 to 25, wherein,
the first protein functional region is a half-molecule monovalent antibody targeting EGFR, and
the second protein functional region is a half-molecule monovalent antibody targeting Met.

27. The bispecific antibody according to any one of claims 24 to 26, wherein,
the heavy chain constant regions of the two half-molecule monovalent antibodies respectively comprise a first CH3 region and a second CH3 region, the sequences of the first CH3 region and the second CH3 region are different, and the heterodimer interaction between the first CH3 region and the second CH3 region is stronger than the homodimer interaction of the first CH3 region and the second CH3 region, respectively.

28. The bispecific antibody according to any one of claims 24 to 27, wherein,
the heavy chain constant region of the half-molecule monovalent antibody is the heavy chain constant region of human IgG1 and has a Knob mutation (e.g., S354C and T366W mutations); and
the heavy chain constant region of the half-molecule monovalent antibody is the heavy chain constant region of human IgG1 and has a Hole mutation (e.g., Y349C, T366S, L368A, and Y407V mutations).

29. The bispecific antibody according to any one of claims 24 to 28, wherein,
the heavy chain constant region of the half-molecule monovalent antibody is the heavy chain constant region of human IgG1, and position 435 of the heavy chain constant region of one half-molecule monovalent antibody is mutated to amino acid R, and position 436 is mutated to amino acid F, according to the EU Numbering System.

30. The bispecific antibody according to any one of claims 24 to 29, wherein,
the heavy chain constant region of the half-molecule monovalent antibody is the heavy chain constant region of human IgG1, and position 405 of the heavy chain constant region of one half-molecule monovalent antibody is mutated to amino acid L, and position 409 of the heavy chain constant region of the other half-molecule monovalent antibody is mutated to amino acid R, according to the EU Numbering System.

31. The bispecific antibody according to any one of claims 21 to 30, wherein it is in IgG form, preferably in IgG1 form;
preferably, the light chains in the bispecific antibody have identical sequences;
preferably, the bispecific antibody has two light chains with identical sequences;
preferably, the bispecific antibody is composed of the following peptide chains:
(1) a peptide chain selected from SEQ ID NO: 17 to SEQ ID NO: 19, and SEQ ID NO: 58 to SEQ ID NO: 75,
(2) a peptide chain selected from SEQ ID NO: 16 and SEQ ID NO: 20, and
(3) a peptide chain selected from SEQ ID NO: 11 to SEQ ID NO: 13, and SEQ ID NO: 57,
wherein, the peptide chain in (3) is two identical copies;
preferably, the peptide chains in (1) and (2), the peptide chains in (2) and (3), and the peptide chains in (1) and (3) are linked by one or more disulfide bonds (e.g., two or three disulfide bonds).

32. The bispecific antibody according to any one of claims 21 to 31, comprising the following peptide chains:
the peptide chain shown in SEQ ID NO: 17, the peptide chain shown in SEQ ID NO: 16, and the peptide chain shown in SEQ ID NO: 12, wherein the peptide chain shown in SEQ ID NO: 12 is two identical copies;
the peptide chain shown in SEQ ID NO: 17, the peptide chain shown in SEQ ID NO: 16, and the peptide chain shown in SEQ ID NO: 13, wherein the peptide chain shown in SEQ ID NO: 13 is two identical copies;
the peptide chain shown in SEQ ID NO: 18, the peptide chain shown in SEQ ID NO: 20, and the peptide chain shown in SEQ ID NO: 12, wherein the peptide chain shown in SEQ ID NO: 12 is two identical copies;
the peptide chain shown in SEQ ID NO: 18, the peptide chain shown in SEQ ID NO: 20, and the peptide chain shown in SEQ ID NO: 13, wherein the peptide chain shown in SEQ ID NO: 13 is two identical copies;
the peptide chain shown in SEQ ID NO: 19, the peptide chain shown in SEQ ID NO: 20, and the peptide chain shown in SEQ ID NO: 12, wherein the peptide chain shown in SEQ ID NO: 12 is two identical copies;
the peptide chain shown in SEQ ID NO: 19, the peptide chain shown in SEQ ID NO: 20, and the peptide chain shown in SEQ ID NO: 13, wherein the peptide chain shown in SEQ ID NO: 13 is an identical copy;
the peptide chain shown in any of SEQ ID NO: 17, 18, 19, 58, 59, 60, 61, 62, 63, 64, 65, 66, 67, 68, 69, 70, 71, 72, 73, 74, or 75, the peptide chain shown in SEQ ID NO: 16, and the peptide chain shown in SEQ ID NO: 57, wherein the peptide chain shown in SEQ ID NO: 57 is an identical copy;
or
the peptide chains shown in any of SEQ ID NO: 17, 18, 19, 58, 59, 60, 61, 62, 63, 64, 65, 66, 67, 68, 69, 70, 71, 72, 73, 74, or 75, as well as the peptide chain shown in SEQ ID NO: 20 and the peptide chain shown in SEQ ID NO: 57, wherein the peptide chain shown in SEQ ID NO: 57 is two identical copies.

33. An isolated nucleic acid molecule encoding the bispecific antibody according to any one of claims 21 to 32.

34. A recombinant expression vector comprising the isolated nucleic acid molecule of claim 33.

35. A recombinant host cell comprising the recombinant expression vector of claim 34, preferably wherein the recombinant host cell is a recombinant CHO-K1 cell.

36. A pharmaceutical composition comprising the bispecific antibody according to any one of claims 21 to 32, and one or more pharmaceutically acceptable excipients.

37. Use of the anti-EGFR antibody or antigen-binding fragment thereof according to any one of claims 1 to 11, or the bispecific antibody according to any one of claims 22 to 33, in the preparation of a medicament for treating or preventing tumors;
preferably, the tumor is a tumor that highly expresses EGFR and/or Met;
preferably, the tumor is one or more selected from glial cell carcinoma, renal cell carcinoma, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, gastric cancer, brain cancer, thyroid cancer, and head and neck cancer;
preferably, the lung cancer is non-small cell lung cancer.

38. An anti-EGFR antibody or antigen-binding fragment thereof according to any one of claims 1 to 11, or a bispecific antibody according to any one of claims 21 to 32, for the treatment or prevention of tumors;
preferably, the tumor is a tumor that highly expresses EGFR and/or Met;
preferably, the tumor is one or more selected from glial carcinoma, renal cell carcinoma, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, gastric cancer, brain cancer, thyroid cancer, and head and neck cancer;
preferably, the lung cancer is non-small cell lung cancer.

39. A method for treating or preventing tumors, comprising administering to a subject in need an effective amount of the anti-EGFR antibody or antigen-binding fragment thereof according to any one of claims 1 to 11, or the bispecific antibody according to any one of claims 21 to 32;
preferably, the tumor is a tumor that highly expresses EGFR and/or Met;
preferably, the tumor is one or more selected from glial cell carcinoma, renal cell carcinoma, lung cancer, prostate cancer, pancreatic cancer, breast cancer, biliary tract cancer, bronchial cancer, lymphoma, ovarian cancer, esophageal cancer, melanoma, hematologic malignancy, bladder cancer, colon cancer, rectal cancer, liver cancer, gastric cancer, brain cancer, thyroid cancer, and head and neck cancer;
preferably, the lung cancer is non-small cell lung cancer.
